(19) Europäisches Patentamt
European Patent Office
Office européen des brevets

(11) EP 4 772 634 A1

(12) EUROPEAN PATENT APPLICATION
published in accordance with Art. 153(4) EPC

(43) Date of publication:
08.07.2026 Bulletin 2026/28

(21) Application number: 24858725.5

(22) Date of filing: 30.08.2024

(51) International Patent Classification (IPC):
*C12N 15/113* (2010.01) *A61K 31/713* (2006.01)
*A61P 7/00* (2006.01) *A61K 48/00* (2006.01)

(52) Cooperative Patent Classification (CPC):
A61K 31/713; A61K 48/00; A61P 7/00;
C12N 15/113

(86) International application number:
PCT/CN2024/115820

(87) International publication number:
WO 2025/045194 (06.03.2025 Gazette 2025/10)

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
GE KH MA MD TN

(30) Priority: 31.08.2023 CN 202311121470

(71) Applicant: Chia Tai Tianqing Pharmaceutical Group Co., Ltd.
Lianyungang, Jiangsu 222062 (CN)

(72) Inventors:
- GE, Xingfeng
Lianyungang, Jiangsu 222062 (CN)
- ZHENG, Jiajia
Lianyungang, Jiangsu 222062 (CN)
- CHEN, Shuo
Lianyungang, Jiangsu 222062 (CN)
- LU, Dandan
Lianyungang, Jiangsu 222062 (CN)
- XU, Hongjiang
Lianyungang, Jiangsu 222062 (CN)

(74) Representative: WSL Patentanwälte Partnerschaft mbB
Kaiser-Friedrich-Ring 98
65185 Wiesbaden (DE)

Remarks:
The complete document including Reference Table(s) and the Sequence Listing(s) can be downloaded from the EPO website

# (54) DOUBLE-STRANDED RNA TARGETING COAGULATION FACTOR XI

(57) Provided are a double-stranded RNA targeting coagulation factor XI, a pharmaceutically acceptable salt thereof, or a ligand conjugate thereof. Further provided are a pharmaceutical composition comprising the double-stranded RNA, the pharmaceutically acceptable salt thereof, or the ligand conjugate thereof, as well as a therapeutic use of the double-stranded RNA, the pharmaceutically acceptable salt thereof, or the ligand conjugate thereof.

EP 4 772 634 A1

## Description

### CROSS-REFERENCE TO RELATED APPLICATIONS

The present application claims priority and benefit to the Chinese Patent No. 202311121470.9 filed with the National Intellectual Property Administration, PRC on August 31, 2023, which is incorporated herein by reference in its entirety.

### TECHNICAL FIELD

The present application relates to the field of biomedicine, and in particular, to a double-stranded ribonucleic acid (dsRNA), a pharmaceutically acceptable salt thereof, a ligand conjugate thereof, or a pharmaceutical composition, which can be used for inhibiting coagulation factor XI.

### BACKGROUND

Coagulation factor XI is a serine protease zymogen that is primarily synthesized in the liver and megakaryocytes. Coagulation factor XI is involved in the intrinsic coagulation pathway. In the coagulation cascade reaction, thrombin can activate coagulation factor XI in a feedback manner, and the activated coagulation factor XI, in turn, promotes the substantial production of thrombin, thereby amplifying the coagulation cascade reaction. Therefore, drugs targeting coagulation factor XI can block the intrinsic pathway and inhibit the amplification of the coagulation cascade reaction, thereby exerting antithrombotic effects without causing excessive hemorrhage.

Currently, discovered coagulation factor XI inhibitors are still relatively few, mainly including monoclonal antibodies, antisense oligonucleotides, small molecule inhibitors, peptide mimetics, peptide inhibitors, and the like. There are no approved coagulation factor XI inhibitors on the market yet.

Small interfering RNAs (siRNAs) can inhibit the expression of a target gene by inhibiting or blocking the translation or transcription of the target gene in a sequence-specific manner on the basis of the RNA interference (RNAi) mechanism, and exert an inhibitory effect at the mRNA level while reducing the level of coagulation factor XI, thereby achieving the purpose of treating diseases.

### BRIEF SUMMARY

In one aspect, the present application provides a double-stranded ribonucleic acid (dsRNA), a pharmaceutically acceptable salt thereof, or a ligand conjugate thereof, wherein the dsRNA comprises a sense strand and an antisense strand, wherein the sense strand comprises a nucleotide sequence having a length of no more than 23 nucleotides, and the antisense strand comprises a nucleotide sequence having a length of no more than 25 nucleotides; the sense strand or the antisense strand is optionally modified; the double-stranded ribonucleic acid (dsRNA), the pharmaceutically acceptable salt thereof, or the ligand conjugate thereof targets coagulation factor XI mRNA.

In another aspect, the present application provides a double-stranded ribonucleic acid (dsRNA), a pharmaceutically acceptable salt thereof, or a ligand conjugate thereof, wherein the dsRNA comprises a sense strand and an antisense strand, wherein the sense strand comprises at least 15 contiguous nucleotides in the nucleotide sequence set forth in SEQ ID NO. 1, SEQ ID NO. 3, SEQ ID NO. 5, SEQ ID NO. 7, SEQ ID NO. 9, SEQ ID NO. 11, SEQ ID NO. 13, SEQ ID NO. 15, SEQ ID NO. 17, SEQ ID NO. 19, SEQ ID NO. 21, SEQ ID NO. 23, SEQ ID NO. 25, or SEQ ID NO. 27 and has a length of no more than 21 nucleotides, and the antisense strand comprises at least 15 contiguous nucleotides in the nucleotide sequence set forth in SEQ ID NO. 2, SEQ ID NO. 4, SEQ ID NO. 6, SEQ ID NO. 8, SEQ ID NO. 10, SEQ ID NO. 12, SEQ ID NO. 14, SEQ ID NO. 16, SEQ ID NO. 18, SEQ ID NO. 20, SEQ ID NO. 22, SEQ ID NO. 24, SEQ ID NO. 26, or SEQ ID NO. 28 and has a length of no more than 23 nucleotides:

sense strand: 5’-GAUCUCCAACUAAAAUACU-3’ (SEQ ID NO. 1),

antisense strand: 5’-AGUAUUUUAGUUGGAGAUCCG-3’ (SEQ ID NO. 2);

sense strand: 5’-GGAUGAUUUUCUUAUAUCA-3’ (SEQ ID NO. 3),

antisense strand: 5’-UGAUAUAAGAAAAUCAUCCUG-3’ (SEQ ID NO. 4);

sense strand: 5’-GGUCUUUUCAGGAUGAUUU-3’ (SEQ ID NO. 5),

antisense strand: 5'-AAAUCAUCCUGAAAAGACCUU-3' (SEQ ID NO. 6);

sense strand: 5'-GUCUUUUCAGGAUGAUUUU-3' (SEQ ID NO. 7),

antisense strand: 5'-AAAAUCAUCCUGAAAAGACCU-3' (SEQ ID NO. 8);

sense strand: 5'-CUUUUCAGGAUGAUUUUCU-3' (SEQ ID NO. 9),

antisense strand: 5'-AGAAAAUCAUCCUGAAAAGAC-3' (SEQ ID NO. 10);

sense strand: 5'-UUUUCAGGAUGAUUUUCUU-3' (SEQ ID NO. 11),

antisense strand: 5'-AAGAAAAUCAUCCUGAAAAGA-3' (SEQ ID NO. 12);

sense strand: 5'-UCAGGAUGAUUUUCUUAUA-3' (SEQ ID NO. 13),

antisense strand: 5'-UAUAAGAAAAUCAUCCUGAAA-3' (SEQ ID NO. 14);

sense strand: 5'-CAGGAUGAUUUUCUUAUAU-3' (SEQ ID NO. 15),

antisense strand: 5'-AUAUAAGAAAAUCAUCCUGAA-3' (SEQ ID NO. 16);

sense strand: 5'-AGGAUGAUUUUCUUAUAUC-3' (SEQ ID NO. 17),

antisense strand: 5'-GAUAUAAGAAAAUCAUCCUGA-3' (SEQ ID NO. 18);

sense strand: 5'-GAUGAUUUUCUUAUAUCAA-3' (SEQ ID NO. 19),

antisense strand: 5'-UUGAUAUAAGAAAAUCAUCCU-3' (SEQ ID NO. 20);

sense strand: 5'-AUGAUUUUCUUAUAUCAAG-3' (SEQ ID NO. 21),

antisense strand: 5'-CUUGAUAUAAGAAAAUCAUCC-3' (SEQ ID NO. 22);

sense strand: 5'-UGAUUUUCUUAUAUCAAGU-3' (SEQ ID NO. 23),

antisense strand: 5'-ACUUGAUAUAAGAAAAUCAUC-3' (SEQ ID NO. 24);

sense strand: 5'-CUUAUAUCAAGUGGUACAU-3' (SEQ ID NO. 25),

antisense strand: 5'-AUGUACCACUUGAUAUAAGAA-3' (SEQ ID NO. 26);

sense strand: 5'-AAACGGAUCUCCAACUAAA-3' (SEQ ID NO. 27),

antisense strand: 5'-UUUAGUUGGAGAUCCGUUUGA-3' (SEQ ID NO. 28);

the sense strand or the antisense strand is optionally modified.

In another aspect, the present application provides a pharmaceutical composition, which comprises the dsRNA, the pharmaceutically acceptable salt thereof, or the ligand conjugate thereof of the present application. In another aspect, the present application provides a pharmaceutical composition, which comprises the dsRNA, the pharmaceutically acceptable salt thereof, or the ligand conjugate thereof of the present application, and a pharmaceutically acceptable carrier or excipient.

In another aspect, the present application provides a kit for treating and/or preventing thromboembolism and complications thereof, which comprises the dsRNA, the pharmaceutically acceptable salt thereof, or the ligand conjugate thereof, or the pharmaceutical composition of the present application, and optionally an instruction for use.

In another aspect, the present application provides a method for treating and/or preventing thromboembolism and complications thereof, which comprises administering to a subject in need of treatment and/or prevention the dsRNA, the pharmaceutically acceptable salt thereof, or the ligand conjugate thereof, or the pharmaceutical composition of the

present application.

In some embodiments, the present application provides a method for treating and/or preventing thromboembolism and complications thereof, which comprises administering to a subject in need of treatment and/or prevention a therapeutically/prophylactically effective amount of the dsRNA, the pharmaceutically acceptable salt thereof, or the ligand conjugate thereof, or the pharmaceutical composition of the present application.

In another aspect, the present application provides use of the dsRNA, the pharmaceutically acceptable salt thereof, or the ligand conjugate thereof, or the pharmaceutical composition of the present application for preparing a medicament for treating and/or preventing thromboembolism and complications thereof.

In another aspect, the present application provides use of the dsRNA, the pharmaceutically acceptable salt thereof, or the ligand conjugate thereof, or the pharmaceutical composition of the present application for treating and/or preventing thromboembolism and complications thereof.

In another aspect, the present application provides the dsRNA, the pharmaceutically acceptable salt thereof, or the ligand conjugate thereof, or the pharmaceutical composition of the present application for use in treating and/or preventing thromboembolism and complications thereof.

In some embodiments, the thromboembolism and complications thereof may include various types of thrombosis, embolism, and thromboembolism, such as arterial embolism and venous embolism, including deep vein thrombosis, pulmonary embolism, myocardial infarction, stroke, and the like.

In some embodiments, the thromboembolism and complications thereof specifically include thromboembolism in end-stage renal disease patients undergoing hemodialysis, venous thromboembolism in patients having undergone knee replacement surgery, stroke or systemic embolism in patients with atrial fibrillation, cancer-associated venous thromboembolism, and the like.

In some embodiments, the dsRNA, the pharmaceutically acceptable salt thereof, or the ligand conjugate thereof, or the pharmaceutical composition of the present application is used as a single therapeutic agent for treating and/or preventing thromboembolism and complications thereof.

In some embodiments, the dsRNA, the pharmaceutically acceptable salt thereof, or the ligand conjugate thereof, or the pharmaceutical composition of the present application is used in combination with an additional therapeutic agent for treating and/or preventing thromboembolism and complications thereof.

## SUMMARY

The exemplary embodiments of the present application will be described below; however, it will be appreciated by those skilled in the art that the protection scope of the present application is not limited thereto, and that various modifications, alterations, or changes can be made based on the spirit and conception of the present application, and the content with these modifications, alterations, or changes shall still fall within the scope of the present application.

The present application provides a double-stranded ribonucleic acid (dsRNA), a pharmaceutically acceptable salt thereof, or a ligand conjugate thereof, wherein the dsRNA comprises a sense strand and an antisense strand, wherein the sense strand comprises a nucleic acid sequence differing by 0, 1, 2, or 3 nucleotides from at least 15 (e.g., 15, 16, 17, 18, or 19) contiguous nucleotides in a nucleotide sequence as shown in Table 1, and the sense strand has a length of no more than 21 nucleotides; the antisense strand comprises a nucleic acid sequence differing by 0, 1, 2, or 3 nucleotides from at least 15 (e.g., 15, 16, 17, 18, or 19) contiguous nucleotides in a nucleotide sequence as shown in Table 2, and the antisense strand has a length of no more than 23 nucleotides.

The present application provides a double-stranded ribonucleic acid (dsRNA), a pharmaceutically acceptable salt thereof, or a ligand conjugate thereof, wherein the dsRNA comprises a sense strand and an antisense strand, wherein the sense strand comprises at least 15 contiguous nucleotides in the nucleotide sequence set forth in SEQ ID NO. 1, SEQ ID NO. 3, SEQ ID NO. 5, SEQ ID NO. 7, SEQ ID NO. 9, SEQ ID NO. 11, SEQ ID NO. 13, SEQ ID NO. 15, SEQ ID NO. 17, SEQ ID NO. 19, SEQ ID NO. 21, SEQ ID NO. 23, SEQ ID NO. 25, or SEQ ID NO. 27 and has a length of no more than 21 nucleotides, and the antisense strand comprises at least 15 contiguous nucleotides in the nucleotide sequence set forth in SEQ ID NO. 2, SEQ ID NO. 4, SEQ ID NO. 6, SEQ ID NO. 8, SEQ ID NO. 10, SEQ ID NO. 12, SEQ ID NO. 14, SEQ ID NO. 16, SEQ ID NO. 18, SEQ ID NO. 20, SEQ ID NO. 22, SEQ ID NO. 24, SEQ ID NO. 26, or SEQ ID NO. 28 and has a length of no more than 23 nucleotides:

sense strand: 5'-GAUCUCCAACUAAAAUACU-3' (SEQ ID NO. 1),
antisense strand: 5'-AGUAUUUUAGUUGGAGAUCCG-3' (SEQ ID NO. 2);
sense strand: 5'-GGAUGAUUUUCUUAUAUCA-3' (SEQ ID NO. 3),
antisense strand: 5'-UGAUAUAAGAAAAUCAUCCUG-3' (SEQ ID NO. 4);
sense strand: 5'-GGUCUUUUCAGGAUGAUUU-3' (SEQ ID NO. 5),
antisense strand: 5'-AAAUCAUCCUGAAAAGACCUU-3' (SEQ ID NO. 6);
sense strand: 5'-GUCUUUUCAGGAUGAUUUU-3' (SEQ ID NO. 7),

antisense strand: 5'-AAAAUCAUCCUGAAAAGACCU-3' (SEQ ID NO. 8);
sense strand: 5'-CUUUUCAGGAUGAUUUUCU-3' (SEQ ID NO. 9),
antisense strand: 5'-AGAAAAUCAUCCUGAAAAGAC-3' (SEQ ID NO. 10);
sense strand: 5'-UUUUCAGGAUGAUUUUCUU-3' (SEQ ID NO. 11),
antisense strand: 5'-AAGAAAAUCAUCCUGAAAAGA-3' (SEQ ID NO. 12);
sense strand: 5'-UCAGGAUGAUUUUCUUAUA-3' (SEQ ID NO. 13),
antisense strand: 5'-UAUAAGAAAAUCAUCCUGAAA-3' (SEQ ID NO. 14);
sense strand: 5'-CAGGAUGAUUUUCUUAUAU-3' (SEQ ID NO. 15),
antisense strand: 5'-AUAUAAGAAAAUCAUCCUGAA-3' (SEQ ID NO. 16);
sense strand: 5'-AGGAUGAUUUUCUUAUAUC-3' (SEQ ID NO. 17),
antisense strand: 5'-GAUAUAAGAAAAUCAUCCUGA-3' (SEQ ID NO. 18);
sense strand: 5'-GAUGAUUUUCUUAUAUCAA-3' (SEQ ID NO. 19),
antisense strand: 5'-UUGAUAUAAGAAAAUCAUCCU-3' (SEQ ID NO. 20);
sense strand: 5'-AUGAUUUUCUUAUAUCAAG-3' (SEQ ID NO. 21),
antisense strand: 5'-CUUGAUAUAAGAAAAUCAUCC-3' (SEQ ID NO. 22);
sense strand: 5'-UGAUUUUCUUAUAUCAAGU-3' (SEQ ID NO. 23),
antisense strand: 5'-ACUUGAUAUAAGAAAAUCAUC-3' (SEQ ID NO. 24);
sense strand: 5'-CUUAUAUCAAGUGGUACAU-3' (SEQ ID NO. 25),
antisense strand: 5'-AUGUACCACUUGAUAUAAGAA-3' (SEQ ID NO. 26);
sense strand: 5'-AAACGGAUCUCCAACUAAA-3' (SEQ ID NO. 27),
antisense strand: 5'-UUUAGUUGGAGAUCCGUUUGA-3' (SEQ ID NO. 28);

the sense strand or the antisense strand is optionally modified.

In some embodiments, the sense strand comprises at least 15 contiguous nucleotides in the nucleotide sequence set forth in SEQ ID NO. 1, SEQ ID NO. 3, SEQ ID NO. 5, SEQ ID NO. 7, SEQ ID NO. 9, SEQ ID NO. 11, SEQ ID NO. 13, SEQ ID NO. 15, SEQ ID NO. 17, SEQ ID NO. 19, SEQ ID NO. 21, SEQ ID NO. 23, SEQ ID NO. 25, or SEQ ID NO. 27 and has a length of no more than 21 nucleotides, wherein the at least 15 contiguous nucleotides may be selected from the group consisting of 15, 16, 17, 18, and 19 contiguous nucleotides.

In some embodiments, the antisense strand comprises at least 15 contiguous nucleotides in the nucleotide sequence set forth in SEQ ID NO. 2, SEQ ID NO. 4, SEQ ID NO. 6, SEQ ID NO. 8, SEQ ID NO. 10, SEQ ID NO. 12, SEQ ID NO. 14, SEQ ID NO. 16, SEQ ID NO. 18, SEQ ID NO. 20, SEQ ID NO. 22, SEQ ID NO. 24, SEQ ID NO. 26, or SEQ ID NO. 28 and has a length of no more than 23 nucleotides, wherein the at least 15 contiguous nucleotides may be selected from the group consisting of 15, 16, 17, 18, 19, 20, and 21 contiguous nucleotides.

In some embodiments, the present application provides a double-stranded ribonucleic acid (dsRNA), a pharmaceutically acceptable salt thereof, or a ligand conjugate thereof, wherein the dsRNA comprises a sense strand and an antisense strand, wherein the sense strand comprises the nucleotide sequence set forth in SEQ ID NO. 1, SEQ ID NO. 3, SEQ ID NO. 5, SEQ ID NO. 7, SEQ ID NO. 9, SEQ ID NO. 11, SEQ ID NO. 13, SEQ ID NO. 15, SEQ ID NO. 17, SEQ ID NO. 19, SEQ ID NO. 21, SEQ ID NO. 23, SEQ ID NO. 25, or SEQ ID NO. 27 and has a length of no more than 21 nucleotides, and the antisense strand comprises the nucleotide sequence set forth in SEQ ID NO. 2, SEQ ID NO. 4, SEQ ID NO. 6, SEQ ID NO. 8, SEQ ID NO. 10, SEQ ID NO. 12, SEQ ID NO. 14, SEQ ID NO. 16, SEQ ID NO. 18, SEQ ID NO. 20, SEQ ID NO. 22, SEQ ID NO. 24, SEQ ID NO. 26, or SEQ ID NO. 28 and has a length of no more than 23 nucleotides:

sense strand: 5'-GAUCUCCAACUAAAAUACU-3' (SEQ ID NO. 1),

antisense strand: 5'-AGUAUUUUAGUUGGAGAUCCG-3' (SEQ ID NO. 2);

sense strand: 5'-GGAUGAUUUUCUUAUAUCA-3' (SEQ ID NO. 3),

antisense strand: 5'-UGAUAUAAGAAAAUCAUCCUG-3' (SEQ ID NO. 4);

sense strand: 5'-GGUCUUUUCAGGAUGAUUU-3' (SEQ ID NO. 5),

antisense strand: 5'-AAAUCAUCCUGAAAAGACCUU-3' (SEQ ID NO. 6);

sense strand: 5'-GUCUUUUCAGGAUGAUUUU-3' (SEQ ID NO. 7),

antisense strand: 5'-AAAAUCAUCCUGAAAAGACCU-3' (SEQ ID NO. 8);

sense strand: 5'-CUUUUCAGGAUGAUUUUCU-3' (SEQ ID NO. 9),

antisense strand: 5'-AGAAAAUCAUCCUGAAAAGAC-3' (SEQ ID NO. 10);

sense strand: 5'-UUUUCAGGAUGAUUUUCUU-3' (SEQ ID NO. 11),

antisense strand: 5'-AAGAAAAUCAUCCUGAAAAGA-3' (SEQ ID NO. 12);

sense strand: 5'-UCAGGAUGAUUUUCUUAUA-3' (SEQ ID NO. 13),

antisense strand: 5'-UAUAAGAAAAUCAUCCUGAAA-3' (SEQ ID NO. 14);

sense strand: 5'-CAGGAUGAUUUUCUUAUAU-3' (SEQ ID NO. 15),

antisense strand: 5'-AUAUAAGAAAAUCAUCCUGAA-3' (SEQ ID NO. 16);

sense strand: 5'-AGGAUGAUUUUCUUAUAUC-3' (SEQ ID NO. 17),

antisense strand: 5'-GAUAUAAGAAAAUCAUCCUGA-3' (SEQ ID NO. 18);

sense strand: 5'-GAUGAUUUUCUUAUAUCAA-3' (SEQ ID NO. 19),

antisense strand: 5'-UUGAUAUAAGAAAAUCAUCCU-3' (SEQ ID NO. 20);

sense strand: 5'-AUGAUUUUCUUAUAUCAAG-3' (SEQ ID NO. 21),

antisense strand: 5'-CUUGAUAUAAGAAAAUCAUCC-3' (SEQ ID NO. 22);

sense strand: 5'-UGAUUUUCUUAUAUCAAGU-3' (SEQ ID NO. 23),

antisense strand: 5'-ACUUGAUAUAAGAAAAUCAUC-3' (SEQ ID NO. 24);

sense strand: 5'-CUUAUAUCAAGUGGUACAU-3' (SEQ ID NO. 25),

antisense strand: 5'-AUGUACCACUUGAUAUAAGAA-3' (SEQ ID NO. 26);

sense strand: 5'-AAACGGAUCUCCAACUAAA-3' (SEQ ID NO. 27),

antisense strand: 5'-UUUAGUUGGAGAUCCGUUUGA-3' (SEQ ID NO. 28);

the sense strand or the antisense strand is optionally modified.

In some embodiments, the present application provides a double-stranded ribonucleic acid (dsRNA), a pharmaceutically acceptable salt thereof, or a ligand conjugate thereof, wherein the dsRNA comprises a sense strand and an antisense strand, wherein the sense strand is the nucleotide sequence set forth in SEQ ID NO. 1, SEQ ID NO. 3, SEQ ID NO. 5, SEQ ID NO. 7, SEQ ID NO. 9, SEQ ID NO. 11, SEQ ID NO. 13, SEQ ID NO. 15, SEQ ID NO. 17, SEQ ID NO. 19, SEQ ID NO. 21, SEQ ID NO. 23, SEQ ID NO. 25, or SEQ ID NO. 27, and the antisense strand is the nucleotide sequence set forth in SEQ ID NO. 2, SEQ ID NO. 4, SEQ ID NO. 6, SEQ ID NO. 8, SEQ ID NO. 10, SEQ ID NO. 12, SEQ ID NO. 14, SEQ ID NO. 16, SEQ ID NO. 18, SEQ ID NO. 20, SEQ ID NO. 22, SEQ ID NO. 24, SEQ ID NO. 26, or SEQ ID NO. 28:

sense strand: 5'-GAUCUCCAACUAAAAUACU-3' (SEQ ID NO. 1),

antisense strand: 5'-AGUAUUUUAGUUGGAGAUCCG-3' (SEQ ID NO. 2);

sense strand: 5'-GGAUGAUUUUCUUAUAUCA-3' (SEQ ID NO. 3),

antisense strand: 5'-UGAUAUAAGAAAAUCAUCCUG-3' (SEQ ID NO. 4);

sense strand: 5'-GGUCUUUUCAGGAUGAUUU-3' (SEQ ID NO. 5),

antisense strand: 5'-AAAUCAUCCUGAAAAGACCUU-3' (SEQ ID NO. 6);

sense strand: 5'-GUCUUUUCAGGAUGAUUUU-3' (SEQ ID NO. 7),

antisense strand: 5'-AAAAUCAUCCUGAAAAGACCU-3' (SEQ ID NO. 8);

sense strand: 5'-CUUUUCAGGAUGAUUUUCU-3' (SEQ ID NO. 9),

antisense strand: 5'-AGAAAAUCAUCCUGAAAAGAC-3' (SEQ ID NO. 10);

sense strand: 5'-UUUUCAGGAUGAUUUUCUU-3' (SEQ ID NO. 11),

antisense strand: 5'-AAGAAAAUCAUCCUGAAAAGA-3' (SEQ ID NO. 12);

sense strand: 5'-UCAGGAUGAUUUUCUUAUA-3' (SEQ ID NO. 13),

antisense strand: 5'-UAUAAGAAAAUCAUCCUGAAA-3' (SEQ ID NO. 14);

sense strand: 5'-CAGGAUGAUUUUCUUAUAU-3' (SEQ ID NO. 15),

antisense strand: 5'-AUAUAAGAAAAUCAUCCUGAA-3' (SEQ ID NO. 16);

sense strand: 5'-AGGAUGAUUUUCUUAUAUC-3' (SEQ ID NO. 17),

antisense strand: 5'-GAUAUAAGAAAAUCAUCCUGA-3' (SEQ ID NO. 18);

sense strand: 5'-GAUGAUUUUCUUAUAUCAA-3' (SEQ ID NO. 19),

antisense strand: 5'-UUGAUAUAAGAAAAUCAUCCU-3' (SEQ ID NO. 20);

sense strand: 5'-AUGAUUUUCUUAUAUCAAG-3' (SEQ ID NO. 21),

antisense strand: 5'-CUUGAUAUAAGAAAAUCAUCC-3' (SEQ ID NO. 22);

sense strand: 5'-UGAUUUUCUUAUAUCAAGU-3' (SEQ ID NO. 23),

antisense strand: 5'-ACUUGAUAUAAGAAAAUCAUC-3' (SEQ ID NO. 24);

sense strand: 5'-CUUAUAUCAAGUGGUACAU-3' (SEQ ID NO. 25),

antisense strand: 5'-AUGUACCACUUGAUAUAAGAA-3' (SEQ ID NO. 26);

sense strand: 5'-AAACGGAUCUCCAACUAAA-3' (SEQ ID NO. 27),

antisense strand: 5'-UUUAGUUGGAGAUCCGUUUGA-3' (SEQ ID NO. 28);

the sense strand or the antisense strand is optionally modified.

In some embodiments, the present application provides a double-stranded ribonucleic acid (dsRNA), a pharmaceutically acceptable salt thereof, or a ligand conjugate thereof, wherein the dsRNA comprises a sense strand and an antisense strand, wherein the sense strand comprises at least 15 contiguous nucleotides in the nucleotide sequence set forth in SEQ ID NO. 1 and has a length of no more than 21 nucleotides, and the antisense strand comprises at least 15 contiguous nucleotides in the nucleotide sequence set forth in SEQ ID NO. 2 and has a length of no more than 23 nucleotides; the sense strand comprises at least 15 contiguous nucleotides in the nucleotide sequence set forth in SEQ ID NO. 3 and has a length of no more than 21 nucleotides, and the antisense strand comprises at least 15 contiguous nucleotides in the nucleotide sequence set forth in SEQ ID NO. 4 and has a length of no more than 23 nucleotides; the sense strand comprises at least 15 contiguous nucleotides in the nucleotide sequence set forth in SEQ ID NO. 5 and has a

length of no more than 21 nucleotides, and the antisense strand comprises at least 15 contiguous nucleotides in the nucleotide sequence set forth in SEQ ID NO. 6 and has a length of no more than 23 nucleotides; the sense strand comprises at least 15 contiguous nucleotides in the nucleotide sequence set forth in SEQ ID NO. 7 and has a length of no more than 21 nucleotides, and the antisense strand comprises at least 15 contiguous nucleotides in the nucleotide sequence set forth in SEQ ID NO. 8 and has a length of no more than 23 nucleotides; the sense strand comprises at least 15 contiguous nucleotides in the nucleotide sequence set forth in SEQ ID NO. 9 and has a length of no more than 21 nucleotides, and the antisense strand comprises at least 15 contiguous nucleotides in the nucleotide sequence set forth in SEQ ID NO. 10 and has a length of no more than 23 nucleotides; the sense strand comprises at least 15 contiguous nucleotides in the nucleotide sequence set forth in SEQ ID NO. 11 and has a length of no more than 21 nucleotides, and the antisense strand comprises at least 15 contiguous nucleotides in the nucleotide sequence set forth in SEQ ID NO. 12 and has a length of no more than 23 nucleotides; the sense strand comprises at least 15 contiguous nucleotides in the nucleotide sequence set forth in SEQ ID NO. 13 and has a length of no more than 21 nucleotides, and the antisense strand comprises at least 15 contiguous nucleotides in the nucleotide sequence set forth in SEQ ID NO. 14 and has a length of no more than 23 nucleotides; the sense strand comprises at least 15 contiguous nucleotides in the nucleotide sequence set forth in SEQ ID NO. 15 and has a length of no more than 21 nucleotides, and the antisense strand comprises at least 15 contiguous nucleotides in the nucleotide sequence set forth in SEQ ID NO. 16 and has a length of no more than 23 nucleotides; the sense strand comprises at least 15 contiguous nucleotides in the nucleotide sequence set forth in SEQ ID NO. 17 and has a length of no more than 21 nucleotides, and the antisense strand comprises at least 15 contiguous nucleotides in the nucleotide sequence set forth in SEQ ID NO. 18 and has a length of no more than 23 nucleotides; the sense strand comprises at least 15 contiguous nucleotides in the nucleotide sequence set forth in SEQ ID NO. 19 and has a length of no more than 21 nucleotides, and the antisense strand comprises at least 15 contiguous nucleotides in the nucleotide sequence set forth in SEQ ID NO. 20 and has a length of no more than 23 nucleotides; the sense strand comprises at least 15 contiguous nucleotides in the nucleotide sequence set forth in SEQ ID NO. 21 and has a length of no more than 21 nucleotides, and the antisense strand comprises at least 15 contiguous nucleotides in the nucleotide sequence set forth in SEQ ID NO. 22 and has a length of no more than 23 nucleotides; the sense strand comprises at least 15 contiguous nucleotides in the nucleotide sequence set forth in SEQ ID NO. 23 and has a length of no more than 21 nucleotides, and the antisense strand comprises at least 15 contiguous nucleotides in the nucleotide sequence set forth in SEQ ID NO. 24 and has a length of no more than 23 nucleotides; the sense strand comprises at least 15 contiguous nucleotides in the nucleotide sequence set forth in SEQ ID NO. 25 and has a length of no more than 21 nucleotides, and the antisense strand comprises at least 15 contiguous nucleotides in the nucleotide sequence set forth in SEQ ID NO. 26 and has a length of no more than 23 nucleotides; or, the sense strand comprises at least 15 contiguous nucleotides in the nucleotide sequence set forth in SEQ ID NO. 27 and has a length of no more than 21 nucleotides, and the antisense strand comprises at least 15 contiguous nucleotides in the nucleotide sequence set forth in SEQ ID NO. 28 and has a length of no more than 23 nucleotides; the sense strand or the antisense strand is optionally modified.

In some embodiments, the present application provides a double-stranded ribonucleic acid (dsRNA), a pharmaceutically acceptable salt thereof, or a ligand conjugate thereof, wherein the dsRNA comprises a sense strand and an antisense strand, wherein the sense strand comprises the nucleotide sequence set forth in SEQ ID NO. 1 and has a length of no more than 21 nucleotides, and the antisense strand comprises the nucleotide sequence set forth in SEQ ID NO. 2 and has a length of no more than 23 nucleotides; the sense strand comprises the nucleotide sequence set forth in SEQ ID NO. 3 and has a length of no more than 21 nucleotides, and the antisense strand comprises the nucleotide sequence set forth in SEQ ID NO. 4 and has a length of no more than 23 nucleotides; the sense strand comprises the nucleotide sequence set forth in SEQ ID NO. 5 and has a length of no more than 21 nucleotides, and the antisense strand comprises the nucleotide sequence set forth in SEQ ID NO. 6 and has a length of no more than 23 nucleotides; the sense strand comprises the nucleotide sequence set forth in SEQ ID NO. 7 and has a length of no more than 21 nucleotides, and the antisense strand comprises the nucleotide sequence set forth in SEQ ID NO. 8 and has a length of no more than 23 nucleotides; the sense strand comprises the nucleotide sequence set forth in SEQ ID NO. 9 and has a length of no more than 21 nucleotides, and the antisense strand comprises the nucleotide sequence set forth in SEQ ID NO. 10 and has a length of no more than 23 nucleotides; the sense strand comprises the nucleotide sequence set forth in SEQ ID NO. 11 and has a length of no more than 21 nucleotides, and the antisense strand comprises the nucleotide sequence set forth in SEQ ID NO. 12 and has a length of no more than 23 nucleotides; the sense strand comprises the nucleotide sequence set forth in SEQ ID NO. 13 and has a length of no more than 21 nucleotides, and the antisense strand comprises the nucleotide sequence set forth in SEQ ID NO. 14 and has a length of no more than 23 nucleotides; the sense strand comprises the nucleotide sequence set forth in SEQ ID NO. 15 and has a length of no more than 21 nucleotides, and the antisense strand comprises the nucleotide sequence set forth in SEQ ID NO. 16 and has a length of no more than 23 nucleotides; the sense strand comprises the nucleotide sequence set forth in SEQ ID NO. 17 and has a length of no more than 21 nucleotides, and the antisense strand comprises the nucleotide sequence set forth in SEQ ID NO. 18 and has a length of no more than 23 nucleotides; the sense strand comprises the nucleotide sequence set forth in SEQ ID NO. 19 and has a length of no more than 21 nucleotides, and the antisense strand comprises the nucleotide sequence set forth in SEQ ID NO. 20 and has a

length of no more than 23 nucleotides; the sense strand comprises the nucleotide sequence set forth in SEQ ID NO. 21 and has a length of no more than 21 nucleotides, and the antisense strand comprises the nucleotide sequence set forth in SEQ ID NO. 22 and has a length of no more than 23 nucleotides; the sense strand comprises the nucleotide sequence set forth in SEQ ID NO. 23 and has a length of no more than 21 nucleotides, and the antisense strand comprises the nucleotide sequence set forth in SEQ ID NO. 24 and has a length of no more than 23 nucleotides; the sense strand comprises the nucleotide sequence set forth in SEQ ID NO. 25 and has a length of no more than 21 nucleotides, and the antisense strand comprises the nucleotide sequence set forth in SEQ ID NO. 26 and has a length of no more than 23 nucleotides; or, the sense strand comprises the nucleotide sequence set forth in SEQ ID NO. 27 and has a length of no more than 21 nucleotides, and the antisense strand comprises the nucleotide sequence set forth in SEQ ID NO. 28 and has a length of no more than 23 nucleotides; the sense strand or the antisense strand is optionally modified.

In some embodiments, the present application provides a double-stranded ribonucleic acid (dsRNA), a pharmaceutically acceptable salt thereof, or a ligand conjugate thereof, wherein the dsRNA comprises a sense strand and an antisense strand, wherein the sense strand is the nucleotide sequence set forth in SEQ ID NO. 1, and the antisense strand is the nucleotide sequence set forth in SEQ ID NO. 2; the sense strand is the nucleotide sequence set forth in SEQ ID NO. 3, and the antisense strand is the nucleotide sequence set forth in SEQ ID NO. 4; the sense strand is the nucleotide sequence set forth in SEQ ID NO. 5, and the antisense strand is the nucleotide sequence set forth in SEQ ID NO. 6; the sense strand is the nucleotide sequence set forth in SEQ ID NO. 7, and the antisense strand is the nucleotide sequence set forth in SEQ ID NO. 8; the sense strand is the nucleotide sequence set forth in SEQ ID NO. 9, and the antisense strand is the nucleotide sequence set forth in SEQ ID NO. 10; the sense strand is the nucleotide sequence set forth in SEQ ID NO. 11, and the antisense strand is the nucleotide sequence set forth in SEQ ID NO. 12; the sense strand is the nucleotide sequence set forth in SEQ ID NO. 13, and the antisense strand is the nucleotide sequence set forth in SEQ ID NO. 14; the sense strand is the nucleotide sequence set forth in SEQ ID NO. 15, and the antisense strand is the nucleotide sequence set forth in SEQ ID NO. 16; the sense strand is the nucleotide sequence set forth in SEQ ID NO. 17, and the antisense strand is the nucleotide sequence set forth in SEQ ID NO. 18; the sense strand is the nucleotide sequence set forth in SEQ ID NO. 19, and the antisense strand is the nucleotide sequence set forth in SEQ ID NO. 20; the sense strand is the nucleotide sequence set forth in SEQ ID NO. 21, and the antisense strand is the nucleotide sequence set forth in SEQ ID NO. 22; the sense strand is the nucleotide sequence set forth in SEQ ID NO. 23, and the antisense strand is the nucleotide sequence set forth in SEQ ID NO. 24; the sense strand is the nucleotide sequence set forth in SEQ ID NO. 25, and the antisense strand is the nucleotide sequence set forth in SEQ ID NO. 26; or, the sense strand is the nucleotide sequence set forth in SEQ ID NO. 27, and the antisense strand is the nucleotide sequence set forth in SEQ ID NO. 28; the sense strand or the antisense strand is optionally modified.

The present application further provides a dsRNA, a pharmaceutically acceptable salt thereof, or a ligand conjugate thereof, wherein the dsRNA comprises a sense strand and an antisense strand; the sense strand and the antisense strand have at least 90%, 91%, 92%, 93%, 94%, 95%, 96%, 97%, 98%, or 99% sequence identity to the full length of the nucleotide sequences of the sense strands and the antisense strands described above, respectively.

In some embodiments, the dsRNA, the pharmaceutically acceptable salt thereof, or the ligand conjugate thereof of the present application is an RNAi drug.

In some embodiments, the dsRNA, the pharmaceutically acceptable salt thereof, or the ligand conjugate thereof of the present application is an siRNA, or a ligand conjugate thereof, or a pharmaceutically acceptable salt thereof.

In a specific embodiment, the dsRNA, the pharmaceutically acceptable salt thereof, or the ligand conjugate thereof has a double-stranded region formed by the complementation of the sense strand and the antisense strand, and optionally an overhang at the 5' end and/or the 3' end of the sense strand and/or the antisense strand. In a preferred embodiment, the dsRNA, the pharmaceutically acceptable salt thereof, or the ligand conjugate thereof has a double-stranded region formed by the complementation of the sense strand and the antisense strand, and an overhang at the 5' end and/or the 3' end of the sense strand and/or the antisense strand. In a preferred embodiment, the dsRNA, the pharmaceutically acceptable salt thereof, or the ligand conjugate thereof has a double-stranded region formed by the complementation of the sense strand and the antisense strand, and an overhang at the 3' end of the antisense strand. In a preferred embodiment, the dsRNA, the pharmaceutically acceptable salt thereof, or the ligand conjugate thereof has a double-stranded region formed by the complementation of the sense strand and the antisense strand, and an overhang at the 3' end of the sense strand.

In some embodiments, the sense strand comprises a nucleic acid sequence differing by 0, 1, 2, or 3 nucleotides from a nucleotide sequence of the nucleotide sequences as shown in Table 1, and the sense strand has a length of no more than 21 nucleotides; the antisense strand comprises a nucleic acid sequence differing by 0, 1, 2, or 3 nucleotides from a nucleotide sequence of the nucleotide sequences as shown in Table 2, and the antisense strand has a length of no more than 23 nucleotides.

In some embodiments, the sense strand is selected from a nucleic acid sequence differing by 0, 1, 2, or 3 nucleotides from a nucleotide sequence of the nucleotide sequences as shown in Table 1; the antisense strand is selected from a nucleotide sequence differing by 0, 1, 2, or 3 nucleotides from a nucleotide sequence of the nucleotide sequences

as shown in Table 2.

In some embodiments, the present application provides a ligand conjugate of the double-stranded ribonucleic acid (dsRNA), which comprises the double-stranded ribonucleic acid (dsRNA) or the pharmaceutically acceptable salt thereof described in the present application, and a ligand.

## Double-Stranded Region

In some embodiments, the double-stranded region of the dsRNA has a length of 15-23 nucleotide pairs; for example, the double-stranded region of the dsRNA has a length of 15, 16, 17, 18, 19, 20, 21, 22, or 23 nucleotide pairs.

In some embodiments, the double-stranded region of the dsRNA has a length of 19-23 nucleotide pairs; for example, the double-stranded region of the dsRNA has a length of 19, 20, 21, 22, or 23 nucleotide pairs.

## Overhang

In some embodiments, the sense strand or the antisense strand optionally comprises an overhang at the 5' end and/or the 3' end.

In some embodiments, the overhang comprises 1, 2, 3, 4, or 5 nucleotides. In some embodiments, the overhang comprises 1 or 2 nucleotides.

In some embodiments, the sense strand optionally comprises an overhang at the 5' end and/or the 3' end. In some embodiments, the sense strand optionally comprises an overhang of 1, 2, 3, 4, or 5 nucleotides at the 5' end and/or the 3' end. In some embodiments, the sense strand optionally comprises an overhang of 1 or 2 nucleotides at the 5' end and/or the 3' end.

In some embodiments, the antisense strand optionally comprises an overhang at the 5' end and/or the 3' end. In some embodiments, the antisense strand optionally comprises an overhang of 1, 2, 3, 4, or 5 nucleotides at the 5' end and/or the 3' end. In some embodiments, the antisense strand optionally comprises an overhang of 1 or 2 nucleotides at the 5' end and/or the 3' end.

In some embodiments, the overhang is selected from the group consisting of unmodified or modified A, G, C, U, and T.

In a specific embodiment, the overhang may have 1, 2, 3, 4, or 5 nucleotides, preferably 1 or 2 nucleotides, at the 5' end and/or the 3' end of the sense strand or the antisense strand. In a specific embodiment, the dsRNA, the pharmaceutically acceptable salt thereof, or the ligand conjugate thereof comprises an additional sequence as the overhang, and the additional sequence may comprise 1, 2, 3, 4, or 5 nucleotides, preferably 1 or 2 nucleotides, linked to the 5' end and/or the 3' end of the sense strand or the antisense strand.

For example, in the case where the ribonucleotides at the 5' end and/or the 3' end of the sense strand or the antisense strand serve as the overhang: when the sense strand has 21 nucleotides, the antisense strand has 23 nucleotides, and positions 1-21 of the sense strand are complementary to positions 1-21 of the antisense strand (in the direction from the 5' end to the 3' end), the nucleotides at positions 22-23 of the antisense strand constitute the overhang located at the 3' end of the antisense strand, that is, the 3' end of the sense strand is a blunt end; when the sense strand has 21 nucleotides, the antisense strand has 21 nucleotides, and positions 1-20 of the sense strand are complementary to positions 1-20 of the antisense strand (in the direction from the 5' end to the 3' end), the nucleotide at position 21 of the sense strand and the nucleotide at position 21 of the antisense strand constitute the overhang at the 3' end of the sense strand and the overhang at the 3' end of the antisense strand, respectively; when the sense strand has 21 nucleotides, the antisense strand has 23 nucleotides, and positions 1-21 of the sense strand are complementary to positions 3-23 of the antisense strand (in the direction from the 5' end to the 3' end), the nucleotides at positions 1-2 of the antisense strand constitute the overhang located at the 5' end of the antisense strand, that is, the 5' end of the sense strand is a blunt end.

In some embodiments, in the case where the nucleotides at the 5' end and/or the 3' end of the sense strand or the antisense strand serve as the overhang, the antisense strand optionally comprises an overhang at the 5' end and/or the 3' end, and the overhang is selected from the group consisting of unmodified or modified ribonucleotide sequences CG, AA, GA, CC, CA, AC, GC, AA, UG, GG, CU, UU, and UC. In some embodiments, the antisense strand optionally comprises an overhang at the 3' end, and the overhang is selected from the group consisting of unmodified or modified ribonucleotide sequences CG, AA, GA, CC, CA, AC, GC, AA, UG, GG, CU, UU, and UC. In a specific embodiment, the dsRNA, the pharmaceutically acceptable salt thereof, or the ligand conjugate thereof comprises an additional sequence at the 5' end and/or the 3' end of the antisense strand or the sense strand as the overhang or a portion of the overhang, and the additional sequence may comprise **1,** 2, 3, 4, or 5 nucleosides, preferably 1 or 2 nucleotides, at the 5' end and/or the 3' end. In some embodiments, the overhang consists of nucleotides at the 5' end and/or the 3' end of the antisense strand or the sense strand and an additional sequence, or the overhang may consist of only an additional sequence.

In some embodiments, the overhang is an additional sequence optionally located at the 5' end and/or the 3' end of the antisense strand, and the overhang is selected from the group consisting of unmodified or modified CG, AA, GA, CC,

CA, AC, GC, AA, UG, GG, CU, UU, and UC. In some embodiments, the overhang is an additional sequence located at the 3' end of the antisense strand, and the overhang is selected from the group consisting of unmodified or modified CG, AA, GA, CC, CA, AC, GC, AA, UG, GG, CU, UU, and UC.

In some embodiments, the additional sequence in the overhang is selected from the group consisting of unmodified or modified U and T; for example, the additional sequence may be selected from the group consisting of uu and dTdT.

In some embodiments, when the overhang has 1 nucleotide, the additional sequence as the overhang may be selected from the group consisting of unmodified or modified U and T. In some embodiments, when the overhang has 1 nucleotide, the additional sequence as the overhang may be selected from the group consisting of 2'-O-methyl-modified U (hereinafter referred to as u) and deoxythymine nucleotide (hereinafter referred to as dT).

In some embodiments, when the overhang has 2 nucleotides, the additional sequence as the overhang may be selected from the group consisting of unmodified or modified UU and TT. In some embodiments, when the overhang has 2 nucleotides, the additional sequence as the overhang may be selected from the group consisting of uu and dTdT.

In some embodiments, the sense strand optionally comprises an overhang at the 5' end and/or the 3' end, and the overhang is selected from the group consisting of uu and dTdT.

In some embodiments, the antisense strand optionally comprises an overhang at the 5' end and/or the 3' end, and the overhang is selected from the group consisting of uu and dTdT.

In some embodiments, the overhang is linked to an adjacent nucleotide via a phosphate group or a phosphorothioate group.

In some embodiments, one or more nucleotides in the overhang are linked via a phosphate group or a phosphorothioate group.

## Modification

In some embodiments, the sense strand or the antisense strand is optionally modified.

In some embodiments, one or more nucleotides of the sense strand or the antisense strand are modified.

In some embodiments, each nucleotide may be modified by the same or different modifications, and the modifications may include, but are not limited to, one or more changes in one or two of non-linked phosphate oxygens and/or one or more of linked phosphate oxygens; changes in components of ribose (e.g., 2' hydroxyl in ribose); total replacement of the phosphate moiety with a "dephosphorylation" linker; modification of bases; and modification of the ribose-phosphate backbone.

In some embodiments, before each nucleotide modification, the base is selected from the group consisting of naturally occurring bases, e.g., A, U, C, G, and T, and the modification occurring on a nucleotide base refers to a chemical modification on the basis of the backbone of A, U, C, G, and T.

In some embodiments, 1, 2, 3, 4, 5, 6, 7, 8, 9, 10, 11, 12, 13, 14, 15, 16, 17, 18, 19, 20, or 21 nucleotides of the sense strand are modified; or a number of nucleotides within a range formed by any of the aforementioned values, e.g., 1-21, 1-20, 1-19, 1-18, 1-17, 1-16, 1-15, 1-14, 1-13, 1-12, 1-11, or 1-10 nucleotides, are modified.

In some embodiments, 1 or more, 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, 19 or more, 20 or more, or 21 or more nucleotides of the sense strand are modified; typically, 10 or more, 11 or more, 12 or more, 13 or more, 14 or more, 15 or more, 16 or more, 17 or more, 18 or more, 19 or more, 20 or more, or 21 or more nucleotides of the sense strand are modified.

In some embodiments, all nucleotides of the sense strand are modified.

In some embodiments, 1, 2, 3, 4, 5, 6, 7, 8, 9, 10, 11, 12, 13, 14, 15, 16, 17, 18, 19, 20, 21, 22, or 23 nucleotides of the antisense strand are modified; or a number of nucleotides within a range formed by any of the aforementioned values, e.g., 1-23, 1-22, 1-21, 1-20, 1-19, 1-18, 1-17, 1-16, 1-15, 1-14, 1-13, 1-12, 1-11, or 1-10 nucleotides, are modified.

In some embodiments, 1 or more, 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, 19 or more, 20 or more, 21 or more, 22 or more, or 23 or more nucleotides of the antisense strand are modified; typically, 10 or more, 11 or more, 12 or more, 13 or more, 14 or more, 15 or more, 16 or more, 17 or more, 18 or more, 19 or more, 20 or more, 21 or more, 22 or more, or 23 or more nucleotides of the antisense strand are modified. In some embodiments, all nucleotides of the antisense strand are modified.

In some embodiments, all nucleotides of the sense strand and all nucleotides of the antisense strand are modified.

In some embodiments, the modification is selected from the group consisting of glycosyl modification of nucleotides, modification of bases, modification of linkages between nucleotides, and terminal modification. In some embodiments, the modification is selected from the group consisting of glycosyl modification of nucleotides, modification of linkages between nucleotides, and terminal modification.

In some embodiments, the glycosyl modification of nucleotides is selected from the group consisting of dehydroxylation, fluorination, amination, alkylation, hydroxyalkylation, and hydroxyalkenylation.

In some embodiments, the glycosyl modification of nucleotides occurs at the 2' position of the glycosyl.

In some embodiments, the glycosyl modification is selected from the group consisting of 2'-dehydroxylation, 2'-fluoro, 2'-amino, 2'-alkyl, 2'-O-alkyl, 2'-O-ether, and 2'-O-alkenyl. In some embodiments, the glycosyl modification is selected from the group consisting of 2'-dehydroxylation, 2'-fluoro, 2'-amino, 2'-methyl, 2'-ethyl, 2'-methyl-O-methyl, 2'-ethyl-O-methyl, 2'-O-methyl, 2'-O-ethyl, 2'-O-ethyl-O-methyl, and 2'-O-allyl.

In some embodiments, the modification of linkages between nucleotides is selected from the group consisting of phosphorothioate (PS), phosphorodithioate (PS2), methylphosphonate (MP), methoxypropylphosphonate (MOP), and aminophosphonate. In some embodiments, the modification of linkages between nucleotides is selected from phosphorothioate (PS). In some embodiments, the nucleotides are linked via phosphorodithioate bonds.

In some embodiments, the terminal modification is selected from a modification on a phosphate group or a hydroxyl group at the terminus. In some embodiments, the terminal modification is selected from the group consisting of a 5'-end modification and a 3'-end modification. In some embodiments, the terminal modification is selected from the group consisting of 5'-phosphate, 5'-methylphosphonate (5'-MP), 5'-phosphorothioate (5'-PS), and 5'-(E)-vinylphosphonate (5'-(E)-VP) on the ribosyl group of the nucleotide at the terminus. In some embodiments, the terminal modification is selected from the group consisting of 5'-phosphate and 5'-(E)-vinylphosphate on the ribosyl group of the nucleotide at the terminus.

In some embodiments, the modification is selected from the group consisting of 2'-dehydroxylation, 2'-fluoro, 2'-amino, 2'-methyl, 2'-ethyl, 2'-methyl-O-methyl, 2'-ethyl-O-methyl, 2'-O-methyl, 2'-O-ethyl, 2'-O-allyl, phosphorothioate, methylphosphonate, aminophosphonate, 5'-phosphate, 3'-phosphate, 5'-(E)-vinylphosphate, and 3'-(E)-vinylphosphate.

In some embodiments, the modification is selected from the group consisting of 2'-dehydroxylation, 2'-fluoro, 2'-O-methyl, 2'-O-ethyl, 2'-O-ethyl-O-methyl, 2'-O-allyl, phosphorothioate, and 5'-(E)-vinylphosphate.

In some embodiments, the modification is selected from the group consisting of 2'-fluoro, 2'-O-methyl, phosphorothioate, and 5'-(E)-vinylphosphate.

As will be described in detail below, when referring to the nucleotide at position n of the sense strand and the antisense strand, the numbering starts from the 5' end unless specifically indicated that it starts from the 3' end.

In some embodiments, a phosphorothioate modification is present between the first two nucleotides and/or between the first three nucleotides at one or both of the two ends of the two nucleic acid strands of the dsRNA, the pharmaceutically acceptable salt thereof, or the ligand conjugate thereof. For example, taking the sense strand as an example, a phosphorothioate modification is present between the nucleotides at positions 1 and 2 of the sense strand and/or between the nucleotides at positions 2 and 3 of the sense strand.

In some embodiments, in the case where no additional sequence serves as the overhang, the phosphorothioate modification is present between the nucleotides at positions 1 and 2 of the sense strand and/or between the nucleotides at positions 2 and 3 of the sense strand.

In some embodiments, in the case where no additional sequence serves as the overhang, the phosphorothioate modification is present between the nucleotides at positions 1 and 2 of the antisense strand, between the nucleotides at positions 2 and 3 of the antisense strand, between the nucleotides at positions 1 and 2 of the 3' end of the antisense strand, and/or between the nucleotides at positions 2 and 3 of the 3' end of the antisense strand. In some embodiments, in the case where no additional sequence serves as the overhang, the phosphorothioate modification is present between the nucleotides at positions 1 and 2 of the antisense strand, between the nucleotides at positions 2 and 3 of the antisense strand, between the nucleotides at positions 19 and 20 of the antisense strand, and/or between the nucleotides at positions 20 and 21 of the antisense strand.

In some embodiments, when an additional sequence is present as the overhang, the phosphorothioate modification may be located between nucleotides in the additional sequence.

In some embodiments, the nucleotide at the 5'-end of the antisense strand is modified by 5'-(E)-vinylphosphate. In some embodiments, when an additional sequence is present as the overhang, the 5'-end modification may be present on a nucleotide of the additional sequence.

In some embodiments, 2, 3, 4, or 5 nucleotides of the sense strand are modified by 2'-fluoro. In some embodiments, 14, 15, 16, or 17 nucleotides of the sense strand are modified by 2'-O-methyl. In some embodiments, in the sense strand, 3 or 4 nucleotides are modified by 2'-fluoro, and 15, 16, 17, or 18 nucleotides are modified by 2'-O-methyl. In some embodiments, 0, 1, or 2 nucleotides of the nucleotides at positions 1 to 6 of the sense strand are modified by 2'-fluoro.

In some embodiments, 4, 5, or 6 nucleotides of the nucleotides at positions 1 to 6 of the sense strand are modified by 2'-O-methyl.

In some embodiments, 2 or 3 nucleotides of the nucleotides at positions 7 to 9 of the sense strand are modified by 2'-fluoro.

In some embodiments, 0 or 1 nucleotide of the nucleotides at positions 7 to 9 of the sense strand is modified by 2'-O-methyl.

In some embodiments, 0 or 1 nucleotide of the nucleotides at positions 10 to 19 of the sense strand is modified by 2'-fluoro.

In some embodiments, 8, 9, or 10 nucleotides of the nucleotides at positions 10 to 19 of the sense strand are modified by 2'-O-methyl.

In some embodiments, the nucleotides at positions 7, 8, and 9 of the sense strand are modified by 2'-fluoro, and 0, 1, or 2 nucleotides of the nucleotides at positions 1 to 6 and/or 10 to 19 of the sense strand are also modified by 2'-fluoro. In some embodiments, the nucleotides at positions 7, 8, and 9 of the sense strand are modified by 2'-fluoro, and 0, 1, or 2 nucleotides of the nucleotides at positions 1 to 6 and 10 to 19 of the sense strand are also modified by 2'-fluoro. In some embodiments, the nucleotides at positions 7, 8, and 9 of the sense strand are modified by 2'-fluoro, and 0 or 1 nucleotide of the nucleotides at positions 1 to 6 and 10 to 19 of the sense strand are modified by 2'-fluoro. In some embodiments, 2, 3, 4, 5, 6, or 7 nucleotides of the antisense strand are modified by 2'-fluoro.

In some embodiments, 14, 15, 16, 17, 18, 19, 20, or 21 nucleotides of the antisense strand are modified by 2'-O-methyl.

In some embodiments, 3, 4, 5, or 6 nucleotides of the nucleotides at positions 2, 6, 8, 9, 14, and 16 of the antisense strand are modified by 2'-fluoro. In some embodiments, 4 nucleotides of the nucleotides at positions 2, 6, 14, and 16 of the antisense strand are modified by 2'-fluoro. 6 nucleotides of the nucleotides at positions 2, 6, 8, 9, 14, and 16 of the antisense strand are modified by 2'-fluoro.

In some embodiments, the nucleotides at positions 2, 6, 14, and 16 of the antisense strand are modified by 2'-fluoro, and any one or two of the nucleotides at positions 1, 3 to 5, 7 to 13, 15, and/or 17 to 21 of the antisense strand are also modified by 2'-fluoro. In some embodiments, the nucleotides at positions 2, 6, 14, and 16 of the antisense strand are modified by 2'-fluoro, and any one or two of the nucleotides at positions 1, 3 to 5, 7 to 13, 15, and 17 to 21 of the antisense strand are also modified by 2'-fluoro.

In some embodiments, 14, 15, 16, 17, 18, or 19 nucleotides of the nucleotides at positions 1, 3 to 5, 7 to 13, 15, and/or 17 to 21 of the antisense strand are modified by 2'-O-methyl.

In one specific embodiment, the nucleotides at positions 5, 7, 8, and 9 of the sense strand are modified by 2'-fluoro, and the other ribonucleotides of the sense strand are modified by 2'-O-methyl; and the nucleotides at positions 2, 6, 8, 9, 14, and 16 of the antisense strand are modified by 2'-fluoro, and the other ribonucleotides of the antisense strand are modified by 2'-O-methyl.

In one specific embodiment, the nucleotides at positions 5, 7, 8, and 9 of the sense strand are modified by 2'-fluoro, and the other ribonucleotides of the sense strand are modified by 2'-O-methyl; and the nucleotides at positions 2, 6, 14, and 16 of the antisense strand are modified by 2'-fluoro, and the other ribonucleotides of the antisense strand are modified by 2'-O-methyl.

In one specific embodiment, the nucleotides at positions 7, 8, and 9 of the sense strand are modified by 2'-fluoro, and the other ribonucleotides of the sense strand are modified by 2'-O-methyl; and the nucleotides at positions 2, 6, 14, and 16 of the antisense strand are modified by 2'-fluoro, and the other ribonucleotides of the antisense strand are modified by 2'-O-methyl.

In one specific embodiment, the nucleotides at positions 7, 8, and 9 of the sense strand are modified by 2'-fluoro, and the other ribonucleotides of the sense strand are modified by 2'-O-methyl; and the nucleotides at positions 2, 6, 8, 9, 14, and 16 of the antisense strand are modified by 2'-fluoro, and the other ribonucleotides of the antisense strand are modified by 2'-O-methyl. In one specific embodiment, the nucleotides at positions 7, 8, and 9 of the sense strand are modified by 2'-fluoro, and the other ribonucleotides of the sense strand are modified by 2'-O-methyl; and the nucleotides at positions 2, 6, 14, and 16 of the antisense strand are modified by 2'-fluoro, and the other ribonucleotides of the antisense strand are modified by 2'-O-methyl. In one specific embodiment, the nucleotides at positions 7, 8, and 9 of the sense strand are modified by 2'-fluoro, and the other ribonucleotides of the sense strand are modified by 2'-O-methyl; and the antisense strand comprises 2'-fluoro (e.g., at position 14, etc.) and 2'-O-methyl modifications, and optionally further comprises one or more other types of modifications.

In one specific embodiment, the nucleotides at positions 5, 7, 8, and 9 of the sense strand are modified by 2'-fluoro, and the other ribonucleotides of the sense strand are modified by 2'-O-methyl; and the nucleotides at positions 2, 6, 8, 9, 14, and 16 of the antisense strand are modified by 2'-fluoro, and the other ribonucleotides of the antisense strand are modified by 2'-O-methyl; and a phosphorothioate modification is present between the nucleotides at positions 1 and 2 of the sense strand, between the nucleotides at positions 2 and 3 of the sense strand, between the nucleotides at positions 1 and 2 of the antisense strand, between the nucleotides at positions 2 and 3 of the antisense strand, between the nucleotides at positions 19 and 20 of the antisense strand, and/or between the nucleotides at positions 20 and 21 of the antisense strand. Optionally, the 5'-end of the antisense strand is modified by *5'-(E)*-vinylphosphate; for example, the ribonucleotide at the 5'-end of the antisense strand is modified by *5'-(E)*-vinylphosphate.

In one specific embodiment, the nucleotides at positions 5, 7, 8, and 9 of the sense strand are modified by 2'-fluoro, and the other ribonucleotides of the sense strand are modified by 2'-O-methyl; and the nucleotides at positions 2, 6,

14, and 16 of the antisense strand are modified by 2'-fluoro, and the other ribonucleotides of the antisense strand are modified by 2'-O-methyl; and a phosphorothioate modification is present between the nucleotides at positions 1 and 2 of the sense strand, between the nucleotides at positions 2 and 3 of the sense strand, between the nucleotides at positions 1 and 2 of the antisense strand, between the nucleotides at positions 2 and 3 of the antisense strand, between the nucleotides at positions 19 and 20 of the antisense strand, and/or between the nucleotides at positions 20 and 21 of the antisense strand. Optionally, the 5'-end of the antisense strand is modified by 5'-(E)-vinylphosphate; for example, the ribonucleotide at the 5'-end of the antisense strand is modified by 5'-(E)-vinylphosphate.

In one specific embodiment, the nucleotides at positions 7, 8, and 9 of the sense strand are modified by 2'-fluoro, and the other ribonucleotides of the sense strand are modified by 2'-O-methyl; the nucleotides at positions 2, 6, 14, and 16 of the antisense strand are modified by 2'-fluoro, and the other ribonucleotides of the antisense strand are modified by 2'-O-methyl; and a phosphorothioate modification is present between the nucleotides at positions 1 and 2 of the sense strand, between the nucleotides at positions 2 and 3 of the sense strand, between the nucleotides at positions 1 and 2 of the antisense strand, between the nucleotides at positions 2 and 3 of the antisense strand, between the nucleotides at positions 19 and 20 of the antisense strand, and/or between the nucleotides at positions 20 and 21 of the antisense strand. Optionally, the 5'-end of the antisense strand is modified by 5'-(E)-vinylphosphate; for example, the ribonucleotide at the 5'-end of the antisense strand is modified by 5'-(E)-vinylphosphate.

In one specific embodiment, the nucleotides at positions 7, 8, and 9 of the sense strand are modified by 2'-fluoro, and the other ribonucleotides of the sense strand are modified by 2'-O-methyl; the nucleotides at positions 2, 6, 8, 9, 14, and 16 of the antisense strand are modified by 2'-fluoro, and the other ribonucleotides of the antisense strand are modified by 2'-O-methyl; and a phosphorothioate modification is present between the nucleotides at positions 1 and 2 of the sense strand, between the nucleotides at positions 2 and 3 of the sense strand, between the nucleotides at positions 1 and 2 of the antisense strand, between the nucleotides at positions 2 and 3 of the antisense strand, between the nucleotides at positions 19 and 20 of the antisense strand, and/or between the nucleotides at positions 20 and 21 of the antisense strand. Optionally, the 5'-end of the antisense strand is modified by 5'-(E)-vinylphosphate; for example, the ribonucleotide at the 5'-end of the antisense strand is modified by 5'-(E)-vinylphosphate.

## Ligand

In some embodiments, the ligand comprises a branched group and a linker. In some embodiments, the branched group may comprise a targeting group.

In some embodiments, the ligand is linked to the dsRNA via the linker. Herein, a dsRNA to which a ligand is linked is referred to as a dsRNA ligand conjugate.

In some embodiments, the ligand is linked to one or more targeting groups via the branched group. In some embodiments, the branched group comprises the targeting group.

In some embodiments, the ligand comprises at least one targeting group. In some embodiments, the ligand comprises one, two, three, four, or five targeting groups. In some embodiments, the ligand comprises two, three, or four targeting groups. In some embodiments, the ligand comprises three targeting groups.

In some embodiments, the targeting group is selected from a GalNAc group.

In some embodiments, the ligand comprises one, two, three, four, or five GalNAc groups. In some embodiments, the ligand comprises two, three, or four GalNAc groups. In some embodiments, the ligand comprises three GalNAc groups.

In some embodiments, the branched group is selected from the group consisting of:

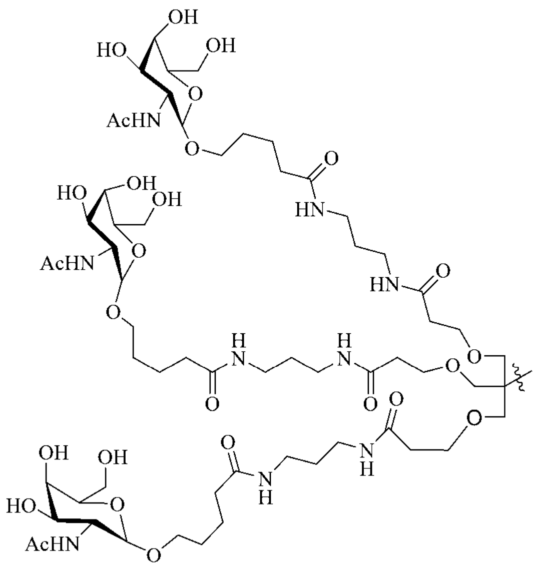

and

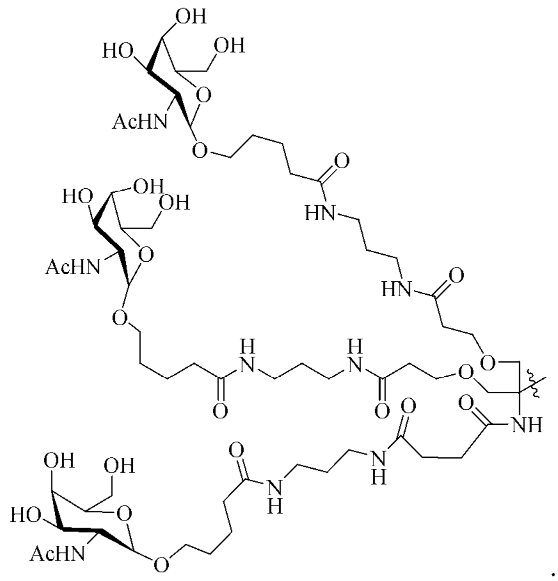

.

In some embodiments, the linker is selected from the group consisting of:

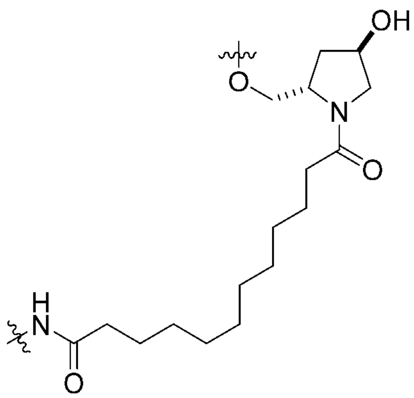

and

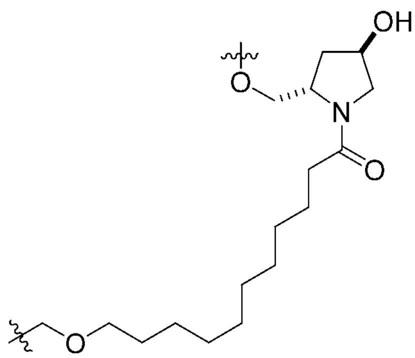

.

In some embodiments, the ligand is selected from the group consisting of:

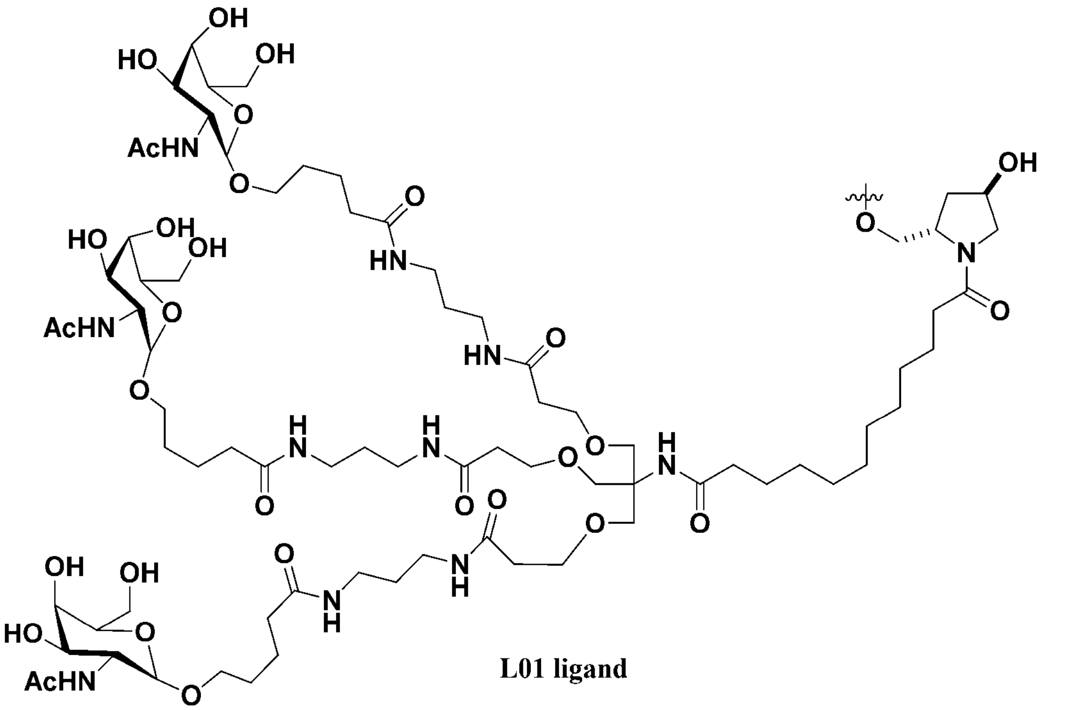

L01 ligand

and

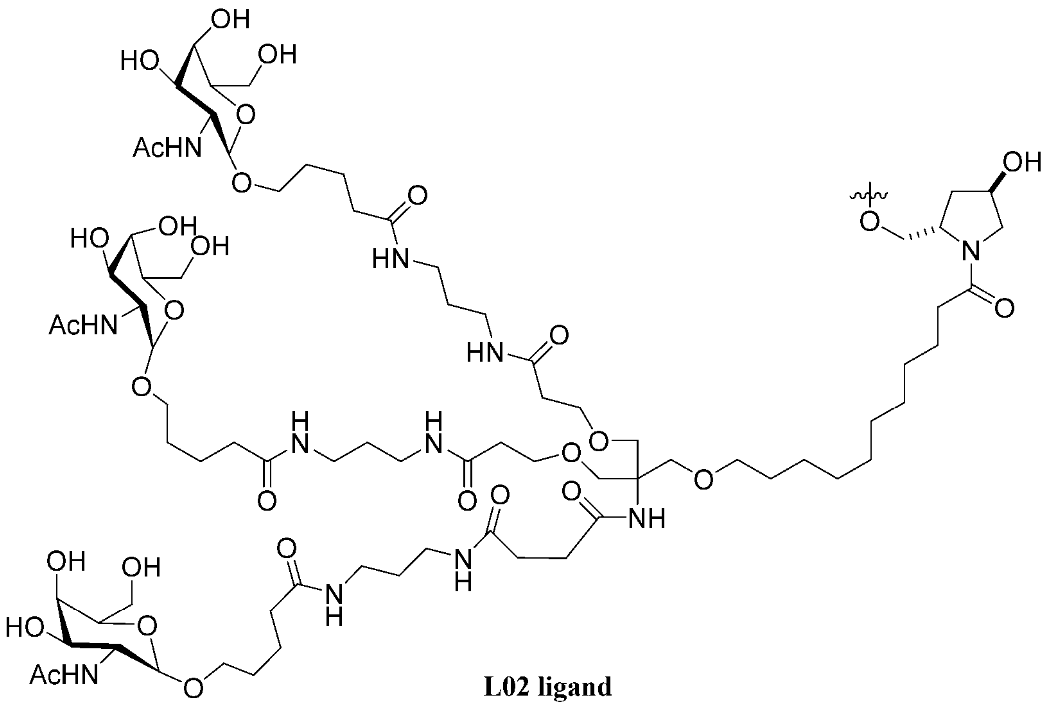

L02 ligand

.

In some embodiments, the ligand is linked to the sense strand or the antisense strand. In some embodiments, the ligand is linked to the 5' or 3' end of the sense strand or the antisense strand. In some embodiments, the ligand is linked to the 5' or 3' end of the sense strand. In some embodiments, the ligand is linked to the 3' end of the sense strand.

In some embodiments, the ligand is linked to the sense strand or the antisense strand of the dsRNA via a phosphate group or a phosphorothioate group. In some embodiments, the ligand is linked to the sense strand via a phosphate group or a phosphorothioate group. In some embodiments, the ligand is linked to the 3' end of the sense strand via a phosphate group or a phosphorothioate group.

In some embodiments, the dsRNA ligand conjugate is as follows:

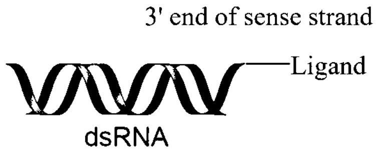


formula 101

In some embodiments, the dsRNA ligand conjugate is selected from the group consisting of:

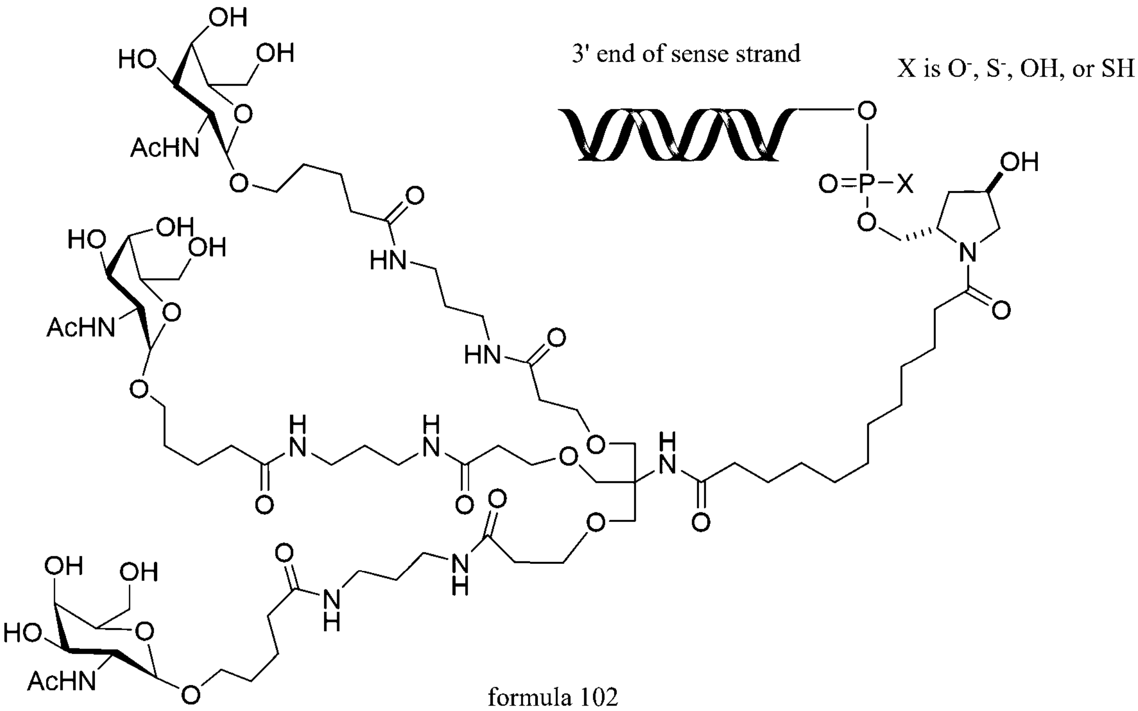


formula 102

and

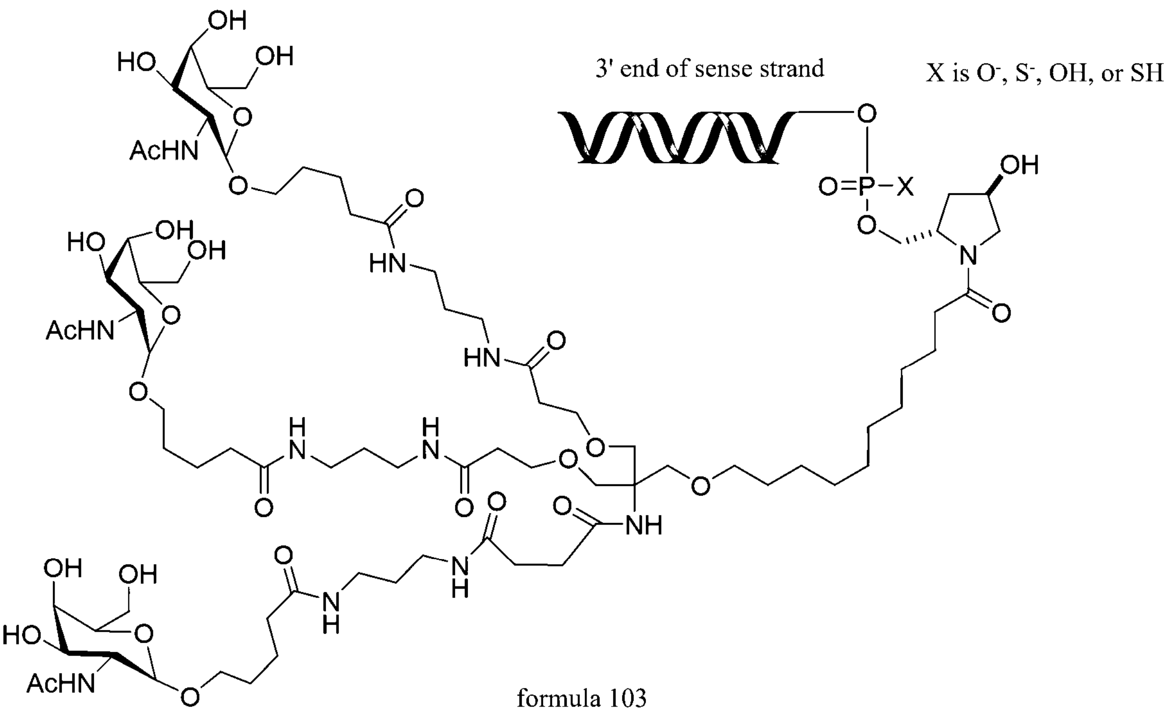


formula 103

In some embodiments, the dsRNA ligand conjugate is selected from the group consisting of:

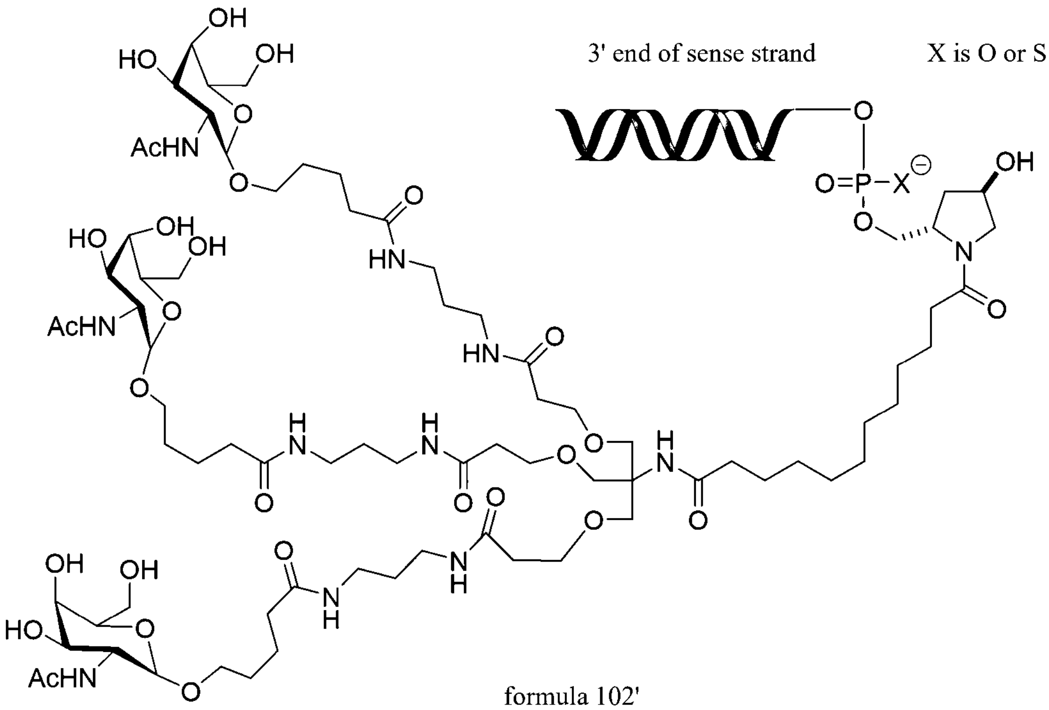

formula 102'

and

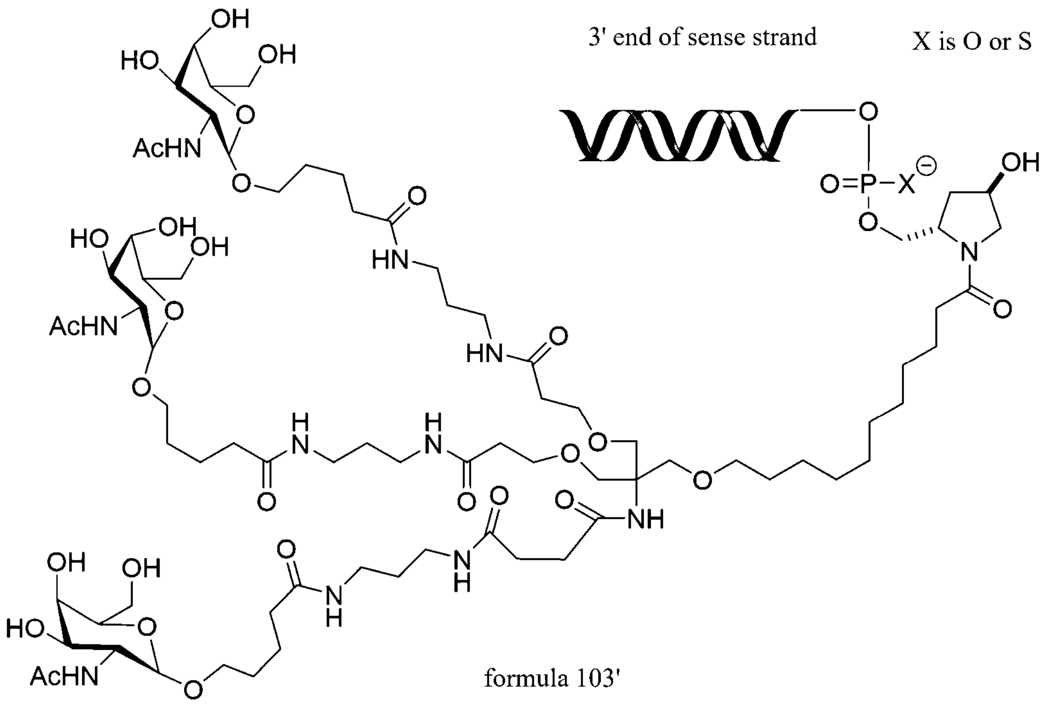

formula 103'

In some embodiments, X is OH or $O^-$.

In some embodiments, the unmodified sense strand of the dsRNA is selected from the group consisting of:

Table 1. Unmodified sense strands

| SEQ ID NO | Sequence in the 5' to 3' direction |
|---|---|
| 1 | GAUCUCCAACUAAAAUACU |

(continued)

| SEQ ID NO | Sequence in the 5' to 3' direction |
|---|---|
| 3 | GGAUGAUUUUCUUAUAUCA |
| 5 | GGUCUUUUCAGGAUGAUUU |
| 7 | GUCUUUUCAGGAUGAUUUU |
| 9 | CUUUUCAGGAUGAUUUUCU |
| 11 | UUUUCAGGAUGAUUUUCUU |
| 13 | UCAGGAUGAUUUUCUUAUA |
| 15 | CAGGAUGAUUUUCUUAUAU |
| 17 | AGGAUGAUUUUCUUAUAUC |
| 19 | GAUGAUUUUCUUAUAUCAA |
| 21 | AUGAUUUUCUUAUAUCAAG |
| 23 | UGAUUUUCUUAUAUCAAGU |
| 25 | CUUAUAUCAAGUGGUACAU |
| 27 | AAACGGAUCUCCAACUAAA |

.

In some embodiments, the unmodified antisense strand of the dsRNA is selected from the group consisting of:

Table 2. Unmodified antisense strands

| SEQ ID NO | Sequence in the 5' to 3' direction |
|---|---|
| 2 | AGUAUUUUAGUUGGAGAUCCG |
| 4 | UGAUAUAAGAAAAUCAUCCUG |
| 6 | AAAUCAUCCUGAAAAGACCUU |
| 8 | AAAAUCAUCCUGAAAAGACCU |
| 10 | AGAAAAUCAUCCUGAAAAGAC |
| 12 | AAGAAAAUCAUCCUGAAAAGA |
| 14 | UAUAAGAAAAUCAUCCUGAAA |
| 16 | AUAUAAGAAAAUCAUCCUGAA |
| 18 | GAUAUAAGAAAAUCAUCCUGA |
| 20 | UUGAUAUAAGAAAAUCAUCCU |
| 22 | CUUGAUAUAAGAAAAUCAUCC |
| 24 | ACUUGAUAUAAGAAAAUCAUC |
| 26 | AUGUACCACUUGAUAUAAGAA |
| 28 | UUUAGUUGGAGAUCCGUUUGA |

In some embodiments, the modified sense strand of the dsRNA is selected from the group consisting of:

Table 3. Modified sense strands

| SEQ ID NO | Sequence in the 5' to 3' direction |
|---|---|
| 29 | gsasucUfcCfAfAfcuaaaauacu |
| 31 | gsasucucCfAfAfcuaaaauacu |
| 33 | gsgsauGfaUfUfUfucuuauauca |

(continued)

| SEQ ID NO | Sequence in the 5' to 3' direction |
|---|---|
| 35 | gsgsaugaUfUfUfucuuauauca |
| 37 | gsgsucUfuUfUfCfaggaugauuu |
| 39 | gsgsucuuUfUfCfaggaugauuu |
| 41 | gsuscuUfuUfCfAfggaugauuuu |
| 43 | gsuscuuuUfCfAfggaugauuuu |
| 45 | csusuuUfcAfGfGfaugauuuucu |
| 47 | csusuuucAfGfGfaugauuuucu |
| 49 | ususuuCfaGfGfAfugauuuucuu |
| 51 | ususuucaGfGfAfugauuuucuu |
| 53 | uscsagGfaUfGfAfuuuucuuaua |
| 55 | usesaggaUfGfAfuuuucuuaua |
| 57 | csasggAfuGfAfUfuuucuuauau |
| 59 | csasggauGfAfUfuuucuuauau |
| 61 | asgsgaUfgAfUfUfuucuuauauc |
| 63 | asgsgaugAfUfUfuucuuauauc |
| 65 | gsasugAfuUfUfUfcuuauaucaa |
| 67 | psasugauUfUfUfcuuauaucaa |
| 69 | asusgaUfuUfUfCfuuauaucaag |
| 71 | asusgauuUfUfCfuuauaucaag |
| 73 | usgsauUfuUfCfUfuauaucaagu |
| 75 | usgsauuuUfCfUfuauaucaagu |
| 77 | csusuaUfaUfCfAfagugguacau |
| 79 | csusuauaUfCfAfaguaguacau |
| 81 | asasacGfgAfUfCfuccaacuaaa |
| 83 | asasacggAfUfCfuccaacuaaa |

In some embodiments, the modified antisense strand of the dsRNA is selected from the group consisting of:

Table 4. Modified antisense strands

| SEQ ID NO | Sequence in the 5' to 3' direction |
|---|---|
| 30 | asGfsuauUfuUfAfguugGfaGfaucscsg |
| 32 | asGfsuauUfuuaguugGfaGfaucscsg |
| 34 | usGfsauaUfaAfGfaaaaUfcAfuccsusg |
| 36 | usGfsauaUfaagaaaaUfcAfuccsusg |
| 38 | asAfsaucAfuCfCfugaaAfaGfaccsusu |
| 40 | asAfsaucAfuccugaaAfaGfaccsusu |
| 42 | asAfsaauCfaUfCfcugaAfaAfgacscsu |
| 44 | asAfsaauCfauccugaAfaAfgacscsu |
| 46 | asGfsaaaAfuCfAfuccuGfaAfaagsasc |
| 48 | asGfsaaaAfucauccuGfaAfaagsasc |

(continued)

| SEQ ID NO | Sequence in the 5' to 3' direction |
|---|---|
| 50 | asAfsgaaAfaUfCfauccUfgAfaaasgsa |
| 52 | asAfsgaaAfaucauccUfgAfaaasgsa |
| 54 | usAfsuaaGfaAfAfaucaUfcCfugasasa |
| 56 | usAfsuaaGfaaaaucaUfcCfugasasa |
| 58 | asUfsauaAfgAfAfaaucAfuCfcugsasa |
| 60 | asUfsauaAfgaaaaucAfuCfcugsasa |
| 62 | gsAfsuauAfaGfAfaaauCfaUfccusgsa |
| 64 | gsAfsuauAfagaaaauCfaUfccusgsa |
| 66 | usUfsgauAfuAfAfgaaaAfuCfaucscsu |
| 68 | usUfsgauAfuaagaaaAfuCfaucscsu |
| 70 | csUfsugaUfaUfAfagaaAfaUfcauscsc |
| 72 | csUfsugaUfauaagaaAfaUfcauscsc |
| 74 | asCfsuugAfuAfUfaagaAfaAfucasusc |
| 76 | asCfsuugAfuauaagaAfaAfucasusc |
| 78 | asUfsguaCfcAfCfuugaUfaUfaagsasa |
| 80 | asUfsguaCfcacuugaUfaUfaagsasa |
| 82 | usUfsuagUfuGfGfagauCfcGfuuusgsa |
| 84 | usUfsuagUfuggagauCfcGfuuusgsa |

The dsRNA of the present application may be formed by comprising any one of the sense strands described above and any one of the antisense strands described above.

For example, the dsRNA comprises any one of the following sense strands: SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 55, SEQ ID NO: 57, SEQ ID NO: 59, SEQ ID NO: 61, SEQ ID NO: 63, SEQ ID NO: 65, SEQ ID NO: 67, SEQ ID NO: 69, SEQ ID NO: 71, SEQ ID NO: 73, SEQ ID NO: 75, SEQ ID NO: 77, SEQ ID NO: 79, SEQ ID NO: 81, and SEQ ID NO: 83; and

the dsRNA comprises any one of the following antisense strands: SEQ ID NO: 2, SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, SEQ ID NO: 48, SEQ ID NO: 50, SEQ ID NO: 52, SEQ ID NO: 54, SEQ ID NO: 56, SEQ ID NO: 58, SEQ ID NO: 60, SEQ ID NO: 62, SEQ ID NO: 64, SEQ ID NO: 66, SEQ ID NO: 68, SEQ ID NO: 70, SEQ ID NO: 72, SEQ ID NO: 74, SEQ ID NO: 76, SEQ ID NO: 78, SEQ ID NO: 80, SEQ ID NO: 82, and SEQ ID NO: 84.

In some embodiments, the dsRNA (unmodified) is selected from the group consisting of:

Table 5. dsRNAs (unmodified)

| dsRNA No. | Sense strand SEQ ID NO | Sequence in the 5' to 3' direction | Antisense strand SEQ ID NO | Sequence in the 5' to 3' direction |
|---|---|---|---|---|
| TD01 | 1 | GAUCUCCAACUAA AAUACU | 2 | AGUAUUUUAGUUGGAG AUCCG |
| TD02 | 3 | GGAUGAUUUUCUU AUAUCA | 4 | UGAUAUAAGAAAAUCA UCCUG |

(continued)

| dsRNA No. | Sense strand SEQ ID NO | Sequence in the 5' to 3' direction | Antisense strand SEQ ID NO | Sequence in the 5' to 3' direction |
|---|---|---|---|---|
| TD03 | 5 | GGUCUUUUCAGGA UGAUUU | 6 | AAAUCAUCCUGAAAAG ACCUU |
| TD04 | 7 | GUCUUUUCAGGAU GAUUUU | 8 | AAAAUCAUCCUGAAAA GACCU |
| TD05 | 9 | CUUUUCAGGAUGA UUUUCU | 10 | AGAAAAUCAUCCUGAA AAGAC |
| TD06 | 11 | UUUUCAGGAUGAU UUUCUU | 12 | AAGAAAAUCAUCCUGA AAAGA |
| TD07 | 13 | UCAGGAUGAUUUU CUUAUA | 14 | UAUAAGAAAAUCAUCC UGAAA |
| TD08 | 15 | CAGGAUGAUUUUC UUAUAU | 16 | AUAUAAGAAAAUCAUC CUGAA |
| TD09 | 17 | AGGAUGAUUUUCU UAUAUC | 18 | GAUAUAAGAAAAUCAU CCUGA |
| TD10 | 19 | GAUGAUUUUCUUA UAUCAA | 20 | UUGAUAUAAGAAAAUC AUCCU |
| TD11 | 21 | AUGAUUUUCUUAU AUCAAG | 22 | CUUGAUAUAAGAAAAU CAUCC |
| TD12 | 23 | UGAUUUUCUUAUA UCAAGU | 24 | ACUUGAUAUAAGAAAA UCAUC |
| TD13 | 25 | CUUAUAUCAAGUG GUACAU | 26 | AUGUACCACUUGAUAU AAGAA |
| TD14 | 27 | AAACGGAUCUCCA ACUAAA | 28 | UUUAGUUGGAGAUCCG UUUGA |

In some embodiments, the dsRNA (modified) is selected from the group consisting of:

Table 6. dsRNAs (modified)

| dsRNA No. | Sense strand SEQ ID NO | Sequence in the 5' to 3' direction | Antisense strand SEQ ID NO | Sequence in the 5' to 3' direction |
|---|---|---|---|---|
| TD01M 01 | 29 | gsasucUfcCfAfAfcuaa aauacu | 30 | asGfsuauUfuUfAfguugGf aGfaucscsg |
| TD01M 02 | 31 | gsasucucCfAfAfcuaaa auacu | 32 | asGfsuauUfuuaguugGfaG faucscsg |
| TD02M 01 | 33 | gsgsauGfaUfUfUfucuu auauca | 34 | usGfsauaUfaAfGfaaaaUf cAfuccsusg |
| TD02M 02 | 35 | gsgsaugaUfUfUfucuua uauca | 36 | usGfsauaUfaagaaaaUfcAf uccsusg |

(continued)

| dsRNA No. | Sense strand SEQ ID NO | Sequence in the 5' to 3' direction | Antisense strand SEQ ID NO | Sequence in the 5' to 3' direction |
|---|---|---|---|---|
| TD03M 01 | 37 | gsgsucUfuUfUfCfagga ugauuu | 38 | asAfsaucAfuCfCfugaaAf aGfaccsusu |
| TD03M 02 | 39 | gsgsucuuUfUfCfaggau gauuu | 40 | asAfsaucAfuccugaaAfaGf accsusu |
| TD04M 01 | 41 | gsuscuUfuUfCfAfggau gauuuu | 42 | asAfsaauCfaUfCfcugaAfa Afgacscsu |
| TD04M 02 | 43 | gsuscuuuUfCfAfggaug auuuu | 44 | asAfsaauCfauccugaAfaAf gacscsu |
| TD05M 01 | 45 | csusuuUfcAfGfGfauga uuuucu | 46 | asGfsaaaAfuCfAfuccuGf aAfaagsasc |
| TD05M 02 | 47 | csusuuucAfGfGfaugau uuucu | 48 | asGfsaaaAfucauccuGfaAf aagsasc |
| TD06M 01 | 49 | ususuuCfaGfGfAfugau uuucuu | 50 | asAfsgaaAfaUfCfauccUf gAfaaasgsa |
| TD06M 02 | 51 | ususuucaGfGfAfugauu uucuu | 52 | asAfsgaaAfaucauccUfgAf aaasgsa |
| TD07M 01 | 53 | uscsagGfaUfGfAfuuuu cuuaua | 54 | usAfsuaaGfaAfAfaucaUf cCfugasasa |
| TD07M 02 | 55 | uscsaggaUfGfAfuuuuc uuaua | 56 | usAfsuaaGfaaaaucaUfcCf ugasasa |
| TD08M 01 | 57 | csasggAfuGfAfUfuuuc uuauau | 58 | asUfsauaAfgAfAfaaucAf uCfcugsasa |
| TD08M 02 | 59 | csasggauGfAfUfuuucu uauau | 60 | asUfsauaAfgaaaaucAfuCf cugsasa |
| TD09M 01 | 61 | asgsgaUfgAfUfUfuucu uauauc | 62 | gsAfsuauAfaGfAfaaauCf aUfccusgsa |
| TD09M 02 | 63 | asgsgaugAfUfUfuucuu auauc | 64 | gsAfsuauAfagaaaauCfaUf ccusgsa |
| TD10M 01 | 65 | gsasugAfuUfUfUfcuua uaucaa | 66 | usUfsgauAfuAfAfgaaaAf uCfaucscsu |
| TD10M 02 | 67 | gsasugauUfUfUfcuuau aucaa | 68 | usUfsgauAfuaagaaaAfuC faucscsu |
| TD11M 01 | 69 | asusgaUfuUfUfCfuuau aucaag | 70 | csUfsugaUfaUfAfagaaAf aUfcauscsc |
| TD11M 02 | 71 | asusgauuUfUfCfuuaua ucaag | 72 | csUfsugaUfauaagaaAfaUf causcsc |
| TD12M 01 | 73 | usgsauUfuUfCfUfuaua ucaagu | 74 | asCfsuugAfuAfUfaagaAf aAfucasusc |

(continued)

| dsRNA No. | Sense strand SEQ ID NO | Sequence in the 5' to 3' direction | Antisense strand SEQ ID NO | Sequence in the 5' to 3' direction |
|---|---|---|---|---|
| TD12M 02 | 75 | usgsauuuUfCfUfuauau caagu | 76 | asCfsuugAfuauaagaAfaA fucasusc |
| TD13M 01 | 77 | csusuaUfaUfCfAfagug guacau | 78 | asUfsguaCfcAfCfuugaUf aUfaagsasa |
| TD13M 02 | 79 | csusuauaUfCfAfagugg uacau | 80 | asUfsguaCfcacuugaUfaUf aagsasa |
| TD14M 01 | 81 | asasacGfgAfUfCfucca acuaaa | 82 | usUfsuagUfuGfGfagauCf cGfuuusgsa |
| TD14M 02 | 83 | asasacggAfUfCfuccaac uaaa | 84 | usUfsuagUfuggagauCfcG fuuusgsa |

In some embodiments, the dsRNA ligand conjugate (siRNA ligand conjugate) is selected from the group consisting of:

Table 7. dsRNA ligand conjugates

| dsRNA No. | Sense strand SEQ ID NO | Sequence in the 5' to 3' direction | Antisense strand SEQ ID NO | Sequence in the 5' to 3' direction |
|---|---|---|---|---|
| TD01L | 1 | GAUCUCCAACUAAAAU ACUL | 2 | AGUAUUUUAGUUGGA GAUCCG |
| TD02L | 3 | GGAUGAUUUUCUUAUA UCAL | 4 | UGAUAUAAGAAAAUC AUCCUG |
| TD03L | 5 | GGUCUUUUCAGGAUGA UUUL | 6 | AAAUCAUCCUGAAAA GACCUU |
| TD04L | 7 | GUCUUUUCAGGAUGAU UUUL | 8 | AAAAUCAUCCUGAAA AGACCU |
| TD05L | 9 | CUUUUCAGGAUGAUUU UCUL | 10 | AGAAAAUCAUCCUGA AAAGAC |
| TD06L | 11 | UUUUCAGGAUGAUUUU CUUL | 12 | AAGAAAAUCAUCCUG AAAAGA |
| TD07L | 13 | UCAGGAUGAUUUUCUU AUAL | 14 | UAUAAGAAAAUCAUC CUGAAA |
| TD08L | 15 | CAGGAUGAUUUUCUUA UAUL | 16 | AUAUAAGAAAAUCAU CCUGAA |
| TD09L | 17 | AGGAUGAUUUUCUUAU AUCL | 18 | GAUAUAAGAAAAUCA UCCUGA |
| TD10L | 19 | GAUGAUUUUCUUAUAU CAAL | 20 | UUGAUAUAAGAAAAU CAUCCU |

(continued)

| dsRNA No. | Sense strand SEQ ID NO | Sequence in the 5' to 3' direction | Antisense strand SEQ ID NO | Sequence in the 5' to 3' direction |
|---|---|---|---|---|
| TD11L | 21 | AUGAUUUUCUUAUAUC AAGL | 22 | CUUGAUAUAAGAAAA UCAUCC |
| TD12L | 23 | UGAUUUUCUUAUAUCA AGUL | 24 | ACUUGAUAUAAGAAA AUCAUC |
| TD13L | 25 | CUUAUAUCAAGUGGUA CAUL | 26 | AUGUACCACUUGAUA UAAGAA |
| TD14L | 27 | AAACGGAUCUCCAACU AAAL | 28 | UUUAGUUGGAGAUCC GUUUGA |
| TD01M 01L | 29 | gsasucUfcCfAfAfcuaaaauac uL | 30 | asGfsuauUfuUfAfguugGfa Gfaucscsg |
| TD01M 02L | 31 | gsasucucCfAfAfcuaaaauacu L | 32 | asGfsuauUfuuaguugGfaGfa ucscsg |
| TD02M 01L | 33 | gsgsauGfaUfUfUfucuuauauc aL | 34 | usGfsauaUfaAfGfaaaaUfc Afuccsusg |
| TD02M 02L | 35 | gsgsaugaUfUfUfucuuauauca L | 36 | usGfsauaUfaagaaaaUfcAfu ccsusg |
| TD03M 01L | 37 | gsgsucUfuUfUfCfaggaugauu uL | 38 | asAfsaucAfuCfCfugaaAfa Gfaccsusu |
| TD03M 02L | 39 | gsgsucuuUfUfCfaggaugauuu L | 40 | asAfsaucAfuccugaaAfaGfa ccsusu |
| TD04M 01L | 41 | gsuscuUfuUfCfAfggaugauuu uL | 42 | asAfsaauCfaUfCfcugaAfa Afgacscsu |
| TD04M 02L | 43 | gsuscuuuUfCfAfggaugauuuu L | 44 | asAfsaauCfauccugaAfaAfg acscsu |
| TD05M 01L | 45 | csusuuUfcAfGfGfaugauuuuc uL | 46 | asGfsaaaAfuCfAfuccuGfa Afaagsasc |
| TD05M 02L | 47 | csusuuucAfGfGfaugauuuucu L | 48 | asGfsaaaAfucauccuGfaAfa agsasc |
| TD06M 01L | 49 | ususuuCfaGfGfAfugauuuucu uL | 50 | asAfsgaaAfaUfCfauccUfg Afaaasgsa |
| TD06M 02L | 51 | ususuucaGfGfAfugauuuucuu L | 52 | asAfsgaaAfaucauccUfgAfa aasgsa |
| TD07M 01L | 53 | uscsagGfaUfGfAfuuuucuuau aL | 54 | usAfsuaaGfaAfAfaucaUfc Cfugasasa |
| TD07M 02L | 55 | uscsaggaUfGfAfuuuucuuaua L | 56 | usAfsuaaGfaaaaucaUfcCfu gasasa |

(continued)

| dsRNA No. | Sense strand SEQ ID NO | Sequence in the 5' to 3' direction | Antisense strand SEQ ID NO | Sequence in the 5' to 3' direction |
|---|---|---|---|---|
| TD08M 01L | 57 | csasggAfuGfAfUfuuucuuaua uL | 58 | asUfsauaAfgAfAfaaucAfu Cfcugsasa |
| TD08M 02L | 59 | csasggauGfAfUfuuucuuauau L | 60 | asUfsauaAfgaaaaucAfuCfc ugsasa |
| TD09M 01L | 61 | asgsgaUfgAfUfUfuucuuauau cL | 62 | gsAfsuauAfaGfAfaaauCfa Ufccusgsa |
| TD09M 02L | 63 | asgsgaugAfUfUfuucuuauauc L | 64 | gsAfsuauAfagaaaauCfaUfc cusgsa |
| TD10M 01L | 65 | gsasugAfuUfUfUfcuuauauca aL | 66 | usUfsgauAfuAfAfgaaaAfu Cfaucscsu |
| TD10M 02L | 67 | gsasugauUfUfUfcuuauaucaa L | 68 | usUfsgauAfuaagaaaAfuCfa ucscsu |
| TD11M 01L | 69 | asusgaUfuUfUfCfuuauaucaa gL | 70 | csUfsugaUfaUfAfagaaAfa Ufcauscsc |
| TD11M 02L | 71 | asusgauuUfUfCfuuauaucaag L | 72 | csUfsugaUfauaagaaAfaUfc auscsc |
| TD12M 01L | 73 | usgsauUfuUfCfUfuauaucaag uL | 74 | asCfsuugAfuAfUfaagaAfa Afucasusc |
| TD12M 02L | 75 | usgsauuuUfCfUfuauaucaagu L | 76 | asCfsuugAfuauaagaAfaAfu casusc |
| TD13M 01L | 77 | csusuaUfaUfCfAfagugguaca uL | 78 | asUfsguaCfcAfCfuugaUfa Ufaagsasa |
| TD13M 02L | 79 | csusuauaUfCfAfagugguacau L | 80 | asUfsguaCfcacuugaUfaUfa agsasa |
| TD14M 01L | 81 | asasacGfgAfUfCfuccaacuaaa L | 82 | usUfsuagUfuGfGfagauCfc Gfuuusgsa |
| TD14M 02L | 83 | asasacggAfUfCfuccaacuaaa L | 84 | usUfsuagUfuggagauCfcGfu uusgsa |

L denotes the ligand as described above. In some embodiments, the ligand is selected from the group consisting of the L01 ligand and the L02 ligand. The sequence ID number of the sense strand refers to the sequence ID number of the nucleic acid sequence in the sense strand. The L at the 3' end of the sense strand sequence of the serial number of the dsRNA ligand conjugate indicates that in the dsRNA ligand conjugate, a ligand is linked to the 3' end of the sense strand sequence.

## Pharmaceutically Acceptable Salt

In some embodiments, the salt described above is selected from the group consisting of a base addition salt, an acid addition salt, and combinations thereof.

In some embodiments, the base addition salt is selected from the group consisting of sodium, potassium,

calcium, ammonium, organic amine, magnesium salts, and combinations thereof, and the acid addition salt is selected from the group consisting of inorganic acid salts, organic acid salts, and combinations thereof.

In some embodiments, the inorganic acid is selected from the group consisting of hydrochloric acid, hydrobromic acid, nitric acid, carbonic acid, bicarbonate, phosphoric acid, monohydrogen phosphate, dihydrogen phosphate, sulfuric acid, hydrogen sulfate, hydroiodic acid, phosphorous acid, and combinations thereof, and the organic acid is selected from the group consisting of acetic acid, propionic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, tartaric acid, methanesulfonic acid, and combinations thereof.

In some embodiments, the pharmaceutically acceptable salt is selected from a sodium salt.

## Beneficial Effects

The double-stranded ribonucleic acid, the pharmaceutically acceptable salt thereof, or the ligand conjugate thereof of the present application has good inhibitory activity against coagulation factor XI. The double-stranded ribonucleic acid, the pharmaceutically acceptable salt thereof, or the ligand conjugate thereof of the present application has good stability and can function continuously *in vivo* for a long term. The double-stranded ribonucleic acid, the pharmaceutically acceptable salt thereof, or the ligand conjugate thereof of the present application exhibits low off-target effects, minimal toxic and side effects, and high safety. Therefore, the present application has good pharmaceutical prospects.

## Definitions and Description

Unless otherwise stated, the following terms and phrases used herein are intended to have the following meanings. A certain term or phrase, unless otherwise specifically defined, shall not be construed as uncertain or unclear, but interpreted according to the meaning as understood by those of ordinary skill in the art. When referring to a trade name, it is intended to refer to its corresponding commercial product or its active ingredient.

In the present application, unless otherwise stated, the terms "comprise", "comprises", and "comprising" or equivalents thereof are open-ended statements and mean that elements, components, or steps that are not specified may be included in addition to those listed.

When any variable occurs more than once in a compound, nucleotide, or single- or double-stranded structure, the definition of the variable in each case is independent. For example, the modifications described in the present application occur independently, i.e., unless otherwise indicated, modifications in the sense strand do not influence modifications of the antisense strand, modifications in one nucleotide do not influence modifications in another nucleotide, and modifications on the glycosyl of one nucleotide do not influence modifications at another position on the glycosyl of the same nucleotide. The influence includes the presence of the modification and the type of the modification.

The gene sequence of the coagulation factor XI of the present application can be obtained from public databases, and siRNA design is performed by taking the transcript NM_000128.4 of the human coagulation factor XI gene and the transcript XM_005556483.2 of the cynomolgus monkey coagulation factor XI gene as target genes. All siRNA sequences show 100% consistency or 1-3 mismatches with the human transcript and 100% consistency or 1 mismatch with the cynomolgus monkey transcript.

As is known in the art, the term "interfering RNA" or "RNAi" or "interfering RNA sequence" refers to a single-stranded RNA (e.g., a mature miRNA) or a double-stranded RNA (e.g., a duplex RNA such as siRNA, aiRNA, or pre-miRNA) that is capable of reducing or inhibiting the expression of a target gene or sequence (e.g., by mediating the degradation of mRNA complementary to the interfering RNA sequence or inhibiting the translation or transcription of mRNA complementary to the interfering RNA sequence) when the interfering RNA and the target gene or sequence are in the same cell. Interfering RNAs may have substantial or complete identity to a target gene or sequence, or may comprise mismatch regions (i.e., mismatch sequences).

The double-stranded ribonucleic acid of the present application serves as an interfering RNA. Hereinafter, the double-stranded ribonucleic acid of the present application is sometimes referred to as siRNA.

As is known in the art, the term "mismatch region" or "mismatch sequence" refers to a portion of the interfering RNA (e.g., siRNA, aiRNA, miRNA) sequence that does not have 100% complementarity to its target sequence. The interfering RNA (e.g., siRNA, aiRNA, miRNA) may have at least 1, 2, 3, 4, 5, 6, or more mismatch regions. The mismatch region may be consecutive or may be separated by 1, 2, 3, 4, 5, 6, 7, 8, 9, 10, 11, 12, or more nucleotides. The mismatch region may comprise a single nucleotide or may comprise 2, 3, 4, 5, 6, or more nucleotides.

The term "identity" refers to the similarity between two nucleotide sequences or between two amino acid sequences. The identity of sequences preferably relates to the percentage of nucleotides or amino acids that have the same position in two or more sequences of the same length. Specifically, the "% identity" of two amino acid sequences or two nucleotide sequences can be determined by aligning the sequences for optimal alignment (e.g., gaps may be

introduced in either sequence for optimal alignment with the other sequence) and comparing the amino acids or nucleotides at the corresponding positions. Gaps are generally regarded as non-identical positions regardless of the actual position in the alignment. The "optimal alignment" is generally an alignment of two sequences that results in the highest percentage identity. The percentage identity is determined by the number of identical nucleotides in the sequences being compared (i.e., % identity = number of identical positions/total number of positions × 100). The sequence identity of the present application is at least 80%, 85%, 90%, or 95%, preferably at least 90%, and nonlimiting examples include: 80%, 81%, 82%, 83%, 84%, 85%, 86%, 87%, 88%, 89%, 90%, 91%, 92%, 93%, 94%, 95%, 96%, 97%, 98%, 99%, and 100%. The determination of percentage identity between two sequences can be accomplished by using a mathematical algorithm known to those skilled in the art.

As is known in the art, interfering RNA includes "small interfering RNA (siRNA)", which has a length of, for example, about 15-60, 15-50, 15-40, 15-30, 15-25, 19-25, 19-23, or 19-21 nucleotides. The double-stranded ribonucleic acid of the present application may function as such an siRNA and may have a corresponding length. As used in the present application, the nucleotide positions of the sense strand or the antisense strand are numbered from the 5' end. For a dsRNA with a sense strand of 19 nucleotides and an antisense strand of 21 nucleotides, for example, in 5'-GAUCUCCAACUAAAAUACU-3' (SEQ ID NO: 1), position 1 is G, and position 19 is U; in 5'-AGUAUUUUAGUUGGAGAUCCG-3' (SEQ ID NO: 2), position 1 is A, and position 21 is G. For a dsRNA with a sense strand of 19 or more nucleotides and an antisense strand of 21 or more nucleotides, for example, if several nucleotides are added to the end of the sense strand on the basis of SEQ ID NO: 1 and several nucleotides are added to the end of the antisense strand on the basis of SEQ ID NO: 2, the method for numbering the nucleotide positions of the sense strand or the antisense strand remains unchanged, that is, position 1 of the sense strand is still position 1 of SEQ ID NO: 1, and position 1 of the antisense strand is still position 1 of SEQ ID NO: 2.

As is known in the art and unless otherwise stated, the term "complementary", when used to describe the relationship between a first nucleic acid sequence and a second nucleic acid sequence, refers to an ability of an oligonucleotide or polynucleotide comprising the first nucleic acid sequence to hybridize with an oligonucleotide or polynucleotide comprising the second nucleic acid sequence and form a double-stranded structure under specific conditions. As described in the present application, "complementary" sequences may also include double-stranded structures formed by non-Watson-Crick base pairing and/or base pairing formed by non-natural or modified nucleotides, or may be double-stranded structures formed entirely by non-Watson-Crick base pairing and/or base pairing formed by non-natural or modified nucleotides, so long as the above requirements with respect to their ability to hybridize are met.

As is known in the art, "completely complementary" sequences include base pairing of an oligonucleotide or polynucleotide comprising the first nucleic acid sequence with an oligonucleotide or polynucleotide comprising the second nucleic acid sequence over the entire length of the first nucleic acid sequence and the second nucleic acid sequence.

As is known in the art, the "substantially complementary" means that two nucleic acid sequences are completely complementary or that at least 85% (e.g., 85%, 86%, 87%, 88%, 89%, 90%, 91%, 92%, 93%, 94%, 95%, 96%, 97%, 98%, 99%, or 100%) of overlapping nucleotides are complementary.

The terms "complementary", "completely complementary", and "substantially complementary" of the present application may be used in accordance with the base pairing between the sense strand and the antisense strand of the dsRNA, or between the antisense strand of the dsRNA and a target sequence, as will be understood from the context of their use.

In the art, "G", "C", "A", "T", and "U" generally represent the bases of guanine, cytosine, adenine, thymine, and uracil, respectively, but it is also generally known in the art that "G", "C", "A", "T", and "U" each generally also represent nucleotides comprising guanine, cytosine, adenine, thymine, and uracil as the base, respectively; this is a common way of representing deoxyribonucleic acid sequences and/or ribonucleic acid sequences; therefore, in the context of the present disclosure, the meanings represented by "G", "C", "A", "T", and "U" include the various possible cases described above. However, it will be appreciated that the term "ribonucleotide" or "nucleotide" may also refer to a modified nucleotide (as further detailed elsewhere herein) or a nucleotide having an alternative replacement moiety, and those skilled in the art will recognize that guanine, cytosine, adenine, and uracil can be replaced by other moieties without substantially altering the base pairing properties of an oligonucleotide (including a nucleotide having such a replacement moiety). For example, without limitation, a nucleotide comprising inosine as its base may pair with nucleotides comprising adenine, cytosine, or uracil. Thus, nucleotides comprising uracil, guanine, or adenine may be replaced in the nucleotide sequences of the dsRNA characterized in the present application by a nucleotide comprising, for example, inosine. In another example, adenine and cytosine anywhere in the oligonucleotide can be replaced by guanine and uracil, respectively, to form G-U wobble base pairing with the target mRNA. Sequences comprising such replacement moieties are suitable for use in the compositions and methods characterized in the present disclosure.

As is known in the art, the "double-stranded ribonucleic acid", the "double-stranded RNA", and the "dsRNA" are used interchangeably. The term "dsRNA" comprises two antiparallel and complementary nucleic acid strands that have a "sense" or "antisense" orientation relative to a target RNA (e.g., the coagulation factor XI gene). In the examples of the present application, the dsRNA may degrade the target RNA (e.g., mRNA) through the RNA interference (RNAi)

mechanism.

The double-stranded ribonucleic acid of the present application comprises a sense strand and an antisense strand. The term "sense strand" refers to a single strand of the dsRNA double strands that is substantially complementary to a region of the antisense strand. The term "antisense strand" refers to a single strand of the dsRNA double strands that is substantially complementary to a region of the target sequence. Mismatches may occur within the molecule or in the terminal regions when the sense strand is not completely complementary to the antisense strand. In general, mismatches are most tolerated in the terminal regions. Mismatches may occur within the molecule or in the terminal regions when the antisense strand is not completely complementary to the target sequence. In general, mismatches are most tolerated in the terminal regions.

As is known in the art, the two strands of the dsRNA can have the same or different numbers of nucleotides. The length of the double-stranded region formed by complementation may be any length that permits the degradation of the target RNA, and possible lengths are in the range of about 9 to 36 nucleotide pairs, e.g., 15 to 30 pairs, 16 to 28 pairs, 19 to 21 pairs, etc.

As is known in the art, outside of the double-stranded region, the dsRNA may comprise one or more nucleotide overhangs, each of the overhangs referring to at least one unpaired nucleotide/nucleoside analog. For example, when the 3’ end of one strand of the dsRNA exceeds the 5’ end of the other strand (or vice versa), an overhang is present. The nucleotides of the "overhang" may comprise 0-5 nucleotides, where "0" indicates no "overhang", and "5" indicates 5 additional (i.e., unpaired with the other single strand) nucleotides on one single strand of the dsRNA double strands. These optional "overhangs" may be located at the 5’ end and/or the 3’ end of either single strand of the dsRNA. In some embodiments, the "overhang" comprises 0-5 nucleotides. In some embodiments, the "overhang" comprises 0-2 nucleotides. In some embodiments, the "overhang" at the 3’ end and/or the 5’ end of the sense strand of the dsRNA has 0-2 nucleotides. In some embodiments, the "overhang" at the 3’ end and/or the 5’ end of the antisense strand of the dsRNA has 0-2 nucleotides. The nucleotides forming the "overhang" may be A, G, C, U, or T, or modified structures thereof. The nucleotides forming the "overhang" may be U, T, or dT, or modified structures thereof. In some embodiments, the "overhang" includes, but is not limited to, "TT", "dTdT", "UU", or corresponding modified structures thereof, e.g., 2’-methoxy-modified UU, i.e., uu. In some embodiments, the "overhang" at the 3’ end and/or the 5’ end of the antisense strand of the dsRNA is substantially complementary to the target RNA. In some embodiments, the "overhang" at the 3’ end and/or the 5’ end of the antisense strand of the dsRNA is completely complementary to the target RNA. In some embodiments, the "overhang" at the 3’ end of the antisense strand of the dsRNA is completely complementary to the target RNA. The term "blunt end" means that there are no unpaired nucleotides at the end of the dsRNA, i.e., no nucleotide overhang. A dsRNA with "blunt" ends at both ends is a dsRNA with a double-stranded region over the entire length, i.e., no nucleotide overhang at either end of the molecule.

In the present application, the terms "phosphorothioate linkage", "phosphorodithioate linkage", and "phosphorothioate group" can be used interchangeably.

In the present application, the dsRNA or any single strand thereof is optionally modified, and both unmodified and modified ribonucleic acids fall within the protection scope of the present application. The modification does not cause significant weakening or loss of the function of the dsRNA to inhibit the expression of the coagulation factor XI gene. The modification of the dsRNA or any single strand thereof may be located at the 5’ end and/or the 3’ end, a nucleotide, or an internucleotide linkage. The synthesis or modification can be performed by methods well known in the art.

In the present application, "that the sense strand or the antisense strand is optionally modified" means that any nucleotide of the sense strand or the antisense strand is optionally modified.

In the present application, the modification of the nucleotide includes, but is not limited to, those occurring on the glycosyl of the nucleotide, including one or more substituted or removed glycosyl moiety groups, such as the removal of the hydroxyl group at a carbonyl group, or the occurrence of fluorination, amination, alkylation, hydroxyalkylation, or hydroxyalkenylation. Modifications on glycosyl may occur at various positions on the sugar ring. Illustratively, modifications on the glycosyl of the nucleotide include, but are not limited to, 2’-dehydroxylation, 2’-fluoro, 2’-amino, 2’-methyl, 2’-ethyl, 2’-methyl-O-methyl, 2’-ethyl-O-methyl, 2’-O-methyl, 2’-O-ethyl, 2’-O-ethyl-O-methyl, or 2’-O-allyl, and the modifications and the modified nucleoside structures may be as follows:

| Modification | Nucleoside structure | Modification | Nucleoside structure |
|---|---|---|---|
| 2’-Dehydroxylation | 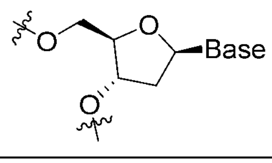 | 2’-Fluoro | 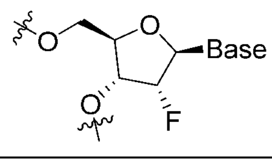 |

(continued)

| Modification | Nucleoside structure | Modification | Nucleoside structure |
|---|---|---|---|
| 2'-Amino | 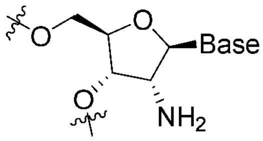 | 2'-Methyl | 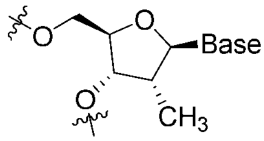 |
| 2'-Ethyl | 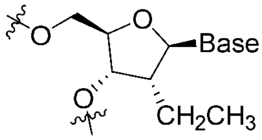 | 2'-Methyl-O-methyl | 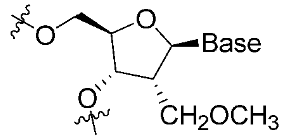 |
| 2'-Ethyl-O-methyl | 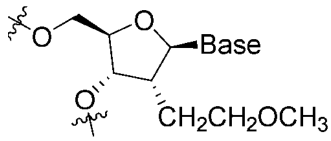 | 2'-O-Methyl | 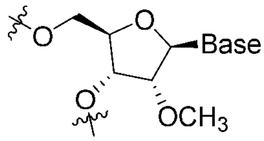 |
| 2'-O-Ethyl | 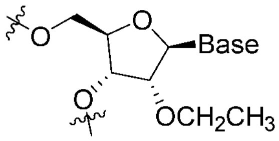 | 2'-O-Ethyl-O-methyl | 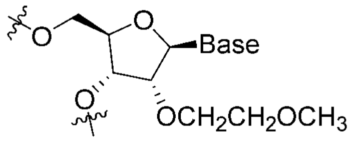 |
| 2'-O-Allyl | 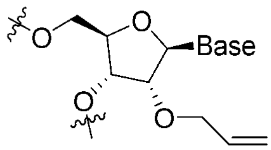 | | |

wherein Base represents a base.

In the present application, the modification of the linkage between nucleotides includes substitution or replacement of atoms or functional groups of phosphate groups, such as phosphorothioate (PS), phosphorodithioate (PS2), methylphosphonate (MP), methoxypropylphosphonate (MOP), and aminophosphonate.

In the present application, uppercase letters G, C, A, U, and T each generally represent a nucleotide comprising guanine, cytosine, adenine, uracil, and thymine as the base, where the letter combination dT represents a deoxyribonucleotide with thymine as the base. Lowercase letters g, c, a, and u indicate that the ribosyl groups of the nucleotides represented by their corresponding uppercase letters are modified by 2'-methoxy, that is, g, c, a, and u represent 2'-O-methyl G, 2'-O-methyl C, 2'-O-methyl A, and 2'-O-methyl U, respectively. Uppercase letters with a lowercase f on the right side indicate that the ribosyl groups of the nucleotides represented by their corresponding uppercase letters are modified by 2'-fluoro, i.e., Gf, Cf, Af, and Uf represent 2'-fluoro G, 2'-fluoro C, 2'-fluoro A, and 2'-fluoro U, respectively. Lowercase letter s indicates that the two nucleotide residues adjacent to s are linked via a phosphorothioate group; for example, "csu" indicates that residues c and u are linked via a phosphorothioate group. VP- indicates that the nucleotide to the right of the hyphen is a (E)-vinylphosphate modified nucleotide; for example, "VP-u" indicates a (E)-vinylphosphate modified 2'-O-methyl U.

In the present application, the modification at the 5' end and/or the 3' end refers to a modification occurring at the 5' end and/or the 3' end of the dsRNA or any single strand thereof, e.g., phosphorylation, conjugation, reverse linkage, or the like. For example, the modification at the 5' end includes, but is not limited to, 5'-phosphate, 5'-methylphosphonate (5'-MP), 5'-phosphorothioate (5'-PS), or 5'-(E)-vinylphosphonate (5'-(E)-VP), and the modifications and the modified nucleotide structures may be as follows:

| Modification | Nucleotide structure | Modification | Nucleotide structure |
|---|---|---|---|
| 5'-Phosphate | 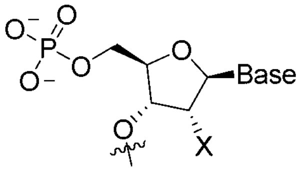 | 5'-Methylphosphonate | 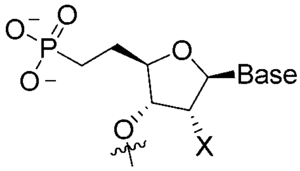 |
| 5'-Phosphorothioate | 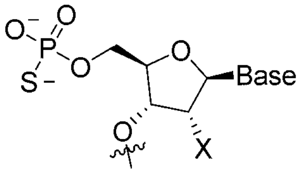 | 5'-(*E*)-Vinylphosphonate | 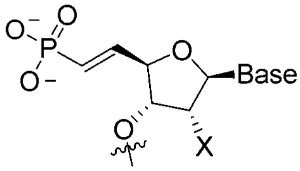 |

wherein Base represents a base, and X is selected from a 2' modification on hydrogen, hydroxyl, or glycosyl.

In the present application, the ligand is a group linked to the dsRNA, comprising a branched group and a linker, and the dsRNA, the linker, and the branched group are linked in sequence (e.g., as shown in formula 104). The branched group comprises at least one (e.g., one, two, three, four, or five) pharmaceutically acceptable targeting group that targets the dsRNA to a particular tissue or enhances cellular uptake. The targeting group is, for example, but is not limited to, a GalNAc (N-acetylgalactosamine, e.g., as shown in formula 105) group. A number of the targeting groups are linked in series or in parallel via the branched group. The GalNAc group may be monovalent, divalent, trivalent, or tetravalent. The monovalent, divalent, trivalent, and tetravalent described herein refer to that after the dsRNA molecule and the ligand comprising GalNAc as the targeting group form the dsRNA ligand conjugate, the molar ratios of the dsRNA molecule to the GalNAc molecule in the dsRNA ligand conjugate are 1:1, 1:2, 1:3, and 1:4, respectively. In some embodiments, when the dsRNA of the present application is conjugated to a ligand comprising GalNAc, the GalNAc molecule is trivalent or tetravalent. In some embodiments, when the dsRNA of the present application is conjugated to a ligand comprising GalNAc, the GalNAc molecule is trivalent.

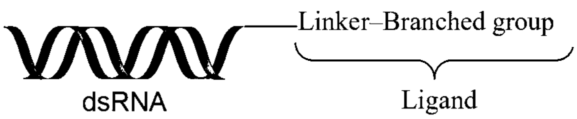


formula 104

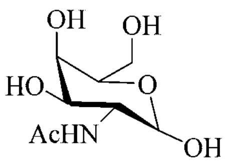

formula 105

In the present application, the ligand may be linked to the phosphate group, the 2'-hydroxyl, the 3'-hydroxyl, or the base of the nucleotide. The ligand may be linked to any nucleotide of the dsRNA, including but not limited to the 5' or 3' terminal nucleotide or the non-terminal intermediate nucleotide of the sense strand or the antisense strand. When the ligand is linked to the end of the dsRNA strand, the ligand may be linked to a phosphate group of a nucleotide; when the ligand is linked to an intermediate nucleotide of the dsRNA, the ligand may be linked to the sugar ring or the base of the nucleotide.

In the present application, for the types or preparation methods of the ligands, reference may be made to methods known in the art, including but not limited to ligands and preparation methods thereof described in WO2009082607, WO2014025805, WO2015006740, and WO2021249484, which are incorporated herein by reference in their entirety. Exemplary ligands include, but are not limited to, L01 or L02 described above. The L01 ligand described in the present application is the same as the L96 ligand in the prior art.

In the present application, unless otherwise stated, the "conjugation" means that two or more chemical moieties, each having a particular function, are non-covalently or covalently linked to each other. Accordingly, the "conjugate" refers to a compound formed by non-covalent or covalent linking between the various chemical moieties. In the present application, conjugates that are linked covalently to each other are preferred.

In the present application, the ligand is linked to the 5' or 3' end of the sense strand or the antisense strand. Preferably, the ligand is linked to the 5' or 3' end of the sense strand. More preferably, the ligand is linked to the 3' end of the sense strand. Illustratively, the dsRNA and the ligand are linked to form the dsRNA ligand conjugate as shown in formula 102 or formula 103 below:

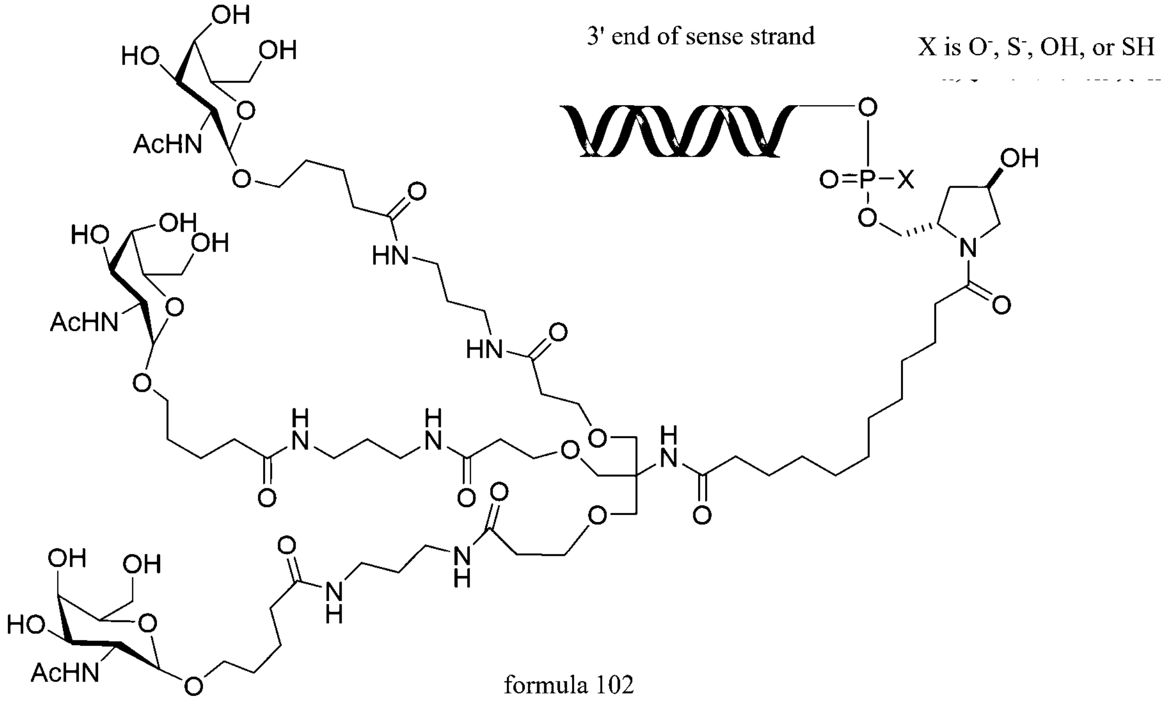


formula 102

,

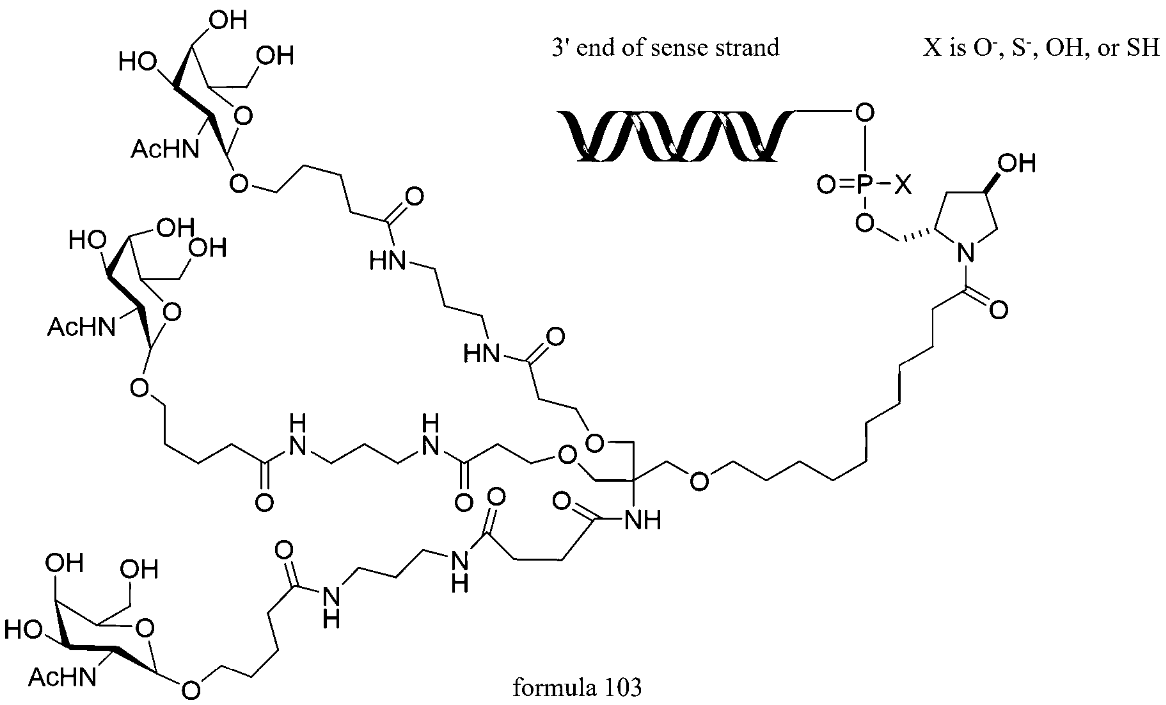


formula 103

.

The compound of the present application (e.g., the double-stranded ribonucleic acid, the pharmaceutically acceptable salt thereof, or the ligand conjugate thereof of the present application) may have particular geometric or stereoisomeric forms, all of which are encompassed within the scope of the present application. All such compounds are contemplated in the present application, including (R)- and (S)- enantiomers, diastereoisomers, and racemic mixtures and

other mixtures thereof, such as an enantiomer- or diastereoisomer-enriched mixture, all of which are encompassed within the scope of the present application. Additional asymmetric carbon atoms may be present in substituents such as alkyl. All these isomers and mixtures thereof are encompassed within the scope of the present application.

Unless otherwise stated, the term "enantiomer" or "optical isomer" refers to stereoisomers that are mirror images of each other.

Unless otherwise stated, the term "diastereoisomer" refers to stereoisomers whose molecules have two or more chiral centers and are not mirror images of each other.

Unless otherwise stated, the absolute configuration of a stereogenic center is represented by a wedged solid bond ( ) and a wedged dashed bond ( ), and the relative configuration of a stereogenic center is represented by a straight solid bond ( ) and a straight dashed bond ( ); a wavy line ( ) represents a wedged solid bond ( ) or a wedged dashed bond ( ), or a wavy line ( ) represents a straight solid bond ( ) and/or a straight dashed bond ( ).

Unless otherwise stated, the term "enriched with one isomer", "isomer-enriched", "enriched with one enantiomer", or "enantiomer-enriched" means that the content of one of the isomers or enantiomers is less than 100% and more than or equal to 60%, or more than or equal to 70%, or more than or equal to 80%, or more than or equal to 90%, or more than or equal to 95%, or more than or equal to 96%, or more than or equal to 97%, or more than or equal to 98%, or more than or equal to 99%, or more than or equal to 99.5%, or more than or equal to 99.6%, or more than or equal to 99.7%, or more than or equal to 99.8%, or more than or equal to 99.9%.

Unless otherwise stated, the term "isomeric excess" or "enantiomeric excess" refers to the difference between the relative percentages of two isomers or enantiomers. For example, if the content of one of the isomers or enantiomers is 90% and the content of the other isomer or enantiomer is 10%, the isomeric or enantiomeric excess (ee value) is 80%.

Optically active (R)- and (S)-isomers and D and L isomers can be prepared by chiral synthesis or chiral reagents or other conventional techniques. An enantiomer of a certain compound of the present application can be prepared by asymmetric synthesis or derivatization using a chiral additive, wherein the resulting diastereoisomeric mixture is separated and the auxiliary group is cleaved so as to provide the desired pure enantiomer. Alternatively, when the molecule comprises a basic functional group (e.g., amino) or an acidic functional group (e.g., carboxyl), the compound reacts with an appropriate optically active acid or base to form a salt of the diastereoisomer, which is then subjected to diastereoisomeric resolution through conventional methods well known in the art to give the pure enantiomer. Furthermore, the enantiomer and the diastereoisomer are generally separated through chromatography using a chiral stationary phase, optionally in combination with chemical derivatization (e.g., carbamate generated from amines). The compound of the present application may comprise an unnatural proportion of atomic isotopes at one or more of the atoms that constitute the compound. For example, the compound may be labeled with a radioisotope, such as tritium ($^3H$), iodine-125 ($^{125}I$), or C-14 ($^{14}C$). For another example, hydrogen can be substituted by deuterium to form a deuterated drug, and the bond formed by deuterium and carbon is firmer than that formed by common hydrogen and carbon; compared with an undeuterated drug, the deuterated drug has the advantages of reduced toxic and side effects, increased drug stability, enhanced efficacy, prolonged drug biological half-life, and the like. All isotopic variations of the compound of the present application, whether radioactive or not, are encompassed within the scope of the present application.

The term "treat", "treating", or "treatment" refers to administering the compound or formulation of the present application (e.g., the double-stranded ribonucleic acid, the pharmaceutically acceptable salt thereof, the ligand conjugate thereof, or the pharmaceutical composition of the present application) to ameliorate or eliminate a disease or one or more symptoms associated with the disease, and includes: (i) inhibiting the disease or disease state, i.e., arresting its development; and (ii) alleviating the disease or disease state, i.e., causing regression of the disease or disease state.

The term "prevent", "preventing", or "prevention" refers to administering the compound or formulation of the present application (e.g., the double-stranded ribonucleic acid, the pharmaceutically acceptable salt thereof, the ligand conjugate thereof, or the pharmaceutical composition of the present application) to prevent a disease or one or more symptoms associated with the disease, and includes: preventing the occurrence of the disease or disease state in a subject, particularly when such a subject is predisposed to the disease state but has not yet been diagnosed with it.

The terms "subject" and "patient" are used interchangeably herein and refer to an animal that has become the subject of treatment, observation, or experiment. In some embodiments, the subject is a mammal, preferably a primate, and more preferably a human.

The term "therapeutically effective amount" refers to an amount of the compound of the present application (e.g., the double-stranded ribonucleic acid, the pharmaceutically acceptable salt thereof, or the ligand conjugate thereof of the present application) for (i) treating or preventing a specific disease, condition, or disorder; (ii) alleviating, ameliorating, or eliminating one or more symptoms of a specific disease, condition, or disorder, or (iii) preventing or delaying onset of one or more symptoms of a specific disease, condition, or disorder described herein. The amount of the compound of the present application composing the "therapeutically effective amount" varies depending on the compound, the disease state and its

severity, the route of administration, and the age of the subject to be treated, but may be determined routinely by those skilled in the art in accordance with their knowledge and the present application.

The therapeutic dose of the compound of the present application may be determined by, for example, the specific use of the treatment, the route of administration of the compound, the health and condition of a patient, and the judgment of a prescribing physician. The proportion or concentration of the compound of the present application in a pharmaceutical composition may not be constant and depends on a variety of factors including dosages, chemical properties (e.g., hydrophobicity), and routes of administration. For example, the compound of the present application may be provided for parenteral administration by a buffered normal saline containing about 0.1-10% w/v of the compound. Certain typical dosages range from about 1 μg/kg body weight/day to about 1 g/kg body weight/day. In certain embodiments, the dosage ranges from about 0.01 mg/kg body weight/day to about 100 mg/kg body weight/day. The dose is likely to depend on variables such as the type and degree of progression of the disease or disorder, the general health condition of a specific patient, the relative biological potency of the compound selected, the excipient formulation, and its route of administration. Effective doses can be extrapolated from dose-response curves derived from *in vitro* or animal model test systems.

The term "pharmaceutically acceptable" is used herein for those compounds, materials, compositions, and/or dosage forms that are, within the scope of sound medical judgment, suitable for use in contact with the tissues of human beings and other animals without excessive toxicity, irritation, allergic response, or other problems or complications, and commensurate with a reasonable benefit/risk ratio.

The pharmaceutically acceptable salt, for example, may be a metal salt, an ammonium salt, a salt formed with an organic base, a salt formed with an inorganic acid, a salt formed with an organic acid, a salt formed with a basic or acidic amino acid, and the like.

As used in the present application, the pharmaceutically acceptable salt includes a pharmaceutically acceptable salt of the double-stranded ribonucleic acid and a pharmaceutically acceptable salt of the double-stranded ribonucleic acid ligand conjugate.

The term "pharmaceutical composition" refers to a mixture consisting of one or more of the compounds of the present application (e.g., the double-stranded ribonucleic acid, the pharmaceutically acceptable salt thereof, or the ligand conjugate thereof of the present application), and a pharmaceutically acceptable carrier or excipient. The pharmaceutical composition is intended to facilitate the administration of the compound of the present application to an organic entity. As used herein, the terms "pharmaceutical composition" and "formulation" have the same meaning and are used interchangeably. The carrier or excipient used herein includes any and all solvents, diluents, or other liquid excipients, dispersants or suspending agents, surfactants, isotonizing agents, thickening agents or emulsifiers, preservatives, solid binders, lubricants, and the like, suitable for the desired particular dosage form. Various carriers or excipients used to formulate pharmaceutically acceptable compositions and methods for their preparation are known in the art. The use of any conventional carrier media other than those incompatible with the compounds of the present application (e.g., those producing any adverse biological effects or otherwise interacting in a deleterious manner with any other component of the pharmaceutically acceptable composition) is encompassed within the scope of the present application. In some specific embodiments, the carrier or excipient used herein is a carrier or excipient conventionally used in the field of dsRNA administration.

The pharmaceutical composition of the present application may be prepared by combining the compound of the present application with a suitable pharmaceutically acceptable carrier or excipient, and may be formulated, for example, into a solid, semisolid, liquid, or gaseous formulation such as tablet, pill, capsule, powder, granule, ointment, emulsion, suspension, suppository, injection, inhalant, gel, microsphere, aerosol, and the like.

Typical routes of administration of the compound or the pharmaceutical composition thereof of the present application include, but are not limited to, oral, rectal, topical, inhalational, parenteral, sublingual, intravaginal, intranasal, intraocular, intraperitoneal, intramuscular, subcutaneous, and intravenous administrations.

The pharmaceutical composition of the present application may be manufactured using methods well known in the art, such as conventional mixing, dissolving, granulating, dragee-making, levigating, emulsifying, and lyophilizing. The kit of the present application comprises the double-stranded ribonucleic acid, the pharmaceutically acceptable salt thereof, the ligand conjugate thereof, or the pharmaceutical composition of the present application, and optionally an instruction for use of the double-stranded ribonucleic acid, the pharmaceutically acceptable salt thereof, the ligand conjugate thereof, or the pharmaceutical composition of the present application in treating and/or preventing thromboembolism and complications thereof.

The solvents used in the present application are commercially available.

Unless otherwise stated, the solvent ratios used in column chromatography and preparative thin-layer silica gel chromatography in the present application are volume ratios.

It is known in the art that modified nucleotide groups can be introduced into the dsRNA described in the present application by using nucleoside monomers with corresponding modifications. Methods for preparing correspondingly modified nucleoside monomers and methods for introducing modified nucleotide groups into dsRNAs are known to those skilled in the art. All modified nucleoside monomers are commercially available or can be prepared using known methods.

It is known in the art that the desired ribonucleic acid can be acquired by conventional ribonucleic acid preparation methods (e.g., solid-phase synthesis and liquid-phase synthesis) in the art; for example, the desired ribonucleic acid can be synthesized by phosphoramidite-based solid-phase synthesis techniques. The method for preparing the double-stranded ribonucleic acid, the pharmaceutically acceptable salt thereof, or the ligand conjugate thereof of the present application comprises the following steps: linking nucleotide monomers in sequence in the 3' to 5' direction according to the type or order of nucleotides in the sense strand or the antisense strand of the double-stranded ribonucleic acid to synthesize the sense strand and the antisense strand. The ligation of each nucleotide monomer involves four reactions: deprotection, coupling, capping, and oxidation or sulfurization. Those skilled in the art can determine conventional reaction conditions and types and amounts of reagents, or make adjustments according to the experimental conditions to achieve the deprotection, coupling, capping, and oxidation or sulfurization reactions.

In some embodiments, for the synthesis of the ligand-comprising ribonucleic acid, the ligand may be linked to the ribonucleic acid by a coupling reaction during or after the synthesis of the oligoribonucleotide, or the ligand may be first linked to a solid support, and then the nucleoside monomer is linked to the ligand-solid support in sequence in the 3' to 5' direction.

Methods for purification and desalting are well known to those skilled in the art. For example, the purification of the ribonucleic acid may be achieved by preparative ion chromatography methods. For another example, the desalting of the ribonucleic acid may be achieved by a reversed-phase chromatography purification method or an ultrafiltration centrifugation method.

Methods for annealing are well known to those skilled in the art. For example, the sense strand and the antisense strand may be mixed in a molar ratio of 1:1, heated to 70-95 °C, and then cooled to room temperature to form a double-stranded structure.

During the synthesis, the concentration of the ribonucleic acid may be detected by, for example, ion exchange chromatography, or the molecular weight may be determined by liquid chromatography-mass spectrometry, or the concentration may be determined by microspectrophotometer to control the synthesis quality; such detection methods are well known to those skilled in the art.

Unless otherwise specified, terms in the singular form shall be deemed to include the plural form and vice versa. Unless otherwise specified, the word "a" or "an" refers to "at least one". Unless otherwise stated, "or" is used to mean "and/or".

All patents, patent applications, and other identified publications are explicitly incorporated herein by reference for the purpose of description and disclosure. These publications are provided solely because they were disclosed prior to the filing date of the present application. All statements as to the dates of these documents or descriptions as to the content of these documents are based on the information available to the applicant and do not constitute any admission as to the correctness of the dates or the content of these documents. Moreover, in any country or region, any reference to these publications herein should not be construed as an admission that the publications form part of the commonly recognized knowledge in the art.

## DETAILED DESCRIPTION

The present application is described in detail below by way of examples, which, however, are not intended to disadvantageously limit the present application in any way. The compounds of the present application can be prepared by a variety of synthetic methods well known to those skilled in the art, including the specific embodiments listed below, embodiments formed by combinations thereof with other chemical synthetic methods, and equivalent alternatives thereof known to those skilled in the art; the preferred embodiments include, but are not limited to, the examples of the present application. It will be apparent to those skilled in the art that various changes and modifications can be made to the specific embodiments of the present application without departing from the spirit and scope of the present application.

The prepared double-stranded ribonucleic acid, pharmaceutically acceptable salt thereof, or ligand conjugate thereof can be verified to be the target product by mass spectrometry or other detection methods.

Reagents and materials

Commercially available protective monomers used in synthesis

| Protective monomer | CAS |
|---|---|
| 5'-O-(4,4-Dimethoxytrityl)-2'-O-[(tert-butyl)dimethylsilyl]-N-isobutyrylguanosine-3'-(2-cyanoethyl-N,N-diisopropyl)phosphoramidite | 147201-04-5 |

(continued)

| Protective monomer | CAS |
|---|---|
| N-Benzoyl-5'-O-(4,4-dimethoxytrityl)-2'-O-[(tert-butyl)dimethylsilyl]adenosine-3'-(2-cyanoethyl-N,N-diisopropyl)phosphoramidite | 104992-55-4 |
| N-Acetyl-5'-O-(4,4-dimethoxytrityl)-2'-O-[(tert-butyl)dimethylsilyl]cytidine-3'-(2-cyanoethyl-N,N-diisopropyl)phosphoramidite | 121058-88-6 |
| 5'-O-[Bis(4-methoxyphenyl)phenylmethyl]-2'-O-[(1,1-dimethylethyl)dimethylsilyl]-uridine, 3'-[2-cyanoethyl N,N-bis(1-methylethyl)phosphoramidite] | 118362-03-1 |
| 5'-O-[Bis(4-methoxyphenyl)phenylmethyl]-2'-deoxythymidine, 3'-[2-cyanoethyl N,N-bis(1-methylethyl)phosphoramidite] | 98796-51-1 |
| N-Benzoyl-5'-O-[bis(4-methoxyphenyl)phenylmethyl]-2'-deoxy-2'-fluoroadenosine-3'-[2-cyanoethyl-N,N-diisopropyl]phosphoramidite | 136834-22-5 |
| N-Acetyl-5'-O-(4,4-dimethoxytrityl)-2'-deoxy-2'-fluorocytidine-3'-(2-cyanoethyl-N,N-diisopropyl) phosphoramidite | 159414-99-0 |
| 5'-O-[Bis(4-methoxyphenyl)phenylmethyl]-2'-deoxy-2'-fluoro-N-(2-methyl-1-oxopropyl)-guanosine-3'-(2-cyanoethyl N,N-diisopropyl)phosphoramidite | 144089-97-4 |
| 5'-O-(4,4-Dimethoxytrityl)-2'-deoxy-2'-fluorouridine-3'-(2-cyanoethyl-N,N-diisopropyl)phosphoramidite | 146954-75-8 |
| N-Benzoyl-5'-O-(4,4-dimethoxytrityl)-2'-O-methyladenosine-3'-(2-cyanoethyl-N,N-diisopropyl) phosphoramidite | 110782-31-5 |
| N-Acetyl-5'-O-(4,4-dimethoxytrityl)-2'-O-methylcytidine-3'-(2-cyanoethyl-N,N-diisopropyl)phosphoramidite | 199593-09-4 |
| N2-Acetyl-5'-O-(4,4-dimethoxytrityl)-2'-O-methylguanosine-3'-(2-cyanoethyl-N,N-diisopropyl)phosphoramidite | 909033-40-5 |
| 5'-O-(4,4-Dimethoxytrityl)-2'-O-methyluridine-3'-(2-cyanoethyl-N,N-diisopropyl)phosphoramidite | 110764-79-9 |

Reagents used in synthesis

| Use | Reagents |
|---|---|
| Washing | 3% TCA/DCM (3% trichloroacetic acid/DCM) |
| Decapping agent DBK | 0.25 mol/L ETT (5-ethylthiotetrazole)/ACN |
| Activating agent ACT | 25% NMI (N-methylmorpholine)/ACN |
| Capping A | 20% acetic anhydride, 30% 2,6-lutidine/ACN |
| Capping B | 0.025 mol/L I2/(pyridine/water/acetonitrile = 9/1/10) |
| Oxidant | 0.2 mol/L PADS (phenylacetyl disulfide)/ACN |
| Sulfuration reagent | 3% TCA/DCM (3% trichloroacetic acid/DCM) |

The present application uses the following abbreviations:
DCM represents dichloromethane; ACN represents acetonitrile; DIC represents diisopropylcarbodiimide; DMAP represents dimethylaminopyridine; DMT represents di(p-methoxytrityl); MEM represents 2-methoxyethoxymethyl.

siRNA sequence design and specificity analysis

siRNA design is performed by taking the transcript NM_000128.4 of the human coagulation factor XI gene and the transcript XM_005556483.2 of the cynomolgus monkey coagulation factor XI gene as target genes. All siRNA sequences show 100% consistency with the human transcript and 100% consistency or 1 mismatch with the cynomolgus

monkey transcript.

**Preparation Example 1. Ligation of GalNAc Ligand (Ligand Comprising GalNAc Group as Targeting Group) to Solid Support**

1.0 g of amino-CPG (amino loading: 30-40 μmol/g) was weighed, and 5 mL of decapping agent DBK was added. The mixture was reacted for 1 min, and the reagent was removed. The operation described above was repeated 4 times. Based on a calculated loading of 35 μmol, 2 eq of GalNAc ligand/acetonitrile solution was added, and then a condensation reagent consisting of 2.5 eq of DIC and 2.5 eq of DMAP was added. The mixture was reacted at 25-30 °C for 24 h. After the reaction was completed, the mixture was filtered to remove the solvent and washed with anhydrous acetonitrile before the GalNAc ligand and the condensation reagent were added according to the above amounts. The mixture was reacted for another 24 h. After the reaction was completed, the solid support was washed with anhydrous acetonitrile, and 4 mL of 1:1 reagent mixture of Capping A and Capping B was added. The mixture was reacted for 2 min, and after the removal of reagents, 4 mL of the reagent mixture was added. The mixture was reacted for 2 min, and the reagents were removed. The mixture was washed with anhydrous acetonitrile and dried *in vacuo* at 30 °C for 1 h for later use. The GalNAc ligand in this example was the L01 (or L02) ligand.

**Preparation Example 2. Preparation of Double-Stranded Ribonucleic Acid**

**2.1.1 Synthesis of GalNAc ligand-ssRNA (sense strand)**

On the K&A nucleic acid synthesizer, the GalNAc ligand-solid support described above was placed into the synthetic column, and the ssRNA was synthesized according to the standard phosphoramidite-based technique. The specific steps include:

1) DMT removal (deprotection): The resin was washed twice with acetonitrile, the DMT on the resin was removed with 3% trichloroacetic acid/DCM, and washing was performed 4-5 times with acetonitrile.

2) Condensation (coupling): Monomers were added to the synthetic column, and a condensation reagent (2.5 eq DIC, 2.5 eq DMAP) was added for condensation at room temperature for 10 min. Washing was performed 4-5 times with acetonitrile.

3) Blocking (capping): Unreacted hydroxyl was blocked first with Capping A and then with Capping B. Washing was performed 4-5 times with acetonitrile.

4) Oxidation: An oxidant was added, the oxidation was performed for 2 min, and washing was performed 4-5 times with acetonitrile.

The steps from 1) to 4) were repeated until the synthesis of all sequences was completed. Finally, DMT was removed with a decapping agent DBK, and washing was performed 4-5 times with acetonitrile.
Phosphorothioate bond formation: The oxidant was replaced with a sulfuration reagent, and the sulfuration time was set to 10 min, so as to complete the phosphorothioate bond formation.

**2.1.2 Synthesis of ssRNA (sense strand without ligand)**

On a K&A nucleic acid synthesizer, the universal CPG solid support was placed into the synthetic column, and the ssRNA was synthesized according to the standard phosphoramidite-based technique. The specific steps include:

1) DMT removal (deprotection): The resin was washed twice with acetonitrile, the DMT on the resin was removed with 3% trichloroacetic acid/DCM, and washing was performed 4-5 times with acetonitrile.

2) Condensation (coupling): Monomers were added to the synthetic column, and a condensation reagent was added for condensation at room temperature for 10 min. Washing was performed 4-5 times with acetonitrile.

3) Blocking (capping): Unreacted hydroxyl was blocked first with Capping A and then with Capping B. Washing was performed 4-5 times with acetonitrile.

4) Oxidation: An oxidant was added, the oxidation was performed for 2 min, and washing was performed 4-5 times with

acetonitrile.

The steps from 1) to 4) were repeated until the synthesis of all sequences was completed. Finally, DMT was removed with a decapping agent DBK, and washing was performed 4-5 times with acetonitrile.

Phosphorothioate bond formation: The oxidant was replaced with a sulfuration reagent, and the sulfuration time was set to 10 min, so as to complete the phosphorothioate bond formation.

**2.2 Synthesis of asRNA (antisense strand without ligand)**

On a K&A nucleic acid synthesizer, the CPG solid support described above was placed into a synthetic column, and synthesis was performed by referring to the method described in 2.1.1 or 2.1.2.

**2.3 Separation of ssRNA or asRNA from solid support**

The cleavage reagent was aqueous ammonia:ethanol = 3:1 (v:v, where the concentration of ammonium hydroxide was 25-28%). 5 mL of the cleavage reagent was added to every 100 mg of the solid support, and the mixture was stirred at 65-70 °C for 3 h. After the reaction was completed, the mixture was cooled to room temperature, precipitated in ice-cold n-butanol, and placed in a refrigerator at -20 °C for 30 min. The centrifugation was performed to give a precipitate, and n-butanol was added for washing; this procedure was repeated twice. Finally, the precipitate was washed with acetone and centrifuged to give a precipitate. The precipitate was dried *in vacuo* to give a crude product, which was then detected by mass spectrometry.

The LC-MS (negative ion mode) conditions are as follows:

Chromatographic column: HILIC chromatographic column
Mobile phase A: 20 mM ammonium formate, pH 6.2
Mobile phase B: 20 mM ammonium formate + 95% ACN
Column temperature: 45 °C
Flow rate: 1 mL/min.

**2.4. Annealing to form siRNA (dsRNA)**

The crude single-stranded RNA product obtained in step 2.3 was dissolved in water, and the support was removed by filtration through a membrane. The concentrations of the sense strand and the antisense strand were determined using a microspectrophotometer. The sense strand and the antisense strand were added (in a molar ratio of 1:1), mixed, denatured at 94 °C for 4 min, and annealed by naturally cooling to room temperature.

**2.5 Purification of siRNA (dsRNA)**

The crude product was purified by using a DNAPac RP 10 × 150 mm 4 μm chromatographic column (mobile phase A: 0.1 mol/L triethylamine, pH 8.0; mobile phase B: 0.1 mol/L triethylamine, pH 8.0 + 50% ACN). The detection wavelengths were 215 nm and 260 nm. The main peak was collected and concentrated by rotary evaporation to remove most of the solvent, so as to give the target product.

**2.6. Desalting and content assay**

The mixture was centrifuged in a 3K ultrafiltration centrifuge tube at 12000× g for 12 min, concentrated by ultrafiltration, and desalted, and after the mixture was concentrated by rotary evaporation, water was added for solvent exchange; the procedures were repeated 5 or more times.

Content assay: The concentration of the concentrated sample was determined using a microspectrophotometer. The double-stranded ribonucleic acids or the ligand conjugates thereof are shown below:

Table 8. Double-stranded ribonucleic acids or ligand conjugates thereof

| dsRNA No. | Sense strand SEQ ID NO | Sequence in the 5' to 3' direction | Antisense strand SEQ ID NO | Sequence in the 5' to 3' direction |
|---|---|---|---|---|
| TD01 | 1 | GAUCUCCAACUAAAAUACU | 2 | AGUAUUUUAGUUGGAGAUCCG |

(continued)

| dsRNA No. | Sense strand SEQ ID NO | Sequence in the 5' to 3' direction | Antisense strand SEQ ID NO | Sequence in the 5' to 3' direction |
|---|---|---|---|---|
| TD02 | 3 | GGAUGAUUUUCUUAUA UCA | 4 | UGAUAUAAGAAAA UCAUCCUG |
| TD03 | 5 | GGUCUUUUCAGGAUGA UUU | 6 | AAAUCAUCCUGAA AAGACCUU |
| TD04 | 7 | GUCUUUUCAGGAUGAU UUU | 8 | AAAAUCAUCCUGA AAAGACCU |
| TD05 | 9 | CUUUUCAGGAUGAUUU UCU | 10 | AGAAAAUCAUCCU GAAAAGAC |
| TD06 | 11 | UUUUCAGGAUGAUUUU CUU | 12 | AAGAAAAUCAUCC UGAAAAGA |
| TD07 | 13 | UCAGGAUGAUUUUCUU AUA | 14 | UAUAAGAAAAUCA UCCUGAAA |
| TD08 | 15 | CAGGAUGAUUUUCUUA UAU | 16 | AUAUAAGAAAAUC AUCCUGAA |
| TD09 | 17 | AGGAUGAUUUUCUUAU AUC | 18 | GAUAUAAGAAAAU CAUCCUGA |
| TD10 | 19 | GAUGAUUUUCUUAUAU CAA | 20 | UUGAUAUAAGAAA AUCAUCCU |
| TD11 | 21 | AUGAUUUUCUUAUAUC AAG | 22 | CUUGAUAUAAGAA AAUCAUCC |
| TD12 | 23 | UGAUUUUCUUAUAUCA AGU | 24 | ACUUGAUAUAAGA AAAUCAUC |
| TD13 | 25 | CUUAUAUCAAGUGGUA CAU | 26 | AUGUACCACUUGA UAUAAGAA |
| TD14 | 27 | AAACGGAUCUCCAACU AAA | 28 | UUUAGUUGGAGAU CCGUUUGA |
| TD01L | 1 | GAUCUCCAACUAAAAU ACUL | 2 | AGUAUUUUAGUUG GAGAUCCG |
| TD02L | 3 | GGAUGAUUUUCUUAUA UCAL | 4 | UGAUAUAAGAAAA UCAUCCUG |
| TD03L | 5 | GGUCUUUUCAGGAUGA UUUL | 6 | AAAUCAUCCUGAA AAGACCUU |
| TD04L | 7 | GUCUUUUCAGGAUGAU UUUL | 8 | AAAAUCAUCCUGA AAAGACCU |
| TD05L | 9 | CUUUUCAGGAUGAUUU UCUL | 10 | AGAAAAUCAUCCU GAAAAGAC |
| TD06L | 11 | UUUUCAGGAUGAUUUU CUUL | 12 | AAGAAAAUCAUCC UGAAAAGA |

(continued)

| dsRNA No. | Sense strand SEQ ID NO | Sequence in the 5' to 3' direction | Antisense strand SEQ ID NO | Sequence in the 5' to 3' direction |
|---|---|---|---|---|
| TD07L | 13 | UCAGGAUGAUUUUCUUAUAL | 14 | UAUAAGAAAAUCAUCCUGAAA |
| TD08L | 15 | CAGGAUGAUUUUCUUAUAUL | 16 | AUAUAAGAAAAUCAUCCUGAA |
| TD09L | 17 | AGGAUGAUUUUCUUAUAUCL | 18 | GAUAUAAGAAAAUCAUCCUGA |
| TD10L | 19 | GAUGAUUUUCUUAUAUCAAL | 20 | UUGAUAUAAGAAAAUCAUCCU |
| TD11L | 21 | AUGAUUUUCUUAUAUCAAGL | 22 | CUUGAUAUAAGAAAAUCAUCC |
| TD12L | 23 | UGAUUUUCUUAUAUCAAGUL | 24 | ACUUGAUAUAAGAAAAUCAUC |
| TD13L | 25 | CUUAUAUCAAGUGGUACAUL | 26 | AUGUACCACUUGAUAUAAGAA |
| TD14L | 27 | AAACGGAUCUCCAACUAAAL | 28 | UUUAGUUGGAGAUCCGUUUGA |
| TD01M01 | 29 | gsasucUfcCfAfAfcuaaaauacu | 30 | asGfsuauUfuUfAfguugGfaGfaucscsg |
| TD01M01L | 29 | gsasucUfcCfAfAfcuaaaauacuL | 30 | asGfsuauUfuUfAfguugGfaGfaucscsg |
| TD01M02 | 31 | gsasucucCfAfAfcuaaaauacu | 32 | asGfsuauUfuuaguugGfaGfaucscsg |
| TD01M02L | 31 | gsasucucCfAfAfcuaaaauacuL | 32 | asGfsuauUfuuaguugGfaGfaucscsg |
| TD02M01 | 33 | gsgsauGfaUfUfUfucuuauauca | 34 | usGfsauaUfaAfGfaaaaUfcAfuccsusg |
| TD02M01L | 33 | gsgsauGfaUfUfUfucuuauaucaL | 34 | usGfsauaUfaAfGfaaaaUfcAfuccsusg |
| TD02M02 | 35 | gsgsaugaUfUfUfucuuauauca | 36 | usGfsauaUfaagaaaaUfcAfuccsusg |
| TD02M02L | 35 | gsgsaugaUfUfUfucuuauaucaL | 36 | usGfsauaUfaagaaaaUfcAfuccsusg |
| TD03M01 | 37 | gsgsucUfuUfUfCfaggaugauuu | 38 | asAfsaucAfuCfCfugaaAfaGfaccsusu |
| TD03M01L | 37 | gsgsucUfuUfUfCfaggaugauuuL | 38 | asAfsaucAfuCfCfugaaAfaGfaccsusu |
| TD03M02 | 39 | gsgsucuuUfUfCfaggaugauuu | 40 | asAfsaucAfuccugaaAfaGfaccsusu |

(continued)

| dsRNA No. | Sense strand SEQ ID NO | Sequence in the 5' to 3' direction | Antisense strand SEQ ID NO | Sequence in the 5' to 3' direction |
|---|---|---|---|---|
| TD03M02L | 39 | gsgsucuuUfUfCfaggaugauuuL | 40 | asAfsaucAfuccugaaAfaGfaccsusu |
| TD04M01 | 41 | gsuscuUfuUfCfAfggaugauuuu | 42 | asAfsaauCfaUfCfcugaAfaAfgacscsu |
| TD04M01L | 41 | gsuscuUfuUfCfAfggaugauuuuL | 42 | asAfsaauCfaUfCfcugaAfaAfgacscsu |
| TD04M02 | 43 | gsuscuuuUfCfAfggaugauuuu | 44 | asAfsaauCfauccugaAfaAfgacscsu |
| TD04M02L | 43 | gsuscuuuUfCfAfggaugauuuuL | 44 | asAfsaauCfauccugaAfaAfgacscsu |
| TD05M01 | 45 | csusuuUfcAfGfGfaugauuuucu | 46 | asGfsaaaAfuCfAfuccuGfaAfaagsasc |
| TD05M01L | 45 | csusuuUfcAfGfGfaugauuuucuL | 46 | asGfsaaaAfuCfAfuccuGfaAfaagsasc |
| TD05M02 | 47 | csusuuucAfGfGfaugauuuucu | 48 | asGfsaaaAfucauccuGfaAfaagsasc |
| TD05M02L | 47 | csusuuucAfGfGfaugauuuucuL | 48 | asGfsaaaAfucauccuGfaAfaagsasc |
| TD06M01 | 49 | ususuuCfaGfGfAfugauuuucuu | 50 | asAfsgaaAfaUfCfauccUfgAfaaasgsa |
| TD06M01L | 49 | ususuuCfaGfGfAfugauuuucuuL | 50 | asAfsgaaAfaUfCfauccUfgAfaaasgsa |
| TD06M02 | 51 | ususuucaGfGfAfugauuuucuu | 52 | asAfsgaaAfaucauccUfgAfaaasgsa |
| TD06M02L | 51 | ususuucaGfGfAfugauuuucuuL | 52 | asAfsgaaAfaucauccUfgAfaaasgsa |
| TD07M01 | 53 | uscsagGfaUfGfAfuuuucuuaua | 54 | usAfsuaaGfaAfAfaucaUfcCfugasasa |
| TD07M01L | 53 | uscsagGfaUfGfAfuuuucuuauaL | 54 | usAfsuaaGfaAfAfaucaUfcCfugasasa |
| TD07M02 | 55 | uscsaggaUfGfAfuuuucuuaua | 56 | usAfsuaaGfaaaaucaUfcCfugasasa |
| TD07M02L | 55 | uscsaggaUfGfAfuuuucuuauaL | 56 | usAfsuaaGfaaaaucaUfcCfugasasa |
| TD08M01 | 57 | csasggAfuGfAfUfuuucuuauau | 58 | asUfsauaAfgAfAfaaucAfuCfcugsasa |
| TD08M01L | 57 | csasggAfuGfAfUfuuucuuauauL | 58 | asUfsauaAfgAfAfaaucAfuCfcugsasa |

(continued)

| dsRNA No. | Sense strand SEQ ID NO | Sequence in the 5' to 3' direction | Antisense strand SEQ ID NO | Sequence in the 5' to 3' direction |
|---|---|---|---|---|
| TD08M02 | 59 | csasggauGfAfUfuuucuuauau | 60 | asUfsauaAfgaaaaucAfuCfcugsasa |
| TD08M02L | 59 | csasggauGfAfUfuuucuuauauL | 60 | asUfsauaAfgaaaaucAfuCfcugsasa |
| TD09M01 | 61 | asgsgaUfgAfUfUfuucuuauauc | 62 | gsAfsuauAfaGfAfaaauCfaUfccusgsa |
| TD09M01L | 61 | asgsgaUfgAfUfUfuucuuauaucL | 62 | gsAfsuauAfaGfAfaaauCfaUfccusgsa |
| TD09M02 | 63 | asgsgaugAfUfUfuucuuauauc | 64 | gsAfsuauAfagaaaauCfaUfccusgsa |
| TD09M02L | 63 | asgsgaugAfUfUfuucuuauaucL | 64 | gsAfsuauAfagaaaauCfaUfccusgsa |
| TD10M01 | 65 | gsasugAfuUfUfUfcuuauaucaa | 66 | usUfsgauAfuAfAfgaaaAfuCfaucscsu |
| TD10M01L | 65 | gsasugAfuUfUfUfcuuauaucaaL | 66 | usUfsgauAfuAfAfgaaaAfuCfaucscsu |
| TD10M02 | 67 | gsasugauUfUfUfcuuauaucaa | 68 | usUfsgauAfuaagaaaAfuCfaucscsu |
| TD10M02L | 67 | gsasugauUfUfUfcuuauaucaaL | 68 | usUfsgauAfuaagaaaAfuCfaucscsu |
| TD11M01 | 69 | asusgaUfuUfUfCfuuauaucaag | 70 | csUfsugaUfaUfAfagaaAfaUfcauscsc |
| TD11M01L | 69 | asusgaUfuUfUfCfuuauaucaagL | 70 | csUfsugaUfaUfAfagaaAfaUfcauscsc |
| TD11M02 | 71 | asusgauuUfUfCfuuauaucaag | 72 | csUfsugaUfauaagaaAfaUfcauscsc |
| TD11M02L | 71 | asusgauuUfUfCfuuauaucaagL | 72 | csUfsugaUfauaagaaAfaUfcauscsc |
| TD12M01 | 73 | usgsauUfuUfCfUfuauaucaagu | 74 | asCfsuugAfuAfUfaagaAfaAfucasusc |
| TD12M01L | 73 | usgsauUfuUfCfUfuauaucaaguL | 74 | asCfsuugAfuAfUfaagaAfaAfucasusc |
| TD12M02 | 75 | usgsauuuUfCfUfuauaucaagu | 76 | asCfsuugAfuauaagaAfaAfucasusc |
| TD12M02L | 75 | usgsauuuUfCfUfuauaucaaguL | 76 | asCfsuugAfuauaagaAfaAfucasusc |
| TD13M01 | 77 | csusuaUfaUfCfAfagugguacau | 78 | asUfsguaCfcAfCfuugaUfaUfaagsasa |

(continued)

| dsRNA No. | Sense strand SEQ ID NO | Sequence in the 5' to 3' direction | Antisense strand SEQ ID NO | Sequence in the 5' to 3' direction |
|---|---|---|---|---|
| TD13M01L | 77 | csusuaUfaUfCfAfagugguaca uL | 78 | asUfsguaCfcAfCfuuga UfaUfaagsasa |
| TD13M02 | 79 | csusuauaUfCfAfagugguacau | 80 | asUfsguaCfcacuugaUfa Ufaagsasa |
| TD13M02L | 79 | csusuauaUfCfAfagugguacau L | 80 | asUfsguaCfcacuugaUfa Ufaagsasa |
| TD14M01 | 81 | asasacGfgAfUfCfuccaacuaaa | 82 | usUfsuagUfuGfGfagau CfcGfuuusgsa |
| TD14M01L | 81 | asasacGfgAfUfCfuccaacuaaa L | 82 | usUfsuagUfuGfGfagau CfcGfuuusgsa |
| TD14M02 | 83 | asasacggAfUfCfuccaacuaaa | 84 | usUfsuagUfuggagauCf cGfuuusgsa |
| TD14M02L | 83 | asasacggAfUfCfuccaacuaaa L | 84 | usUfsuagUfuggagauCf cGfuuusgsa |

Uppercase letters G, C, A, and U generally represent nucleotides comprising guanine, cytosine, adenine, and uracil as the base, respectively; lowercase letters g, c, a, and u represent 2'-methoxy-modified ribosyl groups of the nucleotides represented by the corresponding uppercase letters, respectively, i.e., g, c, a, and u represent 2'-O-methyl G, 2'-O-methyl C, 2'-O-methyl A, and 2'-O-methyl U, respectively; uppercase letters with a lowercase letter f on the right side represent 2'-fluoro-modified ribosyl groups of the nucleotides represented by the corresponding uppercase letters, i.e., Gf, Cf, Af, and Uf represent 2'-fluoro G, 2'-fluoro C, 2'-fluoro A, and 2'-fluoro U, respectively; lowercase letter s indicates that the two nucleotide residues adjacent to s are linked via a phosphorothioate group; for example, "csu" indicates that residues c and u are linked via a phosphorothioate group, and L represents the ligand, e.g., the L01 ligand and the L02 ligand with the structures as shown below:

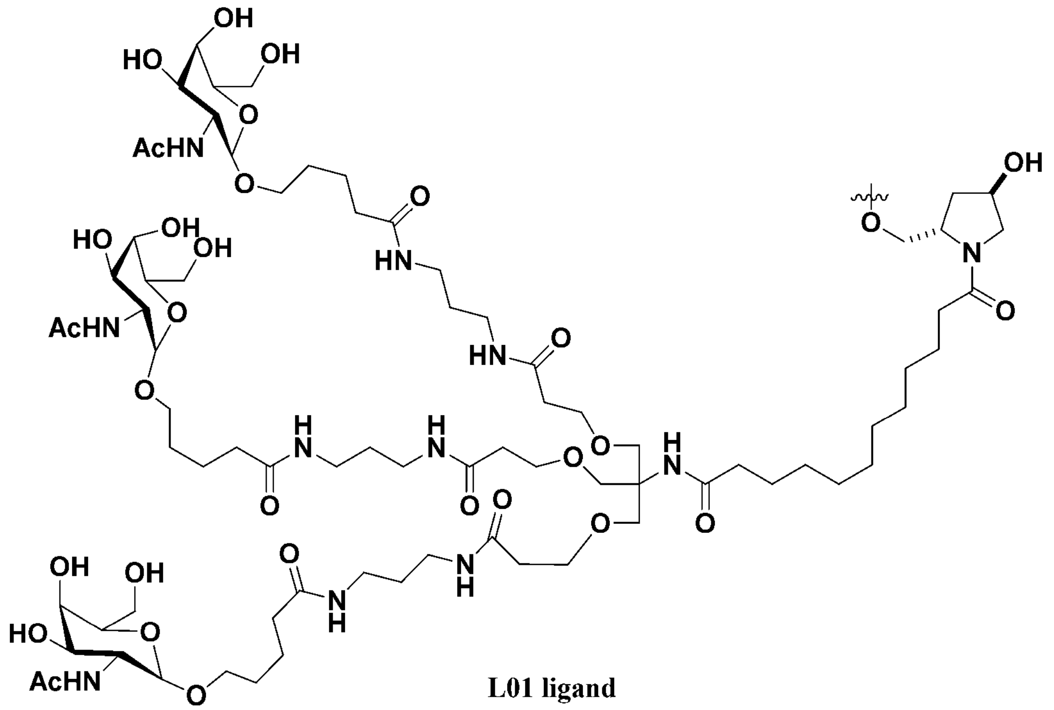

L01 ligand

and

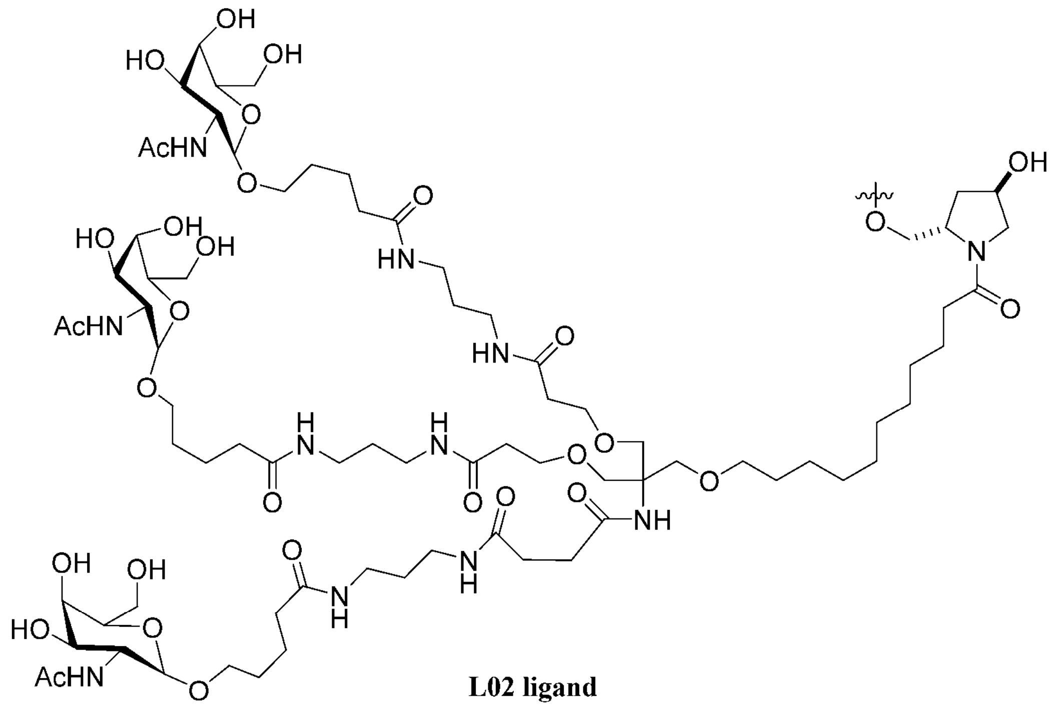

L02 ligand

.

The double-stranded ribonucleic acids, the pharmaceutically acceptable salts thereof, or the ligand conjugates thereof (the ligand L is selected from the group consisting of the L01 ligand and the L02 ligand) described above may be prepared according to the method described in the present application or a method known in the art.

The mass spectrometry data for the double-stranded ribonucleic acids, the pharmaceutically acceptable salts thereof, or the ligand conjugates thereof of the present application were as expected, which confirmed that the target products were obtained.

**Experimental Example 1. *In vitro* Screening**

Cell culture and plasmid/transfection

Cos7 cells (purchased from Nanjing Cobioer Biosciences Co., Ltd.) were cultured in a DMEM complete medium (Hyclone, Cat. No. SH30243.01) containing 10% fetal bovine serum (Gibco, Cat. No. 10099-141) in an incubator at 37 °C with 5% $CO_2$. When the cells grew to near confluence, they were treated with trypsin and released from the culture flask. The Cos7 cells were seeded into a 96-well plate at $2 \times 10^4$ cells/well and cultured overnight. When the cell density reached 70-80%, the pmirGLO-FXI plasmid containing the FXI gene sequence (GenBank accession # NM_000128.4) was transfected into the Cos7 cells. Transfection was performed with 0.3 μL of Lipofectamine 3000 transfection reagent (Invitrogen, Cat. No. L3000015) at a concentration of 50 ng plasmid/well as described by the manufacturer. 6-8 h after plasmid transfection, the medium was removed. siRNA was then transfected into the Cos7 cells that had been transfected with the pmirGLO-FXI plasmid. The siRNA transfection method was as follows: 0.5 μL of siRNA was added to 5 μL of OPTI-MEM medium, and 0.3 μL of Lipofectamine RNAiMAX transfection reagent (Invitrogen, Cat. No. 13778150) was added to 5 μL of OPTI-MEM medium; the two were mixed by pipetting and left to stand at room temperature for 5 min; the complex was added to the culture well, followed by the addition of 90 μL of DMEM complete medium per well, and the culture was performed for another 48 h; each siRNA was tested at 3 concentrations with 2 replicate wells, performing a single-dose experiment with final dsRNA concentrations of 10 nM, 1 nM, and 0.1 nM.

Detection

The medium in the culture wells was removed, and 150 μL of mixed solution (volume ratio of the two = 1:1) of Dual-Glo® Luciferase reagent (Promega) and DMEM complete medium was added to each well. After thorough mixing and incubation at room temperature for 10 min, the chemiluminescence value (Fir) was read using a Spark multifunctional microplate reader (Tecan). Then, 75 μL of Dual-Glo® Stop&Glo® reagent (Promega) was added to each well. After thorough mixing and incubation at room temperature for 10 min, the chemiluminescence value (Ren) was read using the Spark multifunctional microplate reader.

The luminescence ratio per well was calculated as Ratio = Fir/Ren. The luminescence ratio Ratio (test) or Ratio (control) for each test group or control group was the average value of the Ratios for each group. Based on the luminescence ratio of the control group, the luminescence ratios of the test groups were normalized to obtain the ratio R = Ratio (test)/Ratio (control), which represented the expression level of the firefly reporter gene. Inhibition rate (%) of siRNA = (1 - R) × 100%. The results are shown in Table 9.

Table 9. Silencing efficiency

| dsRNA No. | Silencing efficiency (%) | | |
|---|---|---|---|
| | 10 nM | 1 nM | 0.1 nM |
| TD01 | 94.0±0.6 | 88.5±.3 | 76.1±0.5 |
| TD02 | 95.6±0.2 | 90.5±1.1 | 81.3±1.2 |

The experimental results show that the double-stranded ribonucleic acids of the present application have good *in vitro* silencing efficiency.

**Experimental Example 2. Dual-Luciferase Assay**

Cell culture and transfection

Cos7 cells (purchased from Nanjing Cobioer Biosciences Co., Ltd.) were cultured in a DMEM complete medium (Hyclone, Cat. No. SH30243.01) containing 10% fetal bovine serum (Gibco, Cat. No. 10099-141) in an incubator at 37 °C with 5% $CO_2$. When the cells grew to near confluence, they were treated with trypsin and released from the culture flask. The Cos7 cells were seeded into a 96-well plate at $2 \times 10^4$ cells/well and cultured overnight. When the cell density reached 70-80%, the pmirGLO-FXI plasmid containing the FXI gene sequence (GenBank accession # NM_000128.4) was transfected into the Cos7 cells. Transfection was performed with 0.3 μL of Lipofectamine 3000 transfection reagent (Invitrogen, Cat. No. L3000015) at a concentration of 50 ng plasmid/well as described by the manufacturer. 6-8 h after plasmid transfection, the medium was removed. siRNA was then transfected into the Cos7 cells that had been transfected with the pmirGLO-FXI plasmid. The siRNA transfection method was as follows: siRNA was diluted 5-fold with enzyme-free water to concentration ranges from 2 μM to 0.0000256 μM; 0.5 μL of siRNA was added to 5 μL of OPTI-MEM medium, and 0.3 μL of Lipofectamine RNAiMAX transfection reagent (Invitrogen, Cat. No. 13778150) was added to 5 μL of OPTI-MEM medium; the two were mixed by pipetting and left to stand at room temperature for 5 min; the complex was added to the

culture well, followed by the addition of 90 μL of DMEM complete medium per well, and the culture was performed for another 48 h; each siRNA was tested at 8 concentrations.

Detection

The medium in the culture wells was removed, and 150 μL of mixed solution (volume ratio of the two = 1:1) of Dual-Glo® Luciferase reagent (Promega) and DMEM complete medium was added to each well. After thorough mixing and incubation at room temperature for 10 min, the chemiluminescence value (Fir) was read using a Spark multifunctional microplate reader (Tecan). Then, 75 μL of Dual-Glo® Stop&Glo® reagent (Promega) was added to each well. After thorough mixing and incubation at room temperature for 10 min, the chemiluminescence value (Ren) was read using the Spark multifunctional microplate reader.

The luminescence ratio per well was calculated as Ratio = Fir/Ren. The luminescence ratio Ratio (test) or Ratio (control) for each test group or control group was the average value of the Ratios for each group. Based on the luminescence ratio of the control group, the luminescence ratios of the test groups were normalized to obtain the ratio R = Ratio (test)/Ratio (control), which represented the expression level of the firefly reporter gene. Inhibition rate (%) of siRNA = (1 - R) × 100%. The concentration-inhibition rate (%) data were processed using EXCEL software, and the $EC_{50}$ of the siRNA was calculated via a four-parameter nonlinear regression model. The results are shown in Table 10.

Table 10. $EC_{50}$ by dual-luciferase assay

| dsRNA No. | $EC_{50}$(pM) |
|---|---|
| TD01M01 | 37.21 |
| TD02M01 | 15.51 |
| TD01M02 | 40.78 |
| TD02M02 | 18.46 |
| TD03M01 | 29.5 |
| TD04M01 | 39.3 |
| TD05M01 | 83.17 |
| TD06M01 | 43.39 |
| TD07M01 | 35.69 |
| TD08M01 | 16.4 |
| TD09M01 | 24.05 |
| TD10M01 | 31.74 |
| TD11M01 | 142.2 |
| TD12M01 | 34.87 |
| TD13M01 | 30.56 |
| TD14M01 | 90.58 |

The *in vitro* screening results show that the double-stranded ribonucleic acids of the present application have good *in vitro* silencing activity.

**Experimental Example 3. Evaluation of *In Vivo* Activity in Human Coagulation Factor XI Model Mice**

The humanized mice used in this experimental example were purchased from Cyagen Biosciences Co., Ltd. The humanized mice were randomly divided into groups, with 4 mice per group. The compound and PBS control (pH = 7.2) were administered to each group of mice, respectively. The day of the first administration was designated as day 0 of the experiment. The corresponding compound solutions were administered via a single subcutaneous injection based on animal body weight, at administration doses of 1 mg/kg and 5 mg/kg. PBS control mice were each administered PBS, with an administration volume of 10 mL/kg body weight. Orbital blood was collected before administration and on day 7, day 14, day 28, day 42, and day 63 after administration. Plasma was separated, and the content of the coagulation factor XI protein in the plasma was measured at each time point.

About 165 μL of blood was collected from the orbital veins for plasma collection. A 3.8% trisodium citrate anticoagulant was added to the whole blood in a volume ratio of 1:9 (anticoagulant:plasma, v/v), and the mixture was centrifuged at 3000 rpm at 20 °C for 15 min to collect plasma. The content of the coagulation factor XI protein in the plasma obtained from the above groups of mice (test groups administered the compound and PBS control group) was measured using a Human Coagulation Factor XI ELISA kit (Sigma, Cat. No. RAB1385-1KT).

The coagulation factor XI protein inhibition rate was calculated according to the following equation:

Coagulation factor XI protein inhibition rate = (1 - protein content of test group/protein content of control group) × 100%.

Table 11 lists the measurement of FXI protein content in animal plasma before and on day 7, day 14, day 28, day 42, and day 63 after a single subcutaneous administration of siRNA ligand conjugates (TD01M01L01, TD02M01L01) at doses of 1 mg/kg and 5 mg/kg to humanized homozygous mice. As shown in Table 12, the results confirm that after the administration of a single dose of the dsRNA ligand conjugates of the present application, an excellent effect of inhibiting the expression of the human coagulation factor XI protein was demonstrated.

Table 11. *In vivo* experimental design

| Group | Number of animals (mice) | Administration Regimen | | | | | Blood collection schedule |
|---|---|---|---|---|---|---|---|
| | | Dose (mg/kg) | Administration volume (mL/kg) | Route of administration | Frequency of administration | Duration | |
| PBS | 4 | / | 10 | Subcutaneous | Once | Day 0 | Day -4, day 7, day 14, day 28, day 42, day 63 |
| TD01M01L01 | 4 | 1 | 10 | Subcutaneous | Once | Day 0 | |
| | 4 | 5 | 10 | Subcutaneous | Once | Day 0 | |
| TD01M02L01 | 4 | 1 | 10 | Subcutaneous | Once | Day 0 | |
| | 4 | 5 | 10 | Subcutaneous | Once | Day 0 | |
| TD02M01L01 | 4 | 1 | 10 | Subcutaneous | Once | Day 0 | |
| | 4 | 5 | 10 | Subcutaneous | Once | Day 0 | |
| TD02M02L01 | 4 | 1 | 10 | Subcutaneous | Once | Day 0 | |
| | 4 | 5 | 10 | Subcutaneous | Once | Day 0 | |

Table 12. Inhibition on expression effect of coagulation factor XI protein in mouse plasma

| Group | Dose (mg/kg) | Relative inhibition rate (%) of FXI protein | | | | |
|---|---|---|---|---|---|---|
| | | D7 | D14 | D28 | D42 | D63 |
| TD01M01L01 | 1 | 58.1% | 67.5% | 58.6% | 68.8% | 41.3% |
| | 5 | 90.5% | 92.8% | 89.5% | 91.8% | 74.4% |
| TD01M02L01 | 1 | 65.1% | 67.4% | 62.5% | 69.3% | 44.6% |
| | 5 | 90.9% | 93.1% | 92.4% | 93.1% | 76.6% |
| TD02M01L01 | 1 | 61.5% | 63.9% | 49.2% | 52.9% | 41.1% |
| | 5 | 91.8% | 94.0% | 87.8% | 89.4% | 69.0% |
| TD02M02L01 | 1 | 66.7% | 70.2% | 58.6% | 70.5% | 47.0% |
| | 5 | 91.5% | 93.5% | 88.4% | 86.9% | 66.3% |

**Experimental Example 4. Evaluation of *In Vivo* Activity in Human Coagulation Factor XI Model Mice**

The humanized mice used in this experimental example were purchased from Cyagen Biosciences Co., Ltd. The female humanized homozygous mice were randomly divided into groups, with 4 mice per group. The compound and PBS control (pH = 7.2) were administered to each group of mice, respectively. The day of the first administration was designated

as day 0 of the experiment. The corresponding compound solutions were administered via a single subcutaneous injection based on animal body weight, at an administration dose of 3 mg/kg. PBS control mice were each administered PBS, with an administration volume of 10 mL/kg body weight. Orbital blood was collected before administration and on day 3, day 7, day 14, day 21, day 28, day 42, day 56, and day 70 after administration. Serum was separated, and the content of the coagulation factor XI protein in the serum at each time point was measured using a Human Coagulation Factor XI ELISA kit (Thermo, Batch No. 2350072722, Cat. No. EH118RB).

The coagulation factor XI protein inhibition rate was calculated according to the following equation:

Coagulation factor XI protein inhibition rate = (1 - protein content of test group/protein content of control group) × 100%.

Table 13 lists the measurement of coagulation factor XI protein content in animal plasma before and on day 3, day 7, day 14, day 21, day 28, day 42, day 56, and day 70 after a single subcutaneous administration of siRNA ligand conjugates at a dose of 3 mg/kg to humanized homozygous mice. As shown in Table 14, the results confirm that after the administration of a single dose of the siRNA ligand conjugates of the present application, an excellent effect of inhibiting the expression of the human coagulation factor XI protein was demonstrated.

Table 13. *In vivo* experimental design

| Group | Number of animals (mice) | Administration Regimen | | | | | Blood collection schedule |
|---|---|---|---|---|---|---|---|
| | | Dose (mg/kg) | Administration volume (mL/kg) | Route of administration | Frequency of administration | Duration | |
| PBS | 4 | / | 10 | Subcutaneous | Once | Day 0 | Day -4, day 3, day 7, day 14, day 21, day 28, day 42, day 56, day 70 |
| TD01M02 L01 | 4 | 3 | 10 | Subcutaneous | Once | Day 0 | |
| TD02M02 L01 | 4 | 3 | 10 | Subcutaneous | Once | Day 0 | |

Table 14. Inhibition on expression effect of coagulation factor XI protein in mouse plasma

| Group | Relative inhibition rate (%) of FXI protein | | | | | | | |
|---|---|---|---|---|---|---|---|---|
| | D3 | D7 | D14 | D21 | D28 | D42 | D56 | D70 |
| TD01M02L01 | 69.3% | 88.5% | 91.8% | 91.7% | 91.3% | 86.1% | 76.1% | 68.9% |
| TD02M02L01 | 74.4% | 93.7% | 86.4% | 88.0% | 84.9% | 63.3% | 50.4% | 21.4% |

**Experimental Example 5. Evaluation of *In Vivo* Activity in AAV-FXI Model Mice**

4-6 week old C57BL/6 male mice were injected via the tail vein with $1 \times 10^{11}$ vg of AAV8-FXI (WZ Biosciences). On day 28 after modeling, mice with an hFXI protein content of greater than 100 ng/mL were selected. The mice were randomly divided into groups based on body weight and were injected with 3 mg/kg of the dsRNA ligand conjugate of the present application (amount calculated based on siRNA only) on day 0. Orbital blood was collected on day 7, day 14, day 35, and day 56. Serum was separated, and the content of the coagulation factor XI protein in the serum at each time point was measured using a Human Coagulation Factor XI ELISA kit (Thermo, Cat. No. EH118RB).

The coagulation factor XI protein inhibition rate was calculated according to the following equation:

Coagulation factor XI protein inhibition rate = (1 - protein content of test group/protein content of control group) × 100%.

The content of the coagulation factor XI protein in animal serum was measured before and on day 7, day 14, day 35, and day 56 after a single subcutaneous administration of the dsRNA ligand conjugate of the present application at a dose of 3 mg/kg to AAV-FXI mice (see Table 15). As shown in Table 16, the results confirm that after the administration of a

single dose of the dsRNA ligand conjugates of the present application, an excellent effect of inhibiting the expression of the human coagulation factor XI protein was demonstrated.

Table 15. *In vivo* experimental design

| Group | Number of animals (mice) | Administration Regimen | | | | | Blood collection schedule |
|---|---|---|---|---|---|---|---|
| | | Dose (mg/kg) | Administration volume (mL/kg) | Route of administration | Frequency of administration | Duration | |
| PBS(pH7.2) | 3 | / | 10 | Subcutaneous | Once | Day 0 | Day -7, day 7, day 14, day 35, day 56 |
| TD02M02L01 | 3 | 3 | 10 | Subcutaneous | Once | Day 0 | |
| TD05M02L01 | 3 | 3 | 10 | Subcutaneous | Once | Day 0 | |
| TD12M02L01 | 3 | 3 | 10 | Subcutaneous | Once | Day 0 | |

Table 16. Inhibition on expression effect of coagulation factor XI protein in mouse serum

| Group | Relative inhibition rate (%) of FXI protein | | | |
|---|---|---|---|---|
| | D7 | D14 | D35 | D56 |
| TD02M02L01 | 96.3% | 97.3% | 86.0% | 57.4% |
| TD05M02L01 | 96.3% | 96.6% | 81.7% | 51.0% |
| TD12M02L01 | 88.4% | 88.0% | / | / |

**Experimental Example 6. Evaluation of *In Vivo* Activity in Cynomolgus Monkeys**

On day 7 before administration, female cynomolgus monkeys weighing 2-4 kg were fasted overnight, and about 1 mL of blood was collected from the femoral vein. Plasma was collected by centrifugation. FXI protein (Human Coagulation Factor XI ELISAkit, Thermo, Cat. No. EH118RB), FXI activity (coagulation factor XI assay kit, Stago, Cat. No. 00723), and APTT (activated partial thromboplastin time assay kit, Stago, Cat. No. 00595) were measured. The monkeys were evenly divided into groups based on these indicators. On day 0, the dsRNA ligand conjugates of the present application (TD02M02L01, etc.) were injected. On day 0, day 3, day 7, day 14, day 21, day 28, day 37, day 54, and day 68, plasma was collected to measure plasma FXI protein, FXI activity, and activated partial thromboplastin time (APTT).

The coagulation factor XI protein inhibition rate was calculated according to the following equation:

Coagulation factor XI protein inhibition rate = (protein content after administration/protein content before administration - 1) $\times$ 100%.

The coagulation factor XI activity inhibition rate was calculated according to the following equation:

Coagulation factor XI activity inhibition rate = (activity level after administration/activity level before administration - 1) $\times$ 100%.

The activated partial thromboplastin time prolongation rate was calculated according to the following equation:

Activated partial thromboplastin time prolongation rate = (APTT value after administration/APTT value beforeadministration - 1) $\times$ 100%.

The content of the coagulation factor XI protein, the FXI activity, and the activated partial thromboplastin time in animal plasma were measured before and on day 0, day 3, day 7, day 14, day 21, day 28, day 37, day 54, and day 68 after the subcutaneous administration of the dsRNA ligand conjugates of the present application at doses of 4.2 mg/kg and 14 mg/kg to cynomolgus monkeys (Table 17). As shown in Tables 18-20, the results confirm that after administration of the dsRNA ligand conjugates of the present application, an excellent effect of inhibiting the expression and activity of the human coagulation factor XI protein was demonstrated, and the activated partial thromboplastin time (APTT) was significantly prolonged.

Table 17. *In vivo* experimental design

| Group | Number of animals (mice) | Administration Regimen | | | | | Blood collection schedule |
|---|---|---|---|---|---|---|---|
| | | Dose (mg/kg) | Administration volume (mL/kg) | Route of administration | Frequency of administration | Duration | |
| TD02M02L01 | 3 | 4.2 | 0.7 | Subcutaneous | Once | Day 0 | Day -7, day 0, day 3, day 7, day 14, day 21, day 28, day 37, day 54, day 68 |
| TD02M02L01 | 3 | 14 | 0.7 | Subcutaneous | Once | Day 0 | |

Table 18. Inhibition on expression effect of coagulation factor XI protein in cynomolgus monkey plasma

| Group | Relative inhibition rate (%) of FXI protein | | | | | | | |
|---|---|---|---|---|---|---|---|---|
| | D3 | D7 | D14 | D21 | D28 | D37 | D54 | D68 |
| TD02M02L01 | 50.3% | 80.6% | 94.8% | 96.9% | 96.2% | 97.0% | 95.6% | 94.9% |
| TD02M02L01 | 46.3% | 82.9% | 96.6% | 98.4% | 98.1% | 98.5% | 98.5% | 97.6% |

Table 19. Inhibition on activity effect of coagulation factor XI in cynomolgus monkey plasma

| Group | Relative inhibition rate (%) of FXI activity | | | | | | |
|---|---|---|---|---|---|---|---|
| | D3 | D7 | D14 | D21 | D28 | D37 | D54 |
| TD02M02L01 | 20.5% | 66.0% | 84.7% | 88.9% | 89.2% | 92.6% | 91.4% |
| TD02M02L01 | 28.4% | 71.3% | 88.3% | 92.4% | 90.8% | 96.2% | 95.1% |

Table 20. Prolongation of activated partial thromboplastin time in cynomolgus monkey plasma

| Group | Relative prolongation rate (%) of APTT | | | | | | | |
|---|---|---|---|---|---|---|---|---|
| | D3 | D7 | D14 | D21 | D28 | D37 | D54 | D68 |
| TD02M02L01 | 8.2% | 34.5% | 66.0% | 73.2% | 97.4% | 88.4% | 95.5% | 112.3% |
| TD02M02L01 | 6.0% | 25.2% | 60.9% | 116.0% | 70.6% | 100.3% | 124.4% | 132.4% |

**Experimental Example 7. *In Vitro* Stability Evaluation in Liver Homogenate**

1) 1 mL of tissue lysis buffer (100 mM Tris-HCl, 1 mM $MgCl_2$, pH 6.0) was added as per 200 mg of liver tissue (C57BL/6 mice), and the tissue was ground by a tissue grinder at -30 °C. The resultant liquid, i.e., blank liver homogenate, was collected and preserved at -80 °C for later use.

2) 17 μL of stock solution (concentration: 4.1 mg/mL) of the dsRNA ligand conjugate to be tested was pipetted, and 680 μL of enzyme-free water was added to dilute the stock solution to 100 μg/mL for later use. The final concentration of the compound to be tested in the system was 10 μg/mL (1:10).

3) The blank mouse liver homogenate was taken and incubated at 37 °C for 5 min, 18 h, 21 h, and 24 h.

4) 540 μL of blank liver homogenate incubated for 24 h was pipetted into a 1.5 mL Eppendorf tube, and 60 μL of the stock solution of the compound to be tested (100 μg/mL) was added. The mixture was thoroughly mixed by vortexing to give the 0 h sample.

5) 540 μL of blank liver homogenate incubated for 21 h was pipetted into a 1.5 mL Eppendorf tube, and 60 μL of the stock solution of the compound to be tested (100 μg/mL) was added. The mixture was thoroughly mixed by vortexing and incubated on a shaker (37 °C, 300 rpm) for 3 h to give the 3 h sample.

6) 540 μL of blank liver homogenate incubated for 18 h was pipetted into a 1.5 mL Eppendorf tube, and 60 μL of the stock solution of the compound to be tested (100 μg/mL) was added. The mixture was thoroughly mixed by vortexing and incubated on a shaker (37 °C, 300 rpm) for 6 h to give the 6 h sample.

7) 540 μL of blank liver homogenate incubated for 5 min was pipetted into a 1.5 mL Eppendorf tube, and 60 μL of the stock solution of the compound to be tested (100 μg/mL) was added. The mixture was thoroughly mixed by vortexing and incubated on a shaker (37 °C, 300 rpm) for 24 h to give the 24 h sample.

8) Sample pretreatment:

(1) All the samples described above were thoroughly mixed by vortexing. 50 μL of the sample to be tested was pipetted, and 150 μL of Clarity lysis buffer (phenomenex, Cat. No. AL0-8579) was added. The mixture was vortexed for 5 min and centrifuged at 15000 rpm for 10 min.

(2) 1 mL of methanol and 1 mL of equilibration buffer (50 mM ammonium acetate (pH = 5.5) + 0.5% Triton_X100) were added in sequence to the Clarity OTX SPE plate (phenomenex, Cat. No. 8E-S103-EGA).

(3) 185 μL of supernatant obtained after the centrifugation in step 1) was loaded on the SPE kit.

(4) The sample was washed 3 times with 1 mL of equilibration buffer (50 mM ammonium acetate (pH = 5.5)) and then washed 3 times with 1 mL of buffer (50 mM ammonium acetate (pH = 5.5) + 50% acetonitrile).

(5) The sample was eluted twice with 1 mL of eluent (100 mM ammonium bicarbonate (pH = 9.5) + 40% acetonitrile + 10% tetrahydrofuran).

(6) Finally, the sample was dried with nitrogen at 65 °C, and 100 μL of TE buffer (10 mM Tris-HCl + 1 mM EDTA (pH = 8.0)) was added for reconstitution. The mixture was vortexed for 5 min and centrifuged at 15000 rpm for 10 min. The supernatant was collected and loaded for analysis.

(9) The sample concentration was detected by LC-MS/MS, with the zero-point sample as the reference. The peak area of the analyte was used to calculate the remaining percentage, and the data were analyzed.

According to the assay results, the dsRNA ligand conjugates of the present application were found to have good stability: the remaining amount of the sense strand after 24 h of incubation exceeded 50%, and the remaining amount of the antisense strand after 24 h of incubation exceeded 80%.

### Experimental Example 8. Evaluation of *In Vitro* On-Target and Off-Target Effects

Cell culture and transfection

Cos7 cells (purchased from Nanjing Cobioer Biosciences Co., Ltd.) were cultured in a DMEM complete medium (Hyclone, Cat. No. SH30243.01) containing 10% fetal bovine serum (Gibco, Cat. No. 10099-141) in an incubator at 37 °C with 5% $CO_2$. The Cos7 cells were seeded into a 96-well plate at $2 \times 10^4$ cells/well and cultured overnight. The reporter gene plasmid (on-target/off-target plasmid) was transfected into the Cos7 cells using 0.3 μL of Lipofectamine 3000 transfection reagent (Invitrogen, Cat. No. L3000015). 6-8 h after plasmid transfection, the medium was removed. siRNAs with different concentrations were transfected into the Cos7 cells comprising the reporter gene plasmid using Lipofectamine RNAiMAX transfection reagent (Invitrogen, Cat. No. 13778150). The administration concentration for the on-target plasmid was 10 nM-0.00457 nM, and the administration concentration range for the off-target plasmid was 40 nM-0.0183 nM. The cells were cultured at 37 °C with 5% $CO_2$ for 48 h.

Detection

Detection was performed using the dual-luciferase assay kit (Promega, Cat. No. E2940). The luminescence ratio per well was calculated as Ratio = Fir/Ren. Based on the luminescence ratio of the control group, the luminescence ratios

of the test groups were normalized to obtain the ratio R = Ratio (test)/Ratio (control), which represented the expression level of the firefly reporter gene. Inhibition rate (%) of siRNA = (1 - R) × 100%.

The construction principle of the on-target plasmid GSCM was to insert a sequence complementary to the antisense strand into the 3' untranslated region of the firefly luciferase in the pmirGLO plasmid. The construction principle of the off-target plasmid GSSM was to insert a sequence that completely matches positions 1-8 of the 5' end of the antisense strand but does not match at all at other sites into the 3' untranslated region of the firefly luciferase in the pmirGLO plasmid. In order to improve sensitivity, GSSM-X5 was constructed by linking 5 identical GSSM sequences. The construction principle of the off-target plasmid PSCM was to insert a sequence complementary to the sense strand into the 3' untranslated region of the firefly luciferase in the pmirGLO plasmid. The construction principle of the off-target plasmid PSSM was to insert a sequence that completely matches positions 1-8 of the 5' end of the sense strand but does not match at all at other sites into the 3' untranslated region of the firefly luciferase in the pmirGLO plasmid.

The experimental results show that the double-stranded ribonucleic acids (dsRNAs) of the present application have no significant off-target effects.

## Claims

1. A double-stranded ribonucleic acid (dsRNA), a pharmaceutically acceptable salt thereof, or a ligand conjugate thereof, wherein the dsRNA comprises a sense strand and an antisense strand, wherein the sense strand comprises at least 15 contiguous nucleotides in the nucleotide sequence set forth in SEQ ID NO. **1,** SEQ ID NO. 3, SEQ ID NO. 5, SEQ ID NO. 7, SEQ ID NO. 9, SEQ ID NO. 11, SEQ ID NO. 13, SEQ ID NO. 15, SEQ ID NO. 17, SEQ ID NO. 19, SEQ ID NO. 21, SEQ ID NO. 23, SEQ ID NO. 25, or SEQ ID NO. 27 and has a length of no more than 21 nucleotides, and the antisense strand comprises at least 15 contiguous nucleotides in the nucleotide sequence set forth in SEQ ID NO. 2, SEQ ID NO. 4, SEQ ID NO. 6, SEQ ID NO. 8, SEQ ID NO. 10, SEQ ID NO. 12, SEQ ID NO. 14, SEQ ID NO. 16, SEQ ID NO. 18, SEQ ID NO. 20, SEQ ID NO. 22, SEQ ID NO. 24, SEQ ID NO. 26, or SEQ ID NO. 28 and has a length of no more than 23 nucleotides:

   sense strand: 5'-GAUCUCCAACUAAAAUACU-3' (SEQ ID NO. 1),
   antisense strand: 5'-AGUAUUUUAGUUGGAGAUCCG-3' (SEQ ID NO. 2);
   sense strand: 5'-GGAUGAUUUUCUUAUAUCA-3' (SEQ ID NO. 3),
   antisense strand: 5'-UGAUAUAAGAAAAUCAUCCUG-3' (SEQ ID NO. 4);
   sense strand: 5'-GGUCUUUUCAGGAUGAUUU-3' (SEQ ID NO. 5),
   antisense strand: 5'-AAAUCAUCCUGAAAAGACCUU-3' (SEQ ID NO. 6);
   sense strand: 5'-GUCUUUUCAGGAUGAUUUU-3' (SEQ ID NO. 7),
   antisense strand: 5'-AAAAUCAUCCUGAAAAGACCU-3' (SEQ ID NO. 8);
   sense strand: 5'-CUUUUCAGGAUGAUUUUCU-3' (SEQ ID NO. 9),
   antisense strand: 5'-AGAAAAUCAUCCUGAAAAGAC-3' (SEQ ID NO. 10);
   sense strand: 5'-UUUUCAGGAUGAUUUUCUU-3' (SEQ ID NO. 11),
   antisense strand: 5'-AAGAAAAUCAUCCUGAAAAGA-3' (SEQ ID NO. 12);
   sense strand: 5'-UCAGGAUGAUUUUCUUAUA-3' (SEQ ID NO. 13),
   antisense strand: 5'-UAUAAGAAAAUCAUCCUGAAA-3' (SEQ ID NO. 14);
   sense strand: 5'-CAGGAUGAUUUUCUUAUAU-3' (SEQ ID NO. 15),
   antisense strand: 5'-AUAUAAGAAAAUCAUCCUGAA-3' (SEQ ID NO. 16);
   sense strand: 5'-AGGAUGAUUUUCUUAUAUC-3' (SEQ ID NO. 17),
   antisense strand: 5'-GAUAUAAGAAAAUCAUCCUGA-3' (SEQ ID NO. 18);
   sense strand: 5'-GAUGAUUUUCUUAUAUCAA-3' (SEQ ID NO. 19),
   antisense strand: 5'-UUGAUAUAAGAAAAUCAUCCU-3' (SEQ ID NO. 20);
   sense strand: 5'-AUGAUUUUCUUAUAUCAAG-3' (SEQ ID NO. 21),
   antisense strand: 5'-CUUGAUAUAAGAAAAUCAUCC-3' (SEQ ID NO. 22);
   sense strand: 5'-UGAUUUUCUUAUAUCAAGU-3' (SEQ ID NO. 23),
   antisense strand: 5'-ACUUGAUAUAAGAAAAUCAUC-3' (SEQ ID NO. 24);
   sense strand: 5'-CUUAUAUCAAGUGGUACAU-3' (SEQ ID NO. 25),
   antisense strand: 5'-AUGUACCACUUGAUAUAAGAA-3' (SEQ ID NO. 26);
   sense strand: 5'-AAACGGAUCUCCAACUAAA-3' (SEQ ID NO. 27),
   antisense strand: 5'-UUUAGUUGGAGAUCCGUUUGA-3' (SEQ ID NO. 28);

   the sense strand or the antisense strand is optionally modified.

2. The double-stranded ribonucleic acid, the pharmaceutically acceptable salt thereof, or the ligand conjugate thereof according to claim 1, wherein the sense strand comprises the nucleotide sequence set forth in SEQ ID NO. **1,** SEQ ID NO. 3, SEQ ID NO. 5, SEQ ID NO. 7, SEQ ID NO. 9, SEQ ID NO. 11, SEQ ID NO. 13, SEQ ID NO. 15, SEQ ID NO. 17, SEQ ID NO. 19, SEQ ID NO. 21, SEQ ID NO. 23, SEQ ID NO. 25, or SEQ ID NO. 27 and has a length of no more than 21 nucleotides, and the antisense strand comprises the nucleotide sequence set forth in SEQ ID NO. 2, SEQ ID NO. 4, SEQ ID NO. 6, SEQ ID NO. 8, SEQ ID NO. 10, SEQ ID NO. 12, SEQ ID NO. 14, SEQ ID NO. 16, SEQ ID NO. 18, SEQ ID NO. 20, SEQ ID NO. 22, SEQ ID NO. 24, SEQ ID NO. 26, or SEQ ID NO. 28 and has a length of no more than 23 nucleotides.

3. The double-stranded ribonucleic acid, the pharmaceutically acceptable salt thereof, or the ligand conjugate thereof according to claim 1, wherein the sense strand is the nucleotide sequence set forth in SEQ ID NO. 1, SEQ ID NO. 3, SEQ ID NO. 5, SEQ ID NO. 7, SEQ ID NO. 9, SEQ ID NO. 11, SEQ ID NO. 13, SEQ ID NO. 15, SEQ ID NO. 17, SEQ ID NO. 19, SEQ ID NO. 21, SEQ ID NO. 23, SEQ ID NO. 25, or SEQ ID NO. 27, and the antisense strand is the nucleotide sequence set forth in SEQ ID NO. 2, SEQ ID NO. 4, SEQ ID NO. 6, SEQ ID NO. 8, SEQ ID NO. 10, SEQ ID NO. 12, SEQ ID NO. 14, SEQ ID NO. 16, SEQ ID NO. 18, SEQ ID NO. 20, SEQ ID NO. 22, SEQ ID NO. 24, SEQ ID NO. 26, or SEQ ID NO. 28.

4. The double-stranded ribonucleic acid, the pharmaceutically acceptable salt thereof, or the ligand conjugate thereof according to any one of claims 1-3, wherein the sense strand or the antisense strand comprises an overhang at the 5' end and/or the 3' end.

5. The double-stranded ribonucleic acid, the pharmaceutically acceptable salt thereof, or the ligand conjugate thereof according to claim 4, wherein the overhang comprises 1, 2, 3, 4, or 5 nucleotides; or, the overhang comprises 1 or 2 nucleotides; or, the overhang is 1, 2, 3, 4, or 5 nucleotides at the 5' end and/or the 3' end of the sense strand or the antisense strand; or, the dsRNA, the pharmaceutically acceptable salt thereof, or the ligand conjugate thereof comprises an additional sequence as the overhang.

6. The double-stranded ribonucleic acid, the pharmaceutically acceptable salt thereof, or the ligand conjugate thereof according to claim 4 or 5, wherein the additional sequence in the overhang is selected from the group consisting of unmodified or modified A, G, C, U, and T; or is selected from the group consisting of unmodified or modified U and T; or when the overhang has 1 nucleotide, the overhang is selected from the group consisting of u and dT; or when the overhang has 2 nucleotides, the overhang is selected from the group consisting of uu and dTdT.

7. The double-stranded ribonucleic acid, the pharmaceutically acceptable salt thereof, or the ligand conjugate thereof according to any one of claims 4 to 6, wherein the additional sequence as the overhang is linked to an adjacent nucleotide via a phosphate group or a phosphorothioate group; or, one or more nucleotides in the additional sequence as the overhang are linked via a phosphate group or a phosphorothioate group.

8. The double-stranded ribonucleic acid, the pharmaceutically acceptable salt thereof, or the ligand conjugate thereof according to any one of claims 1 to 7, wherein 1, 2, 3, 4, 5, 6, 7, 8, 9, 10, 11, 12, 13, 14, 15, 16, 17, 18, 19, 20, or 21 nucleotides of the sense strand are modified, or a number of nucleotides within a range formed by any of the aforementioned values are modified; or, 1-21, 1-20, 1-19, 1-18, 1-17, 1-16, 1-15, 1-14, 1-13, 1-12, 1-11, or 1-10 nucleotides of the sense strand are modified; or 1 or more, 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, 19 or more, 20 or more, or 21 or more nucleotides of the sense strand are modified; or, all nucleotides of the sense strand are modified; and/or
1, 2, 3, 4, 5, 6, 7, 8, 9, 10, 11, 12, 13, 14, 15, 16, 17, 18, 19, 20, 21, 22, or 23 nucleotides of the antisense strand are modified, or a number of nucleotides within a range formed by any of the aforementioned values are modified; or, 1-23, 1-22, 1-21, 1-20, 1-19, 1-18, 1-17, 1-16, 1-15, 1-14, 1-13, 1-12, 1-11, or 1-10 nucleotides of the antisense strand are modified; or 1 or more, 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, 19 or more, 20 or more, 21 or more, 22 or more, or 23 or more nucleotides of the antisense strand are modified; or, all nucleotides of the antisense strand are modified.

9. The double-stranded ribonucleic acid, the pharmaceutically acceptable salt thereof, or the ligand conjugate thereof according to any one of claims 1 to 8, wherein the modification is selected from the group consisting of glycosyl modification of nucleotides, modification of bases, modification of linkages between nucleotides, and terminal modification.

10. The double-stranded ribonucleic acid, the pharmaceutically acceptable salt thereof, or the ligand conjugate thereof according to claim 9, wherein the glycosyl modification of nucleotides is selected from the group consisting of 2'-dehydroxylation, 2'-fluoro, 2'-amino, 2'-alkyl, 2'-O-alkyl, 2'-O-ether, and 2'-O-alkenyl; or, the glycosyl modification of nucleotides is selected from the group consisting of 2'-dehydroxylation, 2'-fluoro, 2'-amino, 2'-methyl, 2'-ethyl, 2'-methyl-O-methyl, 2'-ethyl-O-methyl, 2'-O-methyl, 2'-O-ethyl, 2'-O-ethyl-O-methyl, and 2'-O-allyl;

    the modification of linkages between nucleotides is selected from the group consisting of phosphorothioate (PS), phosphorodithioate (PS2), methylphosphonate (MP), methoxypropylphosphonate (MOP), and aminophosphonate; or, the modification of linkages between nucleotides is selected from phosphorothioate (PS); and/or
    the terminal modification is selected from the group consisting of a 5'-end modification and a 3'-end modification; or, the terminal modification is selected from the group consisting of 5'-phosphate, 5'-methylphosphonate (5'-MP), 5'-phosphorothioate (5'-PS), and 5'-(E)-vinylphosphonate (5'-(E)-VP).

11. The double-stranded ribonucleic acid, the pharmaceutically acceptable salt thereof, or the ligand conjugate thereof according to any one of claims 1 to 10, wherein a phosphorothioate modification is present between the nucleotides at positions 1 and 2 of the sense strand and/or the nucleotides at positions 2 and 3 of the sense strand, and a phosphorothioate modification is present between the nucleotides at positions 1 and 2 of the antisense strand, the nucleotides at positions 2 and 3 of the antisense strand, the nucleotides at positions 19 and 20 of the antisense strand, and/or the nucleotides at positions 20 and 21 of the antisense strand.

12. The double-stranded ribonucleic acid, the pharmaceutically acceptable salt thereof, or the ligand conjugate thereof according to any one of claims 1 to 11, wherein 2, 3, 4, or 5 nucleotides of the sense strand are modified by 2'-fluoro, and 14, 15, 16, or 17 nucleotides of the sense strand are modified by 2'-O-methyl; and/or
    2, 3, 4, 5, 6, or 7 nucleotides of the antisense strand are modified by 2'-fluoro, and 14, 15, 16, 17, 18, 19, 20, or 21 nucleotides of the antisense strand are modified by 2'-O-methyl.

13. The double-stranded ribonucleic acid, the pharmaceutically acceptable salt thereof, or the ligand conjugate thereof according to any one of claims 1 to 12, wherein 0, 1, or 2 nucleotides of the nucleotides at positions 1 to 6 of the sense strand are modified by 2'-fluoro, 4, 5, or 6 nucleotides of the nucleotides at positions 1 to 6 of the sense strand are modified by 2'-O-methyl, 2 or 3 nucleotides of the nucleotides at positions 7 to 9 of the sense strand are modified by 2'-fluoro, 0 or 1 nucleotide of the nucleotides at positions 7 to 9 of the sense strand is modified by 2'-O-methyl, 0 or 1 nucleotide of the nucleotides at positions 10 to 19 of the sense strand is modified by 2'-fluoro, and 8, 9, or 10 nucleotides of the nucleotides at positions 10 to 19 of the sense strand are modified by 2'-O-methyl; or the nucleotides at positions 7, 8, and 9 of the sense strand are modified by 2'-fluoro, and 0, 1, or 2 nucleotides of the nucleotides at positions 1 to 6 and/or 10 to 19 of the sense strand are also modified by 2'-fluoro; or the nucleotides at positions 7, 8, and 9 of the sense strand are modified by 2'-fluoro, and 0, 1, or 2 nucleotides of the nucleotides at positions 1 to 6 and/or 10 to 19 of the sense strand are also modified by 2'-fluoro.

14. The double-stranded ribonucleic acid, the pharmaceutically acceptable salt thereof, or the ligand conjugate thereof according to any one of claims 1 to 13, wherein 3 or 4 nucleotides of the nucleotides at positions 2, 6, 14, and 16 of the antisense strand are modified by 2'-fluoro; or 3, 4, 5, or 6 nucleotides of the nucleotides at positions 2, 6, 8, 9, 14, and 16 of the antisense strand are modified by 2'-fluoro; or the nucleotides at positions 2, 6, 14, and 16 of the antisense strand are modified by 2'-fluoro, and any one or two of the nucleotides at positions 1, 3 to 5, 7 to 13, 15, 17 to 21, and/or 17 to 23 of the antisense strand are also modified by 2'-fluoro; or 14, 15, 16, 17, 18, or 19 nucleotides of the nucleotides at positions 1, 3 to 5, 7 to 13, 15, 17 to 21, and/or 17 to 23 of the antisense strand are modified by 2'-O-methyl.

15. The double-stranded ribonucleic acid, the pharmaceutically acceptable salt thereof, or the ligand conjugate thereof according to any one of claims 1 to 14, wherein the ligand comprises at least one targeting group; or the ligand comprises one, two, three, four, or five targeting groups.

16. The double-stranded ribonucleic acid, the pharmaceutically acceptable salt thereof, or the ligand conjugate thereof according to claim 15, wherein the targeting group is a GalNAc group.

17. The double-stranded ribonucleic acid, the pharmaceutically acceptable salt thereof, or the ligand conjugate thereof according to any one of claims 1 to 16, wherein the ligand comprises the following branched group:

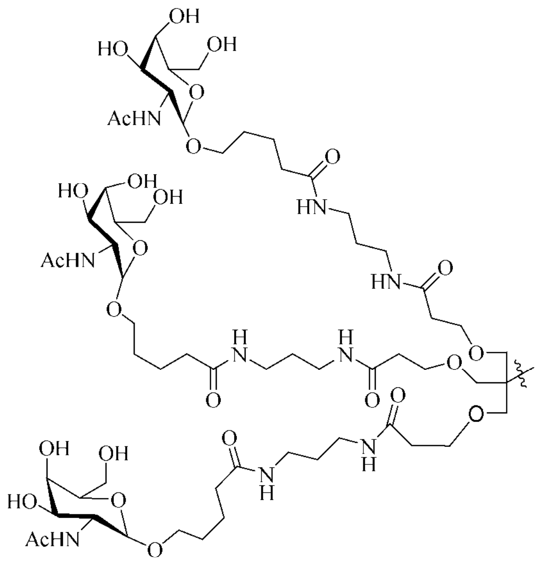

or

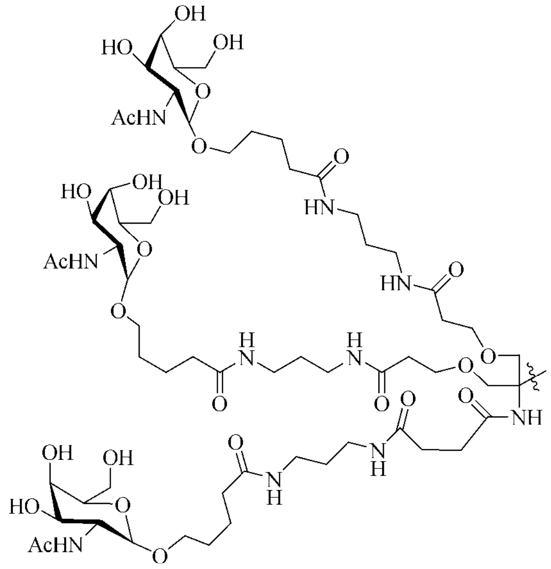

.

**18.** The double-stranded ribonucleic acid, the pharmaceutically acceptable salt thereof, or the ligand conjugate thereof according to any one of claims 1 to 17, wherein the ligand is selected from the group consisting of:

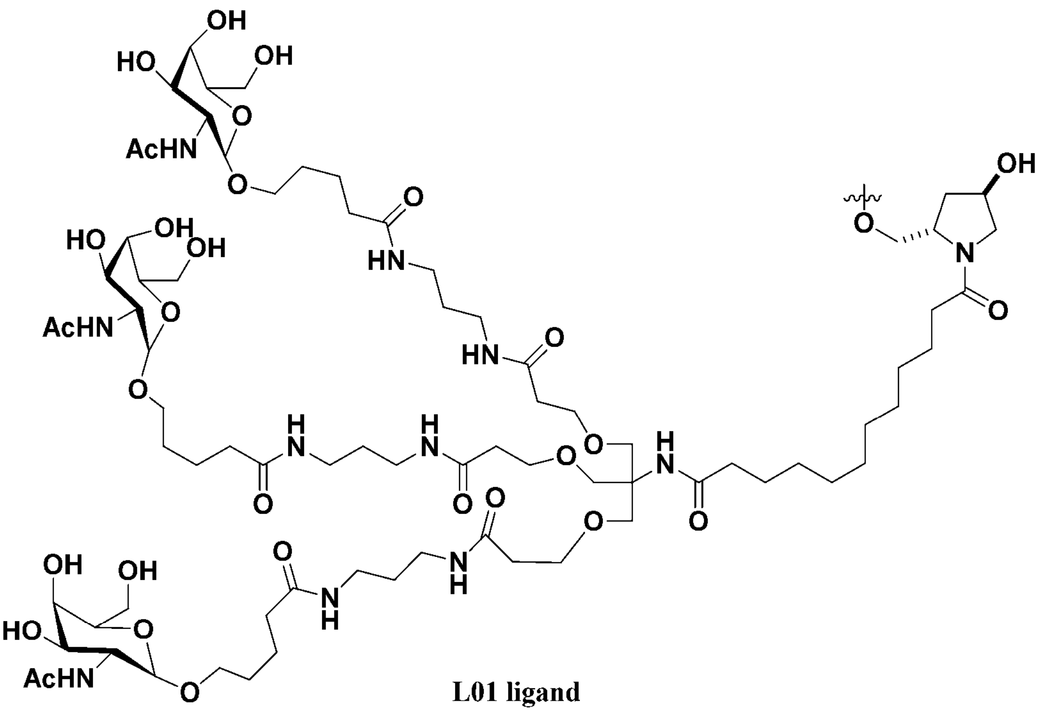

**L01 ligand**

and

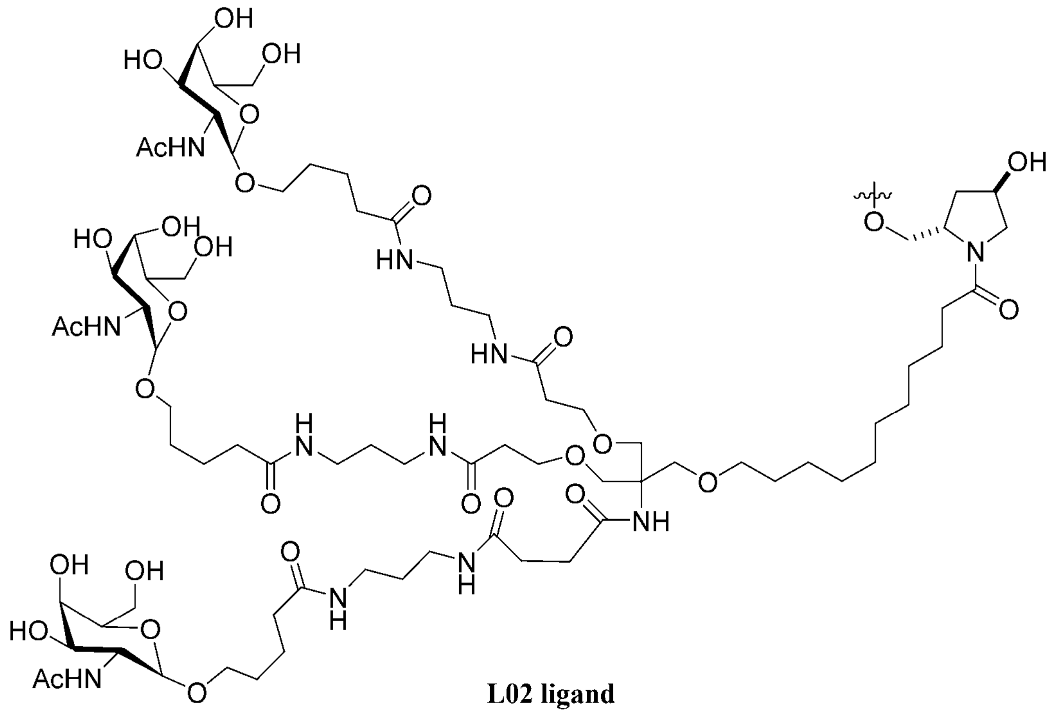

**L02 ligand**

.

**19.** The double-stranded ribonucleic acid, the pharmaceutically acceptable salt thereof, or the ligand conjugate thereof according to any one of claims 1 to 18, wherein the ligand is linked to the 5' or 3' end of the sense strand or the antisense strand; or, the ligand is linked to the 5' or 3' end of the sense strand; or, the ligand is linked to the 3' end of the sense strand.

**20.** The double-stranded ribonucleic acid, the pharmaceutically acceptable salt thereof, or the ligand conjugate thereof according to any one of claims 1 to 19, wherein the double-stranded ribonucleic acid comprises any one of the following sense strands: SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 55, SEQ ID NO: 57, SEQ ID NO: 59, SEQ ID NO: 61, SEQ ID NO: 63, SEQ ID NO: 65, SEQ ID NO: 67, SEQ ID NO: 69, SEQ ID NO: 71, SEQ ID NO: 73, SEQ ID NO: 75, SEQ ID NO: 77, SEQ ID NO: 79, SEQ ID NO: 81, and SEQ ID NO: 83; and
the double-stranded ribonucleic acid comprises any one of the following antisense strands: SEQ ID NO: 2, SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, SEQ ID NO: 48, SEQ ID NO: 50, SEQ ID NO: 52, SEQ ID NO: 54, SEQ ID NO: 56, SEQ ID NO: 58, SEQ ID NO: 60, SEQ ID NO: 62, SEQ ID NO: 64, SEQ ID NO: 66, SEQ ID NO: 68, SEQ ID NO: 70, SEQ ID NO: 72, SEQ ID NO: 74, SEQ ID NO: 76, SEQ ID NO: 78, SEQ ID NO: 80, SEQ ID NO: 82, and SEQ ID NO: 84.

**21.** The double-stranded ribonucleic acid, the pharmaceutically acceptable salt thereof, or the ligand conjugate thereof according to any one of claims 1 to 20, wherein the double-stranded ribonucleic acid comprises a sequence pair of the sense strand and the antisense strand as follows: SEQ ID NO: 1/SEQ ID NO: 2, SEQ ID NO: 3/SEQ ID NO: 4, SEQ ID NO: 5/SEQ ID NO: 6, SEQ ID NO: 7/SEQ ID NO: 8, SEQ ID NO: 9/SEQ ID NO: 10, SEQ ID NO: 11/SEQ ID NO: 12, SEQ ID NO: 13/SEQ ID NO: 14, SEQ ID NO: 15/SEQ ID NO: 16, SEQ ID NO: 17/SEQ ID NO: 18, SEQ ID NO: 19/SEQ ID NO: 20, SEQ ID NO: 21/SEQ ID NO: 22, SEQ ID NO: 23/SEQ ID NO: 24, SEQ ID NO: 25/SEQ ID NO: 26, SEQ ID NO: 27/SEQ ID NO: 28, SEQ ID NO: 29/SEQ ID NO: 30, SEQ ID NO: 31/SEQ ID NO: 32, SEQ ID NO: 33/SEQ ID NO: 34, SEQ ID NO: 35/SEQ ID NO: 36, SEQ ID NO: 37/SEQ ID NO: 38, SEQ ID NO: 39/SEQ ID NO: 40, SEQ ID NO: 41/SEQ ID NO: 42, SEQ ID NO: 43/SEQ ID NO: 44, SEQ ID NO: 45/SEQ ID NO: 46, SEQ ID NO: 47/SEQ ID NO: 48, SEQ ID NO: 49/SEQ ID NO: 50, SEQ ID NO: 51/SEQ ID NO: 52, SEQ ID NO: 53/SEQ ID NO: 54, SEQ ID NO: 55/SEQ ID NO: 56, SEQ ID NO: 57/SEQ ID NO: 58, SEQ ID NO: 59/SEQ ID NO: 60, SEQ ID NO: 61/SEQ ID NO: 62, SEQ ID NO:

63/SEQ ID NO: 64, SEQ ID NO: 65/SEQ ID NO: 66, SEQ ID NO: 67/SEQ ID NO: 68, SEQ ID NO: 69/SEQ ID NO: 70, SEQ ID NO: 71/SEQ ID NO: 72, SEQ ID NO: 73/SEQ ID NO: 74, SEQ ID NO: 75/SEQ ID NO: 76, SEQ ID NO: 77/SEQ ID NO: 78, SEQ ID NO: 79/SEQ ID NO: 80, SEQ ID NO: 81/SEQ ID NO: 82, or SEQ ID NO: 83/SEQ ID NO: 84.

**22.** The double-stranded ribonucleic acid, the pharmaceutically acceptable salt thereof, or the ligand conjugate thereof according to any one of claims 1 to 21, wherein:

the sense strand comprises 5'-gsasucUfcCfAfAfcuaaaauacuL-3' (SEQ ID NO: 29), and the antisense strand comprises 5'-asGfsuauUfuUfAfguugGfaGfaucscsg-3' (SEQ ID NO: 30);
the sense strand comprises 5'-gsasucucCfAfAfcuaaaauacuL-3' (SEQ ID NO: 31), and the antisense strand comprises 5'-asGfsuauUfuuaguugGfaGfaucscsg-3' (SEQ ID NO: 32);
the sense strand comprises 5'-gsgsauGfaUfUfUfucuuauaucaL-3' (SEQ ID NO: 33), and the antisense strand comprises 5'-usGfsauaUfaAfGfaaaaUfcAfuccsusg-3' (SEQ ID NO: 34);
the sense strand comprises 5'-gsgsaugaUfUfUfucuuauaucaL-3' (SEQ ID NO: 35), and the antisense strand comprises 5'-usGfsauaUfaagaaaaUfcAfuccsusg-3' (SEQ ID NO: 36);
the sense strand comprises 5'-gsgsucUfuUfUfCfaggaugauuuL-3' (SEQ ID NO: 37), and the antisense strand comprises 5'-asAfsaucAfuCfCfugaaAfaGfaccsusu-3' (SEQ ID NO: 38);
the sense strand comprises 5'-gsgsucuuUfUfCfaggaugauuuL-3' (SEQ ID NO: 39), and the antisense strand comprises 5'-asAfsaucAfuccugaaAfaGfaccsusu-3' (SEQ ID NO: 40);
the sense strand comprises 5'-gsuscuUfuUfCfAfggaugauuuuL-3' (SEQ ID NO: 41), and the antisense strand comprises 5'-asAfsaauCfaUfCfcugaAfaAfgacscsu-3' (SEQ ID NO: 42);
the sense strand comprises 5'-gsuscuuuUfCfAfggaugauuuuL-3' (SEQ ID NO: 43), and the antisense strand comprises 5'-asAfsaauCfauccugaAfaAfgacscsu-3' (SEQ ID NO: 44);
the sense strand comprises 5'-csusuuUfcAfGfGfaugauuuucuL-3' (SEQ ID NO: 45), and the antisense strand comprises 5'-asGfsaaaAfuCfAfuccuGfaAfaagsasc-3' (SEQ ID NO: 46);
the sense strand comprises 5'-csusuuucAfGfGfaugauuuucuL-3' (SEQ ID NO: 47), and the antisense strand comprises 5'-asGfsaaaAfucauccuGfaAfaagsasc-3' (SEQ ID NO: 48);
the sense strand comprises 5'-ususuuCfaGfGfAfugauuuucuuL-3' (SEQ ID NO: 49), and the antisense strand comprises 5'-asAfsgaaAfaUfCfauccUfgAfaaasgsa-3' (SEQ ID NO: 50);
the sense strand comprises 5'-ususuucaGfGfAfugauuuucuuL-3' (SEQ ID NO: 51), and the antisense strand comprises 5'-asAfsgaaAfaucauccUfgAfaaasgsa-3' (SEQ ID NO: 52);
the sense strand comprises 5'-uscsagGfaUfGfAfuuuucuuauaL-3' (SEQ ID NO: 53), and the antisense strand comprises 5'-usAfsuaaGfaAfAfaucaUfcCfugasasa-3' (SEQ ID NO: 54);
the sense strand comprises 5'-uscsaggaUfGfAfuuuucuuauaL-3' (SEQ ID NO: 55), and the antisense strand comprises 5'-usAfsuaaGfaaaaucaUfcCfugasasa-3' (SEQ ID NO: 56);
the sense strand comprises 5'-esasggAfuGfAfUfuuucuuauauL-3' (SEQ ID NO: 57), and the antisense strand comprises 5'-asUfsauaAfgAfAfaaucAfuCfcugsasa-3' (SEQ ID NO: 58);
the sense strand comprises 5'-csasggauGfAfUfuuucuuauauL-3' (SEQ ID NO: 59), and the antisense strand comprises 5'-asUfsauaAfgaaaaucAfuCfcugsasa-3' (SEQ ID NO: 60);
the sense strand comprises 5'-asgsgaUfgAfUfUfuucuuauaucL-3' (SEQ ID NO: 61), and the antisense strand comprises 5'-gsAfsuauAfaGfAfaaauCfaUfccusgsa-3' (SEQ ID NO: 62);
the sense strand comprises 5'-asgsgaugAfUfUfuucuuauaucL-3' (SEQ ID NO: 63), and the antisense strand comprises 5'-gsAfsuauAfagaaaauCfaUfccusgsa-3' (SEQ ID NO: 64);
the sense strand comprises 5'-gsasugAfuUfUfUfcuuauaucaaL-3' (SEQ ID NO: 65), and the antisense strand comprises 5'-usUfsgauAfuAfAfgaaaAfuCfaucscsu-3' (SEQ ID NO: 66);
the sense strand comprises 5'-gsasugauUfUfUfcuuauaucaaL-3' (SEQ ID NO: 67), and the antisense strand comprises 5'-usUfsgauAfuaagaaaAfuCfaucscsu-3' (SEQ ID NO: 68);
the sense strand comprises 5'-asusgaUfuUfUfCfuuauaucaagL-3' (SEQ ID NO: 69), and the antisense strand comprises 5'-csUfsugaUfaUfAfagaaAfaUfcauscsc-3' (SEQ ID NO: 70);
the sense strand comprises 5'-asusgauuUfUfCfuuauaucaagL-3' (SEQ ID NO: 71), and the antisense strand comprises 5'-csUfsugaUfauaagaaAfaUfcauscsc-3' (SEQ ID NO: 72);
the sense strand comprises 5'-usgsauUfuUfCfUfuauaucaaguL-3' (SEQ ID NO: 73), and the antisense strand comprises 5'-asCfsuugAfuAfUfaagaAfaAfucasusc-3' (SEQ ID NO: 74);
the sense strand comprises 5'-usgsauuuUfCfUfuauaucaaguL-3' (SEQ ID NO: 75), and the antisense strand comprises 5'-asCfsuugAfuauaagaAfaAfucasusc-3' (SEQ ID NO: 76);
the sense strand comprises 5'-csusuaUfaUfCfAfagugguacauL-3' (SEQ ID NO: 77), and the antisense strand comprises 5'-asUfsguaCfcAfCfuugaUfaUfaagsasa-3' (SEQ ID NO: 78);
the sense strand comprises 5'-csusuauaUfCfAfagugguacauL-3' (SEQ ID NO: 79), and the antisense strand

comprises 5'-asUfsguaCfcacuugaUfaUfaagsasa-3' (SEQ ID NO: 80);
the sense strand comprises 5'-asasacGfgAfUfCfuccaacuaaaL-3' (SEQ ID NO: 81), and the antisense strand comprises 5'-usUfsuagUfuGfGfagauCfcGfuuusgsa-3' (SEQ ID NO: 82);
the sense strand comprises 5'-asasacggAfUfCfuccaacuaaaL-3' (SEQ ID NO: 83), and the antisense strand comprises 5'-usUfsuagUfuggagauCfcGfuuusgsa-3' (SEQ ID NO: 84);
L is L01 or L02.

**23.** A pharmaceutical composition, comprising the double-stranded ribonucleic acid, the pharmaceutically acceptable salt thereof, or the ligand conjugate thereof according to any one of claims 1 to 22, and optionally a pharmaceutically acceptable carrier or excipient.

**24.** Use of the double-stranded ribonucleic acid, the pharmaceutically acceptable salt thereof, or the ligand conjugate thereof according to any one of claims 1-22 or the pharmaceutical composition according to claim 23 for preparing a medicament for treating and/or preventing thromboembolism and complications thereof.

**25.** The use according to claim 24, wherein the double-stranded ribonucleic acid, the pharmaceutically acceptable salt thereof, or the ligand conjugate thereof, or the pharmaceutical composition is used in combination with an additional therapeutic agent.

**INTERNATIONAL SEARCH REPORT**

International application No.
**PCT/CN2024/115820**

**A. CLASSIFICATION OF SUBJECT MATTER**

C12N15/113(2010.01)i; A61K31/713(2006.01)i; A61P7/00(2006.01)i; A61K48/00(2006.01)i

According to International Patent Classification (IPC) or to both national classification and IPC

**B. FIELDS SEARCHED**

Minimum documentation searched (classification system followed by classification symbols)

IPC:C12N; A61K; A61P

Documentation searched other than minimum documentation to the extent that such documents are included in the fields searched

Electronic data base consulted during the international search (name of data base and, where practicable, search terms used)

CNTXT, ENTXTC, CJFD, ENTXT, WFB, WFCCP, WFCD, WFCJ, WFCOA, WFCSTA, WFFCP, WFFD, WFFJ, WFFOA, WFS, WFSTR, PubMed, GenBank, ISI Web of Knowledge, Baidu, CNKI, STN: 正大天晴, 凝血因子, 丝氨酸蛋白酶原, 凝血因子XI, factor XI, FXI, dsRNA, siRNA, RNAi, 双链, SEQ ID NOs: 1-28

**C. DOCUMENTS CONSIDERED TO BE RELEVANT**

| Category* | Citation of document, with indication, where appropriate, of the relevant passages | Relevant to claim No. |
|---|---|---|
| A | US 2022364087 A1 (SIRNAOMICS, INC.) 17 November 2022 (2022-11-17)<br>entire document | 1-25 |
| A | WO 2022028457 A1 (TUOJIE BIOTECH (SHANGHAI) CO., LTD.) 10 February 2022 (2022-02-10)<br>claims 1 and 2, and description, paragraphs 2 and 19 | 1-25 |
| A | WO 2023109945 A1 (TUOJIE BIOTECH (SHANGHAI) CO., LTD.) 22 June 2023 (2023-06-22)<br>entire document | 1-25 |
| A | CN 113227376 A (SUZHOU RIBO LIFE SCIENCE CO., LTD.) 06 August 2021 (2021-08-06)<br>entire document | 1-25 |
| A | CN 102245186 A (ISIS PHARMACEUTICALS, INC.) 16 November 2011 (2011-11-16)<br>entire document | 1-25 |
| A | CN 108064162 A (IONIS PHARMACEUTICALS, INC.) 22 May 2018 (2018-05-22)<br>entire document | 1-25 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **28 November 2024** | **04 December 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2024/115820**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/CN2024/115820**

| Patent document cited in search report | | | Publication date (day/month/year) | Patent family member(s) | | | Publication date (day/month/year) |
|---|---|---|---|---|---|---|---|
| US | 2022364087 | A1 | 17 November 2022 | CA | 3215347 | A1 | 20 October 2022 |
| | | | | JP | 2024514887 | A | 03 April 2024 |
| | | | | AU | 2022258464 | A1 | 26 October 2023 |
| | | | | BR | 112023021231 | A2 | 09 January 2024 |
| | | | | EP | 4323523 | A2 | 21 February 2024 |
| | | | | WO | 2022221441 | A2 | 20 October 2022 |
| | | | | WO | 2022221441 | A3 | 24 November 2022 |
| | | | | IL | 307655 | A | 01 December 2023 |
| WO | 2022028457 | A1 | 10 February 2022 | TW | 202229549 | A | 01 August 2022 |
| WO | 2023109945 | A1 | 22 June 2023 | WO | 2023109935 | A1 | 22 June 2023 |
| | | | | TW | 202333751 | A | 01 September 2023 |
| | | | | TW | 202340466 | A | 16 October 2023 |
| CN | 113227376 | A | 06 August 2021 | EP | 3974532 | A1 | 30 March 2022 |
| | | | | EP | 3974532 | A4 | 24 January 2024 |
| | | | | AU | 2020280438 | A1 | 16 December 2021 |
| | | | | JP | 2022533722 | A | 25 July 2022 |
| | | | | TW | 202111120 | A | 16 March 2021 |
| | | | | KR | 20220011689 | A | 28 January 2022 |
| | | | | US | 2023193277 | A1 | 22 June 2023 |
| | | | | CA | 3139195 | A1 | 26 November 2020 |
| | | | | WO | 2020233650 | A1 | 26 November 2020 |
| CN | 102245186 | A | 16 November 2011 | SI | 2379084 | T1 | 30 April 2018 |
| | | | | JP | 2018199700 | A | 20 December 2018 |
| | | | | JP | 6633155 | B2 | 22 January 2020 |
| | | | | US | 2013274308 | A1 | 17 October 2013 |
| | | | | US | 8735370 | B2 | 27 May 2014 |
| | | | | US | 2017035798 | A1 | 09 February 2017 |
| | | | | US | 2021169921 | A1 | 10 June 2021 |
| | | | | US | 11376273 | B2 | 05 July 2022 |
| | | | | MX | 2011004097 | A | 28 July 2011 |
| | | | | EP | 3335715 | A2 | 20 June 2018 |
| | | | | EP | 3335715 | A3 | 08 August 2018 |
| | | | | AU | 2017202862 | A1 | 18 May 2017 |
| | | | | AU | 2017202862 | B2 | 20 September 2018 |
| | | | | NZ | 603603 | A | 27 June 2014 |
| | | | | NO | 2379084 | T3 | 21 April 2018 |
| | | | | BRPI | 0920263 | A2 | 12 July 2016 |
| | | | | BRPI | 0920263 | B1 | 21 September 2021 |
| | | | | JP | 2015211685 | A | 26 November 2015 |
| | | | | JP | 6084255 | B2 | 22 February 2017 |
| | | | | CY | 1119909 | T1 | 27 June 2018 |
| | | | | EP | 2379084 | A2 | 26 October 2011 |
| | | | | EP | 2379084 | A4 | 06 June 2012 |
| | | | | EP | 2379084 | B1 | 22 November 2017 |
| | | | | NZ | 592203 | A | 25 January 2013 |
| | | | | ES | 2657679 | T3 | 06 March 2018 |
| | | | | KR | 20110081294 | A | 13 July 2011 |
| | | | | KR | 101773551 | B1 | 31 August 2017 |
| | | | | PT | 2379084 | T | 19 February 2018 |
| | | | | US | 2020000839 | A1 | 02 January 2020 |

Form PCT/ISA/210 (patent family annex) (July 2022)

**INTERNATIONAL SEARCH REPORT**
**Information on patent family members**

International application No.
**PCT/CN2024/115820**

| Patent document cited in search report | Publication date (day/month/year) | Patent family member(s) | Publication date (day/month/year) |
|---|---|---|---|
| | | US 10772906 B2 | 15 September 2020 |
| | | AU 2009305636 A1 | 22 April 2010 |
| | | RU 2011119479 A | 27 November 2012 |
| | | RU 2535964 C2 | 20 December 2014 |
| | | CA 2966011 A1 | 22 April 2010 |
| | | CA 2966011 C | 19 October 2021 |
| | | US 2023263822 A1 | 24 August 2023 |
| | | JP 2012505660 A | 08 March 2012 |
| | | JP 5809058 B2 | 10 November 2015 |
| | | HUE 035888 T2 | 28 May 2018 |
| | | IL 244116 A0 | 21 April 2016 |
| | | IL 244116 B | 31 March 2019 |
| | | RU 2014134408 A | 20 March 2016 |
| | | RU 2739594 C2 | 28 December 2020 |
| | | JP 2017099395 A | 08 June 2017 |
| | | JP 6397517 B2 | 26 September 2018 |
| | | WO 2010045509 A2 | 22 April 2010 |
| | | WO 2010045509 A3 | 15 July 2010 |
| | | US 2013190384 A1 | 25 July 2013 |
| | | LT 2379084 T | 26 March 2018 |
| | | KR 20170101324 A | 05 September 2017 |
| | | KR 101979134 B1 | 15 May 2019 |
| | | HRP 20171969 T1 | 06 April 2018 |
| | | DK 2379084 T3 | 22 January 2018 |
| | | PL 2379084 T3 | 30 April 2018 |
| | | US 2010137414 A1 | 03 June 2010 |
| | | US 8334372 B2 | 18 December 2012 |
| | | CA 2740785 A1 | 22 April 2010 |
| | | CA 2740785 C | 20 June 2017 |
| | | IL 212267 A0 | 30 June 2011 |
| | | IL 212267 A | 29 February 2016 |
| CN 108064162 A | 22 May 2018 | MX 2020004209 A | 13 August 2020 |
| | | WO 2014179620 A1 | 06 November 2014 |
| | | JP 2020058370 A | 16 April 2020 |
| | | IL 261901 A | 31 October 2018 |
| | | IL 261901 B | 31 May 2020 |
| | | RS 60796 B1 | 30 October 2020 |
| | | EA 201891479 A1 | 30 November 2018 |
| | | EA 036584 B1 | 26 November 2020 |
| | | ES 2778442 T3 | 10 August 2020 |
| | | US 2016076032 A1 | 17 March 2016 |
| | | US 9714421 B2 | 25 July 2017 |
| | | RU 2019124314 A | 21 August 2019 |
| | | IL 263843 A | 31 January 2019 |
| | | IL 263843 B | 31 March 2020 |
| | | KR 20230006933 A | 11 January 2023 |
| | | KR 20190084138 A | 15 July 2019 |
| | | KR 102212275 B1 | 05 February 2021 |
| | | MX 2019010441 A | 17 October 2019 |
| | | JP 2016526874 A | 08 September 2016 |

Form PCT/ISA/210 (patent family annex) (July 2022)

**INTERNATIONAL SEARCH REPORT**
**Information on patent family members**

International application No.
**PCT/CN2024/115820**

| Patent document cited in search report | Publication date (day/month/year) | Patent family member(s) | Publication date (day/month/year) |
|---|---|---|---|
| | | JP 6387084 B2 | 05 September 2018 |
| | | HUE 050394 T2 | 30 November 2020 |
| | | US 2018273953 A1 | 27 September 2018 |
| | | US 10883104 B2 | 05 January 2021 |
| | | HRP 20201378 T1 | 27 November 2020 |
| | | US 2023151365 A1 | 18 May 2023 |
| | | IL 296543 A | 01 November 2022 |
| | | IL 296543 B1 | 01 October 2024 |
| | | EP 4438129 A2 | 02 October 2024 |
| | | IL 242124 B | 28 February 2019 |
| | | JP 2021020901 A | 18 February 2021 |
| | | JP 7177127 B2 | 22 November 2022 |
| | | JP 2016523515 A | 12 August 2016 |
| | | JP 6456362 B2 | 23 January 2019 |
| | | EP 2991656 A2 | 09 March 2016 |
| | | EP 2991656 A4 | 22 February 2017 |
| | | EP 2991656 B1 | 18 December 2019 |
| | | IL 315582 A | 01 November 2024 |
| | | AU 2017200365 A1 | 23 February 2017 |
| | | AU 2017200365 B2 | 08 November 2018 |
| | | AU 2017200365 C1 | 18 April 2019 |
| | | US 2016017323 A1 | 21 January 2016 |
| | | US 2015176007 A1 | 25 June 2015 |
| | | US 9145558 B2 | 29 September 2015 |
| | | EP 3633039 A1 | 08 April 2020 |
| | | PT 2992098 T | 05 July 2019 |
| | | US 2024247260 A1 | 25 July 2024 |
| | | IL 274064 A | 30 June 2020 |
| | | IL 274064 B | 30 June 2021 |
| | | AU 2014259759 A1 | 22 October 2015 |
| | | AU 2014259759 B2 | 18 June 2020 |
| | | US 2021395734 A1 | 23 December 2021 |
| | | US 2021130823 A1 | 06 May 2021 |
| | | IL 242132 B | 31 October 2018 |
| | | JP 2020039355 A | 19 March 2020 |
| | | AU 2022202770 A1 | 19 May 2022 |
| | | AU 2022202770 B2 | 03 October 2024 |
| | | CA 2921162 A1 | 06 November 2014 |
| | | AU 2014259757 A1 | 22 October 2015 |
| | | AU 2014259757 B2 | 02 March 2017 |
| | | HK 1221404 A1 | 02 June 2017 |
| | | US 2018273952 A1 | 27 September 2018 |
| | | US 10927372 B2 | 23 February 2021 |
| | | UA 120287 C2 | 11 November 2019 |
| | | IL 264241 A | 28 February 2019 |
| | | IL 264241 B | 30 April 2020 |
| | | LT 2992098 T | 10 July 2019 |
| | | JP 2023113843 A | 16 August 2023 |
| | | AU 2020207820 A1 | 06 August 2020 |
| | | MY 178929 A | 23 October 2020 |

Form PCT/ISA/210 (patent family annex) (July 2022)

**INTERNATIONAL SEARCH REPORT**
**Information on patent family members**

International application No.
**PCT/CN2024/115820**

| Patent document cited in search report | Publication date (day/month/year) | Patent family member(s) | Publication date (day/month/year) |
|---|---|---|---|
| | | KR 20210151260 A | 13 December 2021 |
| | | KR 102558571 B1 | 21 July 2023 |
| | | US 2016076030 A1 | 17 March 2016 |
| | | US 9932580 B2 | 03 April 2018 |
| | | AU 2019200820 A1 | 28 February 2019 |
| | | AU 2019200820 B2 | 30 April 2020 |
| | | AU 2014259755 A1 | 22 October 2015 |
| | | AU 2014259755 B2 | 30 August 2018 |
| | | CR 20190269 A | 13 September 2019 |
| | | ES 2819213 T3 | 15 April 2021 |
| | | US 2020224198 A1 | 16 July 2020 |
| | | PT 3524680 T | 04 January 2021 |
| | | HK 1221486 A1 | 02 June 2017 |
| | | MX 2015015263 A | 16 December 2016 |
| | | CL 2015003217 A1 | 08 July 2016 |
| | | AU 2018267625 A1 | 13 December 2018 |
| | | AU 2018267625 B2 | 10 September 2020 |
| | | EP 2992009 A1 | 09 March 2016 |
| | | EP 2992009 A4 | 28 December 2016 |
| | | EP 2992009 B1 | 24 June 2020 |
| | | SG 11201508800 WA | 27 November 2015 |
| | | AU 2019203674 A1 | 27 June 2019 |
| | | AU 2019203674 B2 | 25 March 2021 |
| | | JP 2020007361 A | 16 January 2020 |
| | | JP 7429103 B2 | 07 February 2024 |
| | | NZ 740338 A | 29 April 2022 |
| | | KR 20240147701 A | 08 October 2024 |
| | | MX 2020002184 A | 14 July 2020 |
| | | BR 112015027319 A2 | 26 September 2017 |
| | | BR 112015027319 A8 | 02 January 2018 |
| | | CY 1123369 T1 | 31 December 2021 |
| | | HUE 043697 T2 | 30 September 2019 |
| | | RU 2018136140 A | 17 December 2018 |
| | | RU 2018136140 A3 | 27 April 2022 |
| | | HK 1221475 A1 | 02 June 2017 |
| | | IL 264580 A | 28 February 2019 |
| | | IL 264580 B | 30 April 2020 |
| | | US 2021024923 A1 | 28 January 2021 |
| | | US 2022275365 A9 | 01 September 2022 |
| | | KR 20210014758 A | 09 February 2021 |
| | | EP 2991661 A1 | 09 March 2016 |
| | | EP 2991661 A4 | 15 February 2017 |
| | | EP 2991661 B1 | 13 March 2019 |
| | | PE 20152002 A1 | 21 January 2016 |
| | | EP 2992097 A2 | 09 March 2016 |
| | | EP 2992097 A4 | 04 January 2017 |
| | | EP 2992097 B1 | 06 November 2019 |
| | | DOP 2021000095 A | 15 September 2021 |
| | | IL 273184 A | 30 April 2020 |
| | | IL 273184 B | 29 July 2021 |

Form PCT/ISA/210 (patent family annex) (July 2022)

**INTERNATIONAL SEARCH REPORT**
**Information on patent family members**

International application No.
**PCT/CN2024/115820**

| Patent document cited in search report | Publication date (day/month/year) | Patent family member(s) | | | Publication date (day/month/year) |
|---|---|---|---|---|---|
| | | CL | 2016002262 | A1 | 09 June 2017 |
| | | RU | 2015151203 | A | 02 June 2017 |
| | | RU | 2650510 | C2 | 16 April 2018 |
| | | IL | 273205 | A | 30 April 2020 |
| | | BR | 112015027321 | A2 | 26 September 2017 |
| | | BR | 112015027321 | A8 | 02 January 2018 |
| | | NZ | 631512 | A | 28 October 2016 |
| | | WO | 2014179629 | A2 | 06 November 2014 |
| | | WO | 2014179629 | A3 | 22 January 2015 |
| | | WO | 2014179629 | A8 | 02 June 2016 |
| | | CY | 1121879 | T1 | 14 October 2020 |
| | | KR | 20160002976 | A | 08 January 2016 |
| | | KR | 102315836 | B1 | 22 October 2021 |
| | | KR | 20180051678 | A | 16 May 2018 |
| | | KR | 102138781 | B1 | 28 July 2020 |
| | | EP | 3828275 | A1 | 02 June 2021 |
| | | IL | 283660 | A | 29 July 2021 |
| | | SG | 11201508870 | VA | 27 November 2015 |
| | | KR | 20220108195 | A | 02 August 2022 |
| | | KR | 102651423 | B1 | 27 March 2024 |
| | | HK | 1221403 | A1 | 02 June 2017 |
| | | MX | 2015015234 | A | 03 October 2016 |
| | | IL | 242125 | B | 28 February 2019 |
| | | RU | 2015151199 | A | 05 June 2017 |
| | | RU | 2015151199 | A3 | 27 March 2018 |
| | | RU | 2697152 | C2 | 12 August 2019 |
| | | ES | 2730015 | T3 | 07 November 2019 |
| | | ME | 03390 | B | 20 January 2020 |
| | | PE | 20161430 | A1 | 06 January 2017 |
| | | PH | 12015502493 | A1 | 22 February 2016 |
| | | AU | 2017203436 | A1 | 08 June 2017 |
| | | AU | 2017203436 | B2 | 18 October 2018 |
| | | DK | 3524680 | T3 | 14 December 2020 |
| | | EP | 2992098 | A2 | 09 March 2016 |
| | | EP | 2992098 | A4 | 11 January 2017 |
| | | EP | 2992098 | B1 | 27 March 2019 |
| | | NZ | 753018 | A | 28 January 2022 |
| | | BR | 112015027369 | A2 | 26 September 2017 |
| | | BR | 112015027369 | A8 | 02 January 2018 |
| | | BR | 112015027369 | B1 | 08 June 2021 |
| | | JP | 2019056001 | A | 11 April 2019 |
| | | JP | 6639629 | B2 | 05 February 2020 |
| | | SG | 10201801507 | RA | 28 March 2018 |
| | | JP | 2018027091 | A | 22 February 2018 |
| | | JP | 6592486 | B2 | 16 October 2019 |
| | | WO | 2014179627 | A2 | 06 November 2014 |
| | | WO | 2014179627 | A9 | 26 February 2015 |
| | | WO | 2014179627 | A3 | 16 April 2015 |
| | | JP | 2021107408 | A | 29 July 2021 |
| | | JP | 7339294 | B2 | 05 September 2023 |

Form PCT/ISA/210 (patent family annex) (July 2022)

**INTERNATIONAL SEARCH REPORT**
**Information on patent family members**

International application No.
**PCT/CN2024/115820**

| Patent document cited in search report | Publication date (day/month/year) | Patent family member(s) | | | Publication date (day/month/year) |
|---|---|---|---|---|---|
| | | JP | 2016522817 | A | 04 August 2016 |
| | | JP | 6216444 | B2 | 18 October 2017 |
| | | NZ | 725538 | A | 26 February 2021 |
| | | DK | 2992098 | T3 | 17 June 2019 |
| | | PL | 2992098 | T3 | 30 September 2019 |
| | | DOP | 2016000287 | A | 15 February 2017 |
| | | JP | 2020074787 | A | 21 May 2020 |
| | | US | 2018044676 | A1 | 15 February 2018 |
| | | US | 10683499 | B2 | 16 June 2020 |
| | | US | 2016090596 | A1 | 31 March 2016 |
| | | US | 9957504 | B2 | 01 May 2018 |
| | | CA | 2921514 | A1 | 06 November 2014 |
| | | CA | 2921514 | C | 24 October 2023 |
| | | JP | 2016522683 | A | 04 August 2016 |
| | | JP | 6995478 | B2 | 14 January 2022 |
| | | WO | 2014179626 | A2 | 06 November 2014 |
| | | WO | 2014179626 | A3 | 26 February 2015 |
| | | RU | 2018112167 | A | 07 March 2019 |
| | | AU | 2021204244 | A1 | 22 July 2021 |
| | | AU | 2021204244 | B2 | 19 October 2023 |
| | | JP | 2018183184 | A | 22 November 2018 |
| | | JP | 6652602 | B2 | 26 February 2020 |
| | | SI | 2992009 | T1 | 30 October 2020 |
| | | IL | 273312 | A | 30 April 2020 |
| | | ZA | 201507218 | B | 27 September 2023 |
| | | US | 2015126719 | A1 | 07 May 2015 |
| | | US | 9163239 | B2 | 20 October 2015 |
| | | SG | 10201906382 | QA | 27 August 2019 |
| | | NZ | 631552 | A | 24 February 2017 |
| | | NZ | 728517 | A | 24 December 2021 |
| | | WO | 2014179625 | A1 | 06 November 2014 |
| | | AU | 2017200950 | A1 | 02 March 2017 |
| | | AU | 2017200950 | B2 | 17 January 2019 |
| | | EP | 3524680 | A1 | 14 August 2019 |
| | | EP | 3524680 | B1 | 11 November 2020 |
| | | ZA | 201507216 | B | 30 August 2017 |
| | | US | 2014343123 | A1 | 20 November 2014 |
| | | US | 9127276 | B2 | 08 September 2015 |
| | | CA | 2921518 | A1 | 06 November 2014 |
| | | JP | 2023012548 | A | 25 January 2023 |
| | | KR | 20240042220 | A | 01 April 2024 |
| | | PH | 12019501191 | A1 | 01 March 2021 |
| | | US | 2016090595 | A1 | 31 March 2016 |
| | | US | 9932581 | B2 | 03 April 2018 |
| | | MY | 198359 | A | 28 August 2023 |
| | | JP | 2024116224 | A | 27 August 2024 |
| | | NZ | 712737 | A | 27 August 2021 |
| | | MX | 2021008901 | A | 19 August 2021 |
| | | US | 2018002693 | A1 | 04 January 2018 |
| | | BR | 112015027377 | A2 | 29 August 2017 |

Form PCT/ISA/210 (patent family annex) (July 2022)

**INTERNATIONAL SEARCH REPORT**
**Information on patent family members**

International application No.
**PCT/CN2024/115820**

| Patent document cited in search report | Publication date (day/month/year) | Patent family member(s) | Publication date (day/month/year) |
|---|---|---|---|
| | | BR 112015027377 A8 | 03 October 2017 |
| | | BR 112015027377 B1 | 10 January 2023 |
| | | JP 2020039354 A | 19 March 2020 |
| | | JP 6866459 B2 | 28 April 2021 |
| | | US 2015126720 A1 | 07 May 2015 |
| | | US 9181550 B2 | 10 November 2015 |
| | | UA 121017 C2 | 25 March 2020 |
| | | AU 2014259756 A1 | 22 October 2015 |
| | | AU 2014259756 B2 | 23 February 2017 |
| | | IL 272617 A | 31 March 2020 |
| | | ES 2885174 T3 | 13 December 2021 |
| | | MX 2021008899 A | 19 August 2021 |
| | | EP 3690049 A1 | 05 August 2020 |
| | | KR 20210037752 A | 06 April 2021 |
| | | US 2019367914 A1 | 05 December 2019 |
| | | US 10844379 B2 | 24 November 2020 |
| | | MX 2015015264 A | 12 August 2016 |
| | | JP 2022017514 A | 25 January 2022 |
| | | MX 2015015220 A | 12 January 2016 |
| | | DK 2992009 T3 | 14 September 2020 |
| | | JP 2021074021 A | 20 May 2021 |
| | | CA 2921167 A1 | 06 November 2014 |
| | | US 2024352455 A1 | 24 October 2024 |
| | | AU 2020233603 A1 | 01 October 2020 |
| | | AU 2024200296 A1 | 08 February 2024 |
| | | EA 201592093 A1 | 30 June 2016 |
| | | EA 031393 B1 | 28 December 2018 |
| | | PL 2992009 T3 | 30 November 2020 |
| | | MX 2015015239 A | 03 October 2016 |
| | | AU 2020217347 A1 | 27 August 2020 |
| | | AU 2019204784 A1 | 25 July 2019 |
| | | AU 2019204784 B2 | 27 January 2022 |
| | | AU 2019204784 C1 | 03 November 2022 |
| | | IL 284593 A | 31 August 2021 |
| | | IL 284593 B | 01 October 2022 |
| | | IL 284593 B2 | 01 February 2023 |
| | | JP 2024010070 A | 23 January 2024 |
| | | KR 20160002977 A | 08 January 2016 |
| | | HK 1221485 A1 | 02 June 2017 |
| | | AU 2014259750 A1 | 22 October 2015 |
| | | AU 2014259750 B2 | 28 February 2019 |
| | | IL 242126 B | 31 January 2019 |
| | | KR 20200090966 A | 29 July 2020 |
| | | KR 20230113835 A | 01 August 2023 |
| | | KR 102712053 B1 | 02 October 2024 |
| | | RU 2015151204 A | 02 June 2017 |
| | | RU 2015151204 A3 | 27 March 2018 |
| | | RU 2686080 C2 | 24 April 2019 |
| | | AU 2019202598 A1 | 02 May 2019 |
| | | SI 2992098 T1 | 28 June 2019 |

Form PCT/ISA/210 (patent family annex) (July 2022)

**INTERNATIONAL SEARCH REPORT**
**Information on patent family members**

International application No.
**PCT/CN2024/115820**

| Patent document cited in search report | Publication date (day/month/year) | Patent family member(s) | | | Publication date (day/month/year) |
|---|---|---|---|---|---|
| | | RU | 2015151202 | A | 06 June 2017 |
| | | RU | 2015151202 | A3 | 27 March 2018 |
| | | RU | 2670614 | C2 | 24 October 2018 |
| | | RU | 2670614 | C9 | 23 November 2018 |
| | | PT | 2992009 | T | 21 September 2020 |
| | | PH | 12018501963 | A1 | 20 July 2020 |
| | | DOP | 2015000268 | A | 30 November 2015 |
| | | BR | 112015027322 | A2 | 26 September 2017 |
| | | BR | 112015027322 | A8 | 02 January 2018 |
| | | EP | 4155403 | A1 | 29 March 2023 |
| | | US | 2015126718 | A1 | 07 May 2015 |
| | | US | 9181549 | B2 | 10 November 2015 |
| | | KR | 20160003723 | A | 11 January 2016 |
| | | KR | 102424855 | B1 | 26 July 2022 |
| | | NZ | 631537 | A | 26 May 2017 |
| | | RU | 2019110030 | A | 06 May 2019 |
| | | CR | 20150612 | A | 03 March 2016 |
| | | US | 11299736 | B1 | 12 April 2022 |
| | | LT | 2992009 | T | 10 November 2020 |
| | | RS | 58981 | B1 | 30 August 2019 |
| | | US | 2019055554 | A1 | 21 February 2019 |
| | | CA | 2921509 | A1 | 06 November 2014 |
| | | IL | 284000 | A | 29 July 2021 |
| | | KR | 20160002975 | A | 08 January 2016 |
| | | KR | 101857707 | B1 | 14 May 2018 |
| | | RU | 2015151200 | A3 | 14 January 2019 |
| | | RU | 2015151200 | A | 11 September 2019 |
| | | US | 2021087566 | A1 | 25 March 2021 |
| | | US | 11851655 | B2 | 26 December 2023 |
| | | JP | 2016526018 | A | 01 September 2016 |
| | | JP | 6769866 | B2 | 14 October 2020 |
| | | KR | 20210129257 | A | 27 October 2021 |
| | | KR | 102482890 | B1 | 30 December 2022 |
| | | SG | 10201801813 | YA | 27 April 2018 |
| | | MX | 2019010443 | A | 17 October 2019 |
| | | DK | 2991656 | T3 | 23 March 2020 |
| | | EP | 3546579 | A1 | 02 October 2019 |
| | | KR | 20160002974 | A | 08 January 2016 |
| | | KR | 102235678 | B1 | 05 April 2021 |
| | | BR | 122018009831 | B1 | 21 December 2021 |
| | | IL | 270464 | B | 29 July 2021 |
| | | HRP | 20190987 | T1 | 20 September 2019 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202311121470
- WO 2009082607 A
- WO 2014025805 A
- WO 2015006740 A
- WO 2021249484 A


### Non-patent literature cited in the description


- *CHEMICAL ABSTRACTS*, 147201-04-5
- *CHEMICAL ABSTRACTS*, 104992-55-4
- *CHEMICAL ABSTRACTS*, 121058-88-6
- *CHEMICAL ABSTRACTS*, 118362-03-1
- *CHEMICAL ABSTRACTS*, 98796-51-1
- *CHEMICAL ABSTRACTS*, 136834-22-5
- *CHEMICAL ABSTRACTS*, 159414-99-0
- *CHEMICAL ABSTRACTS*, 144089-97-4
- *CHEMICAL ABSTRACTS*, 146954-75-8
- *CHEMICAL ABSTRACTS*, 110782-31-5
- *CHEMICAL ABSTRACTS*, 199593-09-4
- *CHEMICAL ABSTRACTS*, 909033-40-5
- *CHEMICAL ABSTRACTS*, 110764-79-9